# EUROPEAN PATENT APPLICATION

(11) **EP 2 479 291 A1**
(43) Date of publication of application: **25.07.2012**
(21) Application number: 12165043.6
(22) Date of filing: 27.10.2006
(51) Int. Cl.: C12Q 1/68, C12P 19/34

(54) **Nucleic acid amplification using non-random primers**

(30) Priority: 27.10.2005 US 731529 P; 07.04.2006 US 790458 P
(62) Divisional of application: 06827033.9
(71) Applicant: Life Technologies Corporation, Carlsbad, CA 92008 (US)
(72) Inventor: Castle, John, Seattle, WA Washington 98115 (US); Raymond, Christopher K., Seattle, WA Washington 98115 (US); Armour, Christopher, Kirkland, WA Washington 98034 (US)
(74) Representative: Muir, Benjamin M. J.

(57) **Abstract**

The present invention provides methods for selectively amplifying a target population of nucleic acid molecules (e.g., all mRNA molecules expressed in a cell type except for the most highly expressed mRNA species). The present invention also provides populations of oligonucleotides including the nucleic acid sequences set forth in SEQ ID NOS:1-933. These oligonucleotides can be used, for example, to prime the synthesis of cDNA molecules complementary to mRNA molecules isolated from mammalian blood without priming the synthesis of cDNA molecules complementary to globin mRNA, or ribosomal RNA molecules.

## Description

### FIELD OF THE INVENTION

The present invention relates to oligonucleotides useful for priming the amplification of nucleic acid molecules.

### BACKGROUND OF THE INVENTION

Gene expression analysis often involves amplification of starting nucleic acid molecules. Amplification of nucleic acid molecules may be accomplished by reverse transcription (RT), *in vitro* transcription (IVT) or the polymerase chain reaction (PCR), either individually or in combination. The starting nucleic acid molecules may be mRNA molecules, which are amplified by first synthesizing complementary cDNA molecules, then synthesizing second cDNA molecules that are complementary to the first cDNA molecules, thereby producing double stranded cDNA molecules. The synthesis of first strand cDNA is typically accomplished using a reverse transcriptase and the synthesis of second strand cDNA is typically accomplished using a DNA polymerase. The double stranded cDNA molecules may be used to make complementary RNA molecules using an RNA polymerase, resulting in amplification of the original starting mRNA molecules. The RNA polymerase requires a promoter sequence to direct initiation of RNA synthesis. Complementary RNA molecules may, for example, be used as a template to make additional complementary DNA molecules.

Amplification of nucleic acid molecules requires the use of oligonucleotide primers that specifically hybridize to one or more target nucleic acid molecules in the starting material. Each oligonucleotide primer may include a promoter sequence that is located 5' to the hybridizing portion of the oligonucleotide that hybridizes to the target nucleic acid molecule(s). If the hybridizing portion of an oligonucleotide is too short, then the oligonucleotide does not stably hybridize to a target nucleic acid molecule and priming and subsequent amplification does not occur. Also, if the hybridizing portion of an oligonucleotide is too short, then the oligonucleotide does not specifically hybridize to one or a small number of target nucleic acid molecules, but non-specifically hybridizes to numerous target nucleic acid molecules.

Amplification of a complex mixture of different target nucleic acid molecules (e.g., mRNA molecules) typically requires the use of a population of numerous oligonucleotides having different nucleic acid sequences. The cost of the oligonucleotides increases with the length of the oligonucleotides. In order to control costs, it is preferable to make oligonucleotide primers that are no longer than the minimum length required to ensure specific hybridization of an oligonucleotide to a target sequence.

It is often undesirable to amplify highly expressed RNAs (e.g., ribosomal RNAs). For example, in gene expression experiments that analyze expression of genes in blood cells, amplification of numerous copies of abundant globin mRNAs, or ribosomal RNAs, may obscure subtle changes in the levels of rare mRNAs. Thus, there is a need for populations of oligonucleotide primers that selectively amplify desired nucleic acid molecules within a population of nucleic acid molecules (e.g., oligonucleotide primers that selectively amplify all mRNAs that are expressed in a cell except for the most highly expressed mRNAs). In order to reduce the cost of synthesizing the population of oligonucleotides, the hybridizing portion of each oligonucleotide should be no longer than necessary to ensure specific hybridization to a desired target sequence under defined conditions.

### SUMMARY OF THE INVENTION

In one aspect, the present invention provides methods for selectively amplifying a target population of nucleic acid molecules (e.g., all mRNA molecules expressed in a cell type except for the most highly expressed mRNA species). The methods of this aspect of the invention each include the step of using a population of oligonucleotides to prime the amplification of a target population of nucleic acid molecules within a larger population of nucleic acid molecules. The population of oligonucleotides is selected based on its ability to hybridize under defined conditions to a first subpopulation of a target nucleic acid population, but not to hybridize under the defined conditions to a second subpopulation of the target nucleic acid population.

In another aspect, the present invention provides populations of oligonucleotides including the nucleic acid sequences set forth in SEQ ID NOS:1-933. These oligonucleotides can be used, for example, to prime the synthesis of cDNA molecules complementary to mRNA molecules isolated from mammalian blood without priming the synthesis of cDNA molecules complementary to globin mRNA or ribosomal RNA molecules. In some embodiments, each oligonucleotide in the population of oligonucleotides further comprises a defined sequence portion located 5' to the hybridizing portion. In one embodiment, the defined sequence portion comprises a transcriptional promoter, which may be used as a primer binding site in PCR amplification, or for *in vitro* transcription. In another embodiment, the defined sequence portion comprises a primer binding site that is not a transcriptional promoter. For example, in some embodiments the present invention provides populations of oligonucleotides wherein a transcriptional promoter, such as the T7 promoter (SEQ ID NO:934), is located 5' to a member of the population of oligonucleotides having the sequences set forth in SEQ ID NOS: 1-933. Thus, in some embodiments, the present invention provides populations of oligonucleotides wherein each oligonucleotide consists of the T7 promoter (SEQ ID NO:934) located 5' to a different member of the population of oligonucleotides having the sequences set forth in SEQ ID NOS:1-933. In further embodiments, the present invention provides populations of oligonucleotides wherein the defined sequence portion comprises at least one primer binding site which is useful for priming a PCR synthesis reaction, and which does not include an RNA polymerase promoter sequence. A representative example of a defined sequence portion for use in such embodiments is provided as 5' CCGAACTACCCACTTGCATT 3' (SEQ ID NO:956), which is preferably located 5' to a member of the population of oligonucleotides having the sequences set forth in SEQ ID NOS:1-933.

In another aspect, the present invention provides a reagent for selectively amplifying a target population of nucleic acid molecules (e.g., all mRNA molecules expressed in a cell type except for the most highly expressed mRNA species). The reagent of this aspect of the invention comprises a population of oligonucleotides to prime the amplification of a target population of nucleic acid molecules, wherein each oligonucleotide comprises a hybridizing portion that consists of 6, 7, or 8 nucleotides. In some embodiments, the present invention provides a reagent comprising a population of oligonucleotides wherein the hybridizing portion is a member of the population of oligonucleotides having the sequences set forth in SEQ ID NOS:1-933. Thus, in some embodiments, the present invention provides a reagent comprising a population of oligonucleotides that includes at least 10% (such as at least 20%, 30%, 40%, 50%, 60%, 70%, 80%, 85%, 90%, 95%, or 99%) of the sequences set forth in SEQ ID NOS:1-933. In another embodiment, the present invention provides a reagent comprising populations of oligonucleotides wherein a defined sequence portion, such as a transcriptional promoter, (e.g., the T7 promoter (SEQ ID NO:934)) or a primer binding site (e.g., SEQ ID NO:956) is located 5' to a member of the population of oligonucleotides having the sequences set forth in SEQ ID NOS:1-933. Thus, in some embodiments, the present invention provides a reagent comprising populations of oligonucleotides wherein each oligonucleotide consists of the T7 promoter (SEQ ID NO:934) located 5' to a different member of the population of oligonucleotides having the sequences set forth in SEQ ID NOS:1-933.

In a further aspect, the present invention provides a kit for selectively amplifying a target population of nucleic acid molecules (e.g., all mRNA molecules expressed in a cell type except for the most highly expressed mRNA species). The kit of this aspect of the invention includes a reagent comprising a population of oligonucleotides to prime the amplification of a target population of nucleic acid molecules, wherein each oligonucleotide comprises a hybridizing portion that consists of 6, 7, or 8 nucleotides. In some embodiments, the present invention provides a kit that includes a reagent comprising a population of oligonucleotides wherein the hybridizing portion is a member of the population of oligonucleotides having the sequences set forth in SEQ ID NOS:1-933. Thus, in some embodiments, the present invention provides a kit including a reagent that comprises a population of oligonucleotides that includes at least 10% (such as at least 20%, 30%, 40%, 50%, 60%, 70%, 80%, 85%, 90%, 95%, or 99%) of the sequences set forth in SEQ ID NOS: 1-933. In another embodiment, the present invention provides a kit that includes a reagent comprising populations of oligonucleotides wherein a defined sequence portion, such as the transcriptional promoter, (e.g., the T7 promoter (SEQ ID NO:934)), is located 5' to a member of the population of oligonucleotides having the sequences set forth in SEQ ID NOS:1-933. Thus, in some embodiments, the present invention provides a kit including a reagent that comprises populations of oligonucleotides wherein each oligonucleotide consists of the T7 promoter (SEQ ID NO:934) located 5' to a different member of the population of oligonucleotides having the sequences set forth in SEQ ID NOS:1-933.

In another aspect, the present invention provides methods of selectively amplifying a target population of nucleic acid molecules to generate amplified RNA molecules. The method comprises: (a) providing a population of oligonucleotides, wherein each oligonucleotide comprises a hybridizing portion and transcriptional promoter portion located 5' to the hybridizing portion, wherein the hybridizing portion is a member of the population of oligonucleotides comprising SEQ ID NOS:1-933, (b) annealing the population of oligonucleotides to a sample comprising mRNA isolated from a mammalian subject, (c) synthesizing cDNA from the mRNA using a reverse transcriptase enzyme, (d)synthesizing double stranded cDNA using a DNA polymerase; and (e) transcribing the double-stranded cDNA into RNA using an RNA polymerase that binds to the transcriptional promoter portion of each oligonucleotide to generate amplified RNA.

In another aspect, the present invention provides methods of selectively amplifying a target population of nucleic acid molecules to generate amplified DNA molecules. The method comprises: (a) providing a first population of oligonucleotides, wherein each oligonucleotide comprises a hybridizing portion and a first PCR primer binding site located 5' to the hybridizing portion, wherein the hybridizing portion is a member of the population of oligonucleotides comprising SEQ ID NOS:1-933, (b) annealing the population of oligonucleotides to a sample comprising mRNA isolated from a mammalian subject, (c)synthesizing cDNA from the mRNA using a reverse transcriptase enzyme, (d) synthesizing double stranded cDNA using a DNA polymerase and a second population of oligonucleotides, wherein each oligonucleotide comprises a random hybridizing portion and a second PCR binding site located 5' to the hybridizing portion, and (e) PCR amplifying the double stranded cDNA using thermostable DNA polymerase, a first PCR primer that binds to the first PCR primer binding site and a second PCR primer that binds to the second PCR primer binding site to generate amplified double stranded DNA.

Aspects of the invention include the following aspects numbered #1 to #42:
#1. A method of selectively amplifying a target population of nucleic acid molecules, the method comprising the step of using a population of oligonucleotides to prime the amplification of a target population of nucleic acid molecules within a larger population of nucleic acid molecules, wherein:
   (a) each oligonucleotide comprises a hybridizing portion, wherein the hybridizing portion consists of one of 6, 7, or 8 nucleotides; and
   (b) the population of oligonucleotides is selected to hybridize under defined conditions to a first subpopulation of the target nucleic acid population, but not hybridize under the defined conditions to a second subpopulation of the target nucleic acid population.
#2. The method of Aspect #1, wherein each oligonucleotide further comprises a defined sequence portion located 5' to the hybridizing portion.
#3. The method of Aspect #2, wherein the defined sequence portion comprises a transcriptional promoter.
#4. The method of Aspect #2, wherein the defined sequence portion comprises a primer binding site for PCR amplification.
#5. The method of Aspect #1, wherein the population of hybridizing portions is selected from all possible oligonucleotides having a length of 6 nucleotides, 7 nucleotides, or 8 nucleotides.
#6. The method of Aspect #1, wherein the population of hybridizing portions is selected from all possible oligonucleotides having a length of 6 nucleotides.
#7. The method of Aspect #1, wherein the population of hybridizing portions is selected from the oligonucleotides comprising SEQ ID NOS:1 933.
#8. The method of Aspect #1, wherein the hybridizing portion is a member of the population of oligonucleotides comprising SEQ ID NOS:1 933.
#9. The method of Aspect #1, wherein the population of hybridizing portions is selected from all possible oligonucleotides having a length of 7 nucleotides.
#10. The method of Aspect #1, wherein the population of hybridizing portions is selected from all possible oligonucleotides having a length of 8 nucleotides.
#11. The method of Aspect #1, wherein the target population of nucleic acid molecules is obtained from mammalian blood cells.
#12. The method of Aspect #11, wherein the mammalian blood cells are selected from the group consisting of neutrophils, eosinophils, basophils, lymphocytes, monocytes, erythrocytes, and platelets.
#13. The method of Aspect #1, wherein the target population of nucleic acid molecules comprises mRNA molecules obtained from mammalian blood cells.
#14. The method of Aspect #1, wherein the second subpopulation of the target nucleic acid population consists essentially of the most abundant nucleic acid molecules in the target population of nucleic acid molecules.
#15. The method of Aspect #14, wherein the most abundant nucleic acid molecules are selected from the group consisting of ribosomal RNA, ribosomal DNA, globin DNA, or globin RNA.
#16. The method of Aspect #3, wherein the amplification of the target population of nucleic acid molecules comprises the step of reverse transcribing the target population of nucleic acid molecules followed by in vitro transcription to generate RNA molecules.
#17. The method of Aspect #4, wherein the amplification of the target population further comprises the step of reverse transcribing the target population of nucleic acid molecules followed by PCR amplification to generate double-stranded DNA molecules.
#18. The method of Aspect #16 or 17, wherein the reverse transcription step is carried out in the presence of dNTPs, wherein the dNTP concentration comprises a range of from 50 to 5000 micromolar.
#19. The method of Aspect #18, wherein the dNTP concentration comprises a range of from 1000 to 2000 micromolar.
#20. The method of Aspect #1, wherein the defined hybridization conditions comprise an annealing temperature in the range of from 40°C to 50°C.
#21. The method of Aspect #2, wherein a spacer portion is disposed between the defined sequence portion and the hybridizing portion of each oligonucleotide.
#22. A method of selectively amplifying a target population of nucleic acid molecules, the method comprising the step of using a population of oligonucleotides to prime the amplification of a target population of nucleic acid molecules within a larger population of nucleic acid molecules, wherein:
   (a) each oligonucleotide comprises a hybridizing portion, wherein the hybridizing portion consists of one of 6, 7, or 8 nucleotides and a defined sequence portion located 5' to the hybridizing portion; and
   (b) the population of oligonucleotides is selected to hybridize under defined conditions to a first subpopulation of a target nucleic acid population, but not hybridize under the defined conditions to a second subpopulation of the target nucleic acid population.
#23. The method of Aspect #22, wherein the defined sequence portion comprises a transcriptional promoter suitable for in vitro transcription.
#24. The method of Aspect #22, wherein the defined sequence portion comprises a primer binding site for PCR amplification.
#25. A method of selectively amplifying a target population of nucleic acid molecules, the method comprising the step of using a population of oligonucleotides to prime the amplification of a target population of nucleic acid molecules within a larger population of nucleic acid molecules, wherein each oligonucleotide within the population of oligonucleotides comprises a hybridizing portion and a defined sequence portion located 5' to the hybridizing portion, wherein the hybridizing portion is a member of the population of oligonucleotides comprising SEQ ID NOS:1 933.
#26. The method of Aspect #25, wherein the defined sequence portion comprises a transcriptional promoter suitable for in vitro transcription.
#27. The method of Aspect #25, wherein the population of hybridizing portions comprises at least 10% of the oligonucleotides comprising SEQ ID NOS:1 933.
#28. A population of oligonucleotides comprising SEQ ID NOS:1 933.
#29. A population of oligonucleotides consisting of SEQ ID NOS:1 933.
#30. A population of oligonucleotides comprising at least 10% of the oligonucleotides comprising SEQ ID NOS:1 933.
#31. A reagent for selectively amplifying a target population of nucleic acid molecules, the reagent comprising at least 10% of the oligonucleotides comprising SEQ ID NOS:1 933.
#32. A reagent for selectively amplifying a target population of nucleic acid molecules, the reagent comprising a population of oligonucleotides to prime the amplification of a target population of nucleic acid molecules, wherein each oligonucleotide comprises a hybridizing portion and a defined sequence portion located 5' to the hybridizing portion, wherein the hybridizing portion is a member of the population of oligonucleotides comprising SEQ ID NOS:1 933.
#33. The reagent of Aspect #32, wherein the defined sequence portion comprises a transcriptional promoter suitable for in vitro transcription.
#34. The reagent of Aspect #32, wherein the population of hybridizing portions comprises at least 10% of the oligonucleotides comprising SEQ ID NOS:1 933.
#35. The reagent of Aspect #32, wherein the population of hybridizing portions comprises the oligonucleotides consisting of SEQ ID NOS:1 933.
#36. A kit for selectively amplifying a target population of nucleic acid molecules, the kit comprising a reagent comprising a population of oligonucleotides, wherein each oligonucleotide comprises a hybridizing portion that is a member of the population of oligonucleotides comprising SEQ ID NOS:1 933.
#37. The kit of Aspect #36, wherein the population of hybridizing portions comprises at least 10% of the oligonucleotides comprising SEQ ID NOS:1 933.
#38. The kit of Aspect #36, wherein the population of hybridizing portions comprises the oligonucleotides consisting of SEQ ID NOS:1 933.
#39. The kit of Aspect #36, further comprising at least one of the following components: a reverse transcriptase, a DNA polymerase, a DNA ligase, a RNase H enzyme, a Tris buffer, a potassium salt, a magnesium salt, an ammonium salt, a reducing agent, deoxynucleoside triphosphates, or a ribonuclease inhibitor.
#40. The kit of Aspect #36, further comprising at least one of the following components: an RNA polymerase, an IPPase, a transcription buffer, a Tris buffer, a sodium salt, a magnesium salt, spermidine, a reducing agent, nucleoside triphosphates, or amino-allyl-UTP.
#41. A method of selectively amplifying a target population of nucleic acid molecules, the method comprising the steps of:
   (a) providing a population of oligonucleotides, wherein each oligonucleotide comprises a hybridizing portion and transcriptional promoter portion located 5' to the hybridizing portion, wherein the hybridizing portion is a member of the population of oligonucleotides comprising SEQ ID NOS:1 933;
   (b) annealing the population of oligonucleotides to a sample comprising mRNA isolated from a mammalian subject;
   (c) synthesizing cDNA from the mRNA using a reverse transcriptase enzyme;
   (d) synthesizing double stranded cDNA using a DNA polymerase; and
   (e) transcribing the double-stranded cDNA into RNA using an RNA polymerase that binds to the transcriptional promoter portion of each oligonucleotide to generate amplified RNA.
#42. A method of selectively amplifying a target population of nucleic acid molecules, the method comprising the steps of:
   (a) providing a first population of oligonucleotides, wherein each oligonucleotide comprises a hybridizing portion and a first PCR primer binding site located 5' to the hybridizing portion, wherein the hybridizing portion is a member of the population of oligonucleotides comprising SEQ ID NOS:1 933;
   (b) annealing the population of oligonucleotides to a sample comprising mRNA isolated from a mammalian subject;
   (c) synthesizing cDNA from the mRNA using a reverse transcriptase enzyme;
   (d) synthesizing double stranded cDNA using a DNA polymerase and a second population of oligonucleotides, wherein each oligonucleotide comprises a random hybridizing portion and a second PCR binding site located 5' to the hybridizing portion; and
   (e) PCR amplifying the double stranded cDNA using thermostable DNA polymerase, a first PCR primer that binds to the first PCR primer binding site and a second PCR primer that binds to the second PCR primer binding site to generate amplified double stranded DNA.

### BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
FIGURE 1A shows the number of exact matches for random 6-mers (N6) oligonucleotides on nucleotide sequences in the human RefSeq transcript database as described in EXAMPLE 2;
FIGURE 1B shows the number of exact matches for Not-So-Random (NSR) 6-mer oligonucleotides on nucleotide sequences in the human RefSeq transcript database as described in EXAMPLE 2;
FIGURE 1C shows a representative embodiment of the methods of the invention for synthesizing a preparation of amplified RNA molecules using a mixture of NSR 6-mer oligonucleotides as described in EXAMPLE 4;
FIGURE 1D shows a representative embodiment of the methods of the invention for synthesizing a preparation of amplified DNA molecules using a mixture of NSR 6-mer oligonucleotides as described in EXAMPLE 12;
FIGURE 2 is a histogram plot showing the number of NSR 6-mer binding sites per 100 nucleotides present in the human RefSeq transcript database as described in EXAMPLE 2;
FIGURE 3 is a histogram plot showing the yield of amplified RNA from T7-NSR6-mer primed cDNA as a function of input RNA template amount as described in EXAMPLE 6;
FIGURE 4 is a histogram plot showing the yield of amplified RNA from T7-NSR6-mer and T7-N8 primed cDNA for reporter and background genes as a function of primer and dNTP concentrations as described in EXAMPLE 7;
FIGURE 5 is a histogram plot showing the yield of amplified RNA from T7-NSR and T7-N7 primed cDNA as a function of the amount of input RNA template as described in EXAMPLE 9;
FIGURE 6 is a histogram plot on a linear scale showing the relative composition of T7-NSR and T7-N7 primed cDNA (normalized to N8) following amplification by *in vitro* transcription as described in EXAMPLE 9;
FIGURE 7 is a histogram plot on a logarithmic scale showing the relative composition of T7-NSR and T7-N7 primed cDNA (normalized to N8) following amplification by *in vitro* transcription as described in EXAMPLE 9;
FIGURE 8 is a histogram plot of relative abundance of *in vitro* transcription products from T7-NSR-primed cDNA as a function of RNA template amount as described in EXAMPLE 9;
FIGURE 9 is a histogram plot of relative abundance of *in vitro* transcription products from T7-N7-primed cDNA as a function of RNA template amount as described in EXAMPLE 9;
FIGURE 10 graphically illustrates the correlation in expression values from a panel of 34 reporter genes as measured by quantitative PCR of T7-NSR-primed cDNA (x-axis) versus amplified DNA (aDNA) (y-axis) generated from T7-NSR-primed cDNA as described in EXAMPLE 12; and
FIGURE 11 is a histogram plot of the gene specific activities of a panel of 10 reporter genes as measured by quantitative PCR of amplified DNA generated using a range of primer concentrations.

### DETAILED DESCRIPTION OF THE PREFERRED EMBODIMENT

Unless specifically defined herein, all terms used herein have the same meaning as they would to one skilled in the art of the present invention. Practitioners are particularly directed to Sambrook et al., Molecular Cloning: A Laboratory Manual, 2d ed., Cold Spring Harbor Press, Plainsview, New York; and Ausubel et al., Current Protocols in Molecular Biology (Supplement 47), John Wiley & Sons, New York, 1999, for definitions and terms of the art.

In one aspect, the present invention provides methods for selectively amplifying a target population of nucleic acid molecules. The methods of this aspect of the invention each include the step of using a population of oligonucleotides to prime the amplification (e.g., by reverse transcription, *in vitro* transcription, or polymerase chain reaction (PCR), or a combination thereof) of a target population of nucleic acid molecules within a larger population of nucleic acid molecules, wherein (a) each oligonucleotide comprises a hybridizing portion that consists of 6 nucleotides, or 7 nucleotides, or 8 nucleotides; and (b) the population of oligonucleotides is selected to hybridize under defined conditions to a first subpopulation of a target nucleic acid population, but not hybridize under the defined conditions to a second subpopulation of the target nucleic acid population. The population of oligonucleotides may also include a defined sequence portion located 5' to the hybridizing portion. In one embodiment, the defined sequence portion comprises a transcriptional promoter, which can also be used as a primer binding site. Therefore, in certain embodiments of this aspect of the invention, each oligonucleotide of the population of oligonucleotides comprises a hybridizing portion that consists of 6 nucleotides, or 7 nucleotides, or 8 nucleotides; and a transcriptional promoter portion located 5' to the hybridizing portion. In another embodiment, the defined sequence portion includes a first primer binding site for use in a PCR amplification reaction, and which may optionally include a transcriptional promoter. By way of example, the populations of oligonucleotides provided by the present invention are useful in the practice of the methods of this aspect of the invention.

For example, in one embodiment of the present invention, a population of oligonucleotides (SEQ ID NOS:1-933), that each have a length of 6 nucleotides, was identified that can be used as primers to prime the amplification of all, or substantially all, mRNA molecules from mammalian blood cells, but that do not prime the amplification of globin mRNA or ribosomal RNAs from mammalian blood cells. The identified population of oligonucleotides (SEQ ID NOS:1-933) is referred to as Not-So-Random (NSR) primers. Thus, this population of oligonucleotides (SEQ ID NOS:1-933) can be used to prime the synthesis of a population of nucleic acid molecules (e.g., cDNAs) that are representative of a starting population of mRNA molecules isolated from mammalian blood cells, but that does not include a large number of cDNA molecules that correspond to globin mRNAs or to ribosomal RNAs. The present invention also provides populations of oligonucleotides wherein a defined sequence, such as the T7 promoter (SEQ ID NO:934), is located 5' to a member of the population of oligonucleotides having the sequences set forth in SEQ ID NOS:1-933. Thus, each oligonucleotide may include a hybridizing portion (selected from SEQ ID NOS:1-933) that hybridizes to target nucleic acid molecules (e.g., mRNAs), and a promoter sequence located 5' to the hybridizing portion. The promoter sequence may be incorporated into DNA molecules amplified using the oligonucleotides (that include the T7 promoter) as primers, and can thereafter promote transcription from the DNA molecules. Alternatively, a defined sequence portion such as a transcriptional promoter may be covalently attached to the cDNA molecule, for example, by DNA ligase enzyme.

The first subpopulation of a target nucleic acid population can include, for example, all mRNAs expressed in a cell or tissue except for a selected group of mRNAs, such as, for example, the most abundantly expressed mRNAs. An abundantly expressed mRNA typically constitutes at least 0.1 % of all the mRNA expressed in the cell or tissue (and may constitute, for example, more than 50%, or more than 60%, or more than 70% of all the mRNA expressed in the cell or tissue). An example of an abundantly expressed mRNA is globin mRNA in certain blood cells. Useful transcription promoter sequences include the T7 promoter (5'AATTAATACGACTCACTATAGGGAGA3') (SEQ ID NO:934)), the SP6 promoter (5'ATTTAGGTGACACTATAGAAGNG3' (SEQ ID NO:935)), and the T3 promoter (5'AATTAACCCTCACTAAAGGGAGA3' (SEQ ID NO:936)).

The methods of the invention are useful for transcriptome profiling of total RNA in a biological sample, such as whole blood, in which it is desirable to reduce the presence of a group of mRNAs (that do not hybridize to the NSR primers) from an amplified sample, such as, for example, highly expressed RNAs (e.g. globin mRNA or ribosomal RNAs). In some embodiments, the methods of the invention may be used to reduce the amount of a group of nucleic acid molecules that do not hybridize to the NSR primers in amplified nucleic acid derived from an mRNA sample by at least 2 fold up to 1000 fold, such as at least 10 fold, 50 fold, 100 fold, 500 fold, or greater, in comparison to the amount of amplified nucleic acid molecules that do hybridize to the NSR primers.

Populations of oligonucleotides used to practice the method of this aspect of the invention are selected from within a larger population of oligonucleotides, wherein (a) the subpopulation of oligonucleotides is selected based on its ability to hybridize under defined conditions to a first subpopulation of a target nucleic acid population, but not hybridize under the defined conditions to a second subpopulation of the target nucleic acid population; and (b) the population of oligonucleotides comprises all possible oligonucleotides having a length of 6 nucleotides, 7 nucleotides, or 8 nucleotides.

Composition of population of oligonucleotides. The population of oligonucleotides includes all possible oligonucleotides having a length of 6 nucleotides, or 7 nucleotides, or 8 nucleotides. The population of oligonucleotides may include only all possible oligonucleotides having a length of 6 nucleotides, or all possible oligonucleotides having a length of 7 nucleotides, or all possible oligonucleotides having a length of 8 nucleotides. Optionally the population of oligonucleotides may include other oligonucleotides in addition to all possible oligonucleotides having a length of 6 nucleotides, or all possible oligonucleotides having a length of 7 nucleotides, or all possible oligonucleotides having a length of 8 nucleotides. Typically, each member of the population of oligonucleotides is no more than 30 nucleotides long.

Sequences of population of oligonucleotides. There are 4,096 possible oligonucleotides having a length of 6 nucleotides; 16,384 possible oligonucleotides having a length of 7 nucleotides; and 65,536 possible oligonucleotides having a length of 8 nucleotides. The sequences of the oligonucleotides that constitute the population of oligonucleotides can readily be generated by a computer program, such as Microsoft Word.

Selection of subpopulation of oligonucleotides. The subpopulation of oligonucleotides is selected from the population of oligonucleotides based on the ability of the members of the subpopulation of oligonucleotides to hybridize, under defined conditions, to a first subpopulation of a target nucleic acid population, but not hybridize under the same defined conditions to a substantial number of members of a second subpopulation of the target nucleic acid population. A target nucleic acid population is a population of nucleic acid molecules (e.g., mRNA or DNA molecules) that includes nucleic acid molecules that are to be amplified (e.g., using reverse transcription, *in vitro* transcription, the polymerase chain reaction, or a combination thereof) to produce amplified RNA, single stranded DNA, or double stranded DNA, and also includes nucleic acid molecules that are not to be amplified. The subpopulation of oligonucleotides is made up of oligonucleotides that each hybridize, under defined conditions, to sequences distributed throughout the population of the nucleic acid molecules that are to be amplified, but that do not hybridize, under the same defined conditions, to most (or any) of the nucleic acid molecules that are not to be amplified. The subpopulation of oligonucleotides hybridizes, under defined conditions, to target nucleic acid sequences other than those that have been intentionally avoided.

For example, the population of all mRNA molecules expressed in mammalian blood cells (e.g., human blood cells) can be a target population of nucleic acid molecules. This target population contains many mRNA molecules that encode globin proteins. This target population also contains many ribosomal RNA molecules (e.g., 5S, 18S, and 28S ribosomal RNAs). It is typically undesirable to amplify the globin mRNAs or the ribosomal RNAs. For example, in gene expression experiments that analyze expression of genes in blood cells, amplification of numerous copies of abundant globin mRNAs, or ribosomal RNAs, may obscure subtle changes in the levels of less abundant mRNAs. Consequently, in the practice of the present invention, a subpopulation of oligonucleotides is selected that does not hybridize, under defined conditions, to most (or any) globin mRNAs or to most (or any) ribosomal RNAs, but that does hybridize, under the same defined conditions, to most (preferably all) of the other mRNA molecules expressed in the blood cells. Examples of blood cells include leukocytes (e.g., neutrophils, eosinophils, basophils, lymphocytes, and monocytes), erythrocytes, and platelets.

In order to select a subpopulation of oligonucleotides that hybridizes under defined conditions to a first subpopulation of a target nucleic acid population, but does not hybridize under the defined conditions to a second subpopulation of the target nucleic acid population, it is necessary to know the complete, or substantially complete, nucleic acid sequences of the member(s) of the second subpopulation of the target nucleic acid population. Thus, for example, if the second subpopulation includes globin mRNAs and ribosomal RNAs, it is necessary to know the nucleic acid sequences of the globin mRNAs (or a representative globin mRNA) and the 5S, 18S, and 28S ribosomal RNAs (or a representative member of each of the foregoing classes of ribosomal RNA).

A suitable software program is then used to compare the sequences of all of the oligonucleotides in the population of oligonucleotides (e.g., the population of all possible 6 nucleic acid oligonucleotides) to the sequences of the globin mRNA(s) and ribosomal RNAs to determine which of the oligonucleotides will hybridize to any portion of the globin mRNA(s) and ribosomal RNAs under defined hybridization conditions. Only the oligonucleotides that do not hybridize to any portion of the globin mRNA(s) and ribosomal RNAs, under defined hybridization conditions, are selected. Perl script may easily be written that permits comparison of nucleic acid sequences, and identification of sequences that hybridize to each other under defined hybridization conditions.

Thus, for example, as described more fully in Example 1, the subpopulation of all possible 6 nucleic acid oligonucleotides that were not exactly complementary to any portion of any ribosomal RNA sequence or that were not exactly complementary to any portion of a globin mRNA sequence were identified. In general, the subpopulation of oligonucleotides (that hybridizes under defined conditions to a first subpopulation of a target nucleic acid population, but does not hybridize under the defined conditions to a second subpopulation of the target nucleic acid population) must contain enough different oligonucleotide sequences to hybridize to all, or substantially all, nucleic acid molecules in the target nucleic acid population. Example 2 herein shows that the population of oligonucleotides having the nucleic acid sequences set forth in SEQ ID NOS:1-933 hybridizes to all, or substantially all, nucleic acid sequences within a population of gene transcripts stored in the publicly accessible database called RefSeq.

Additional defined nucleic acid sequence portions. The selected subpopulation of oligonucleotides (e.g., SEQ ID NOS:1-933) can be used to prime the amplification of a target population of nucleic acid molecules. Alternatively, a population of oligonucleotides can be used as primers wherein each oligonucleotide includes the sequence of one member of the selected subpopulation of oligonucleotides, and also includes an additional defined nucleic acid sequence. The additional defined nucleic acid sequence is typically located 5' to the sequence of the member of the selected subpopulation of oligonucleotides. Typically, the population of oligonucleotides includes the sequences of all members of the selected subpopulation of oligonucleotides (e.g., the population of oligonucleotides can include all of the sequences set forth in SEQ ID NOS:1-933).

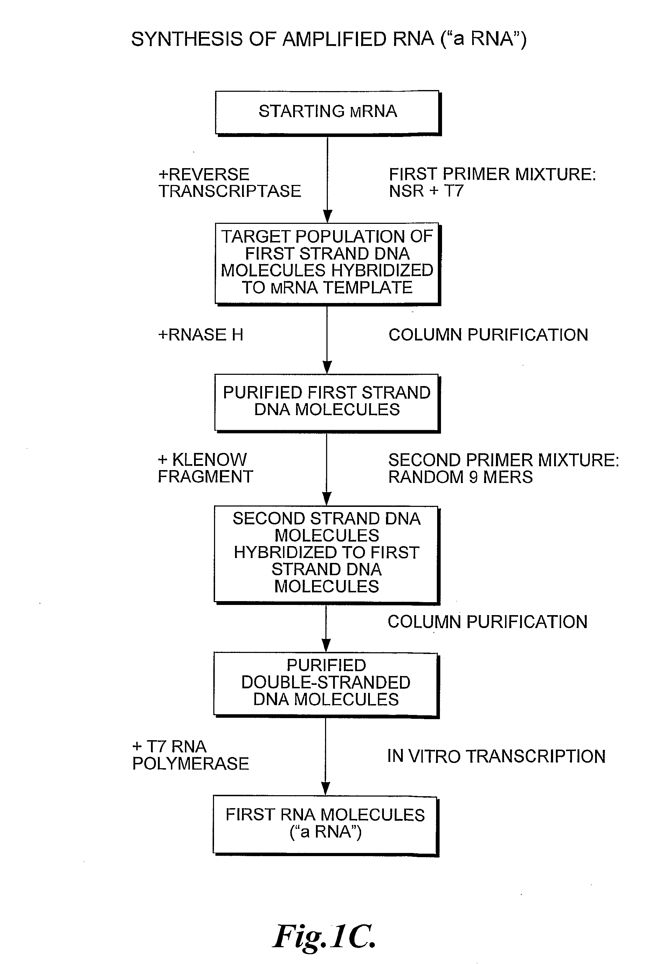

The additional defined nucleic acid sequence is selected so that it does not affect the hybridization specificity of the oligonucleotide to a complementary target sequence. For example, as described in FIGURE 1C, each oligonucleotide can include a transcriptional promoter sequence located 5' to the sequence of the member of the selected subpopulation of oligonucleotides. The promoter sequence may be incorporated into the amplified nucleic acid molecules which can, therefore, be used as templates for the synthesis of RNA. Any RNA polymerase promoter sequence can be included in the defined sequence portion of the population of oligonucleotides. Representative examples include the T7 promoter (SEQ ID NO:934), the SP6 promoter (SEQ ID NO:935), and the T3 promoter (SEQ ID NO:936).

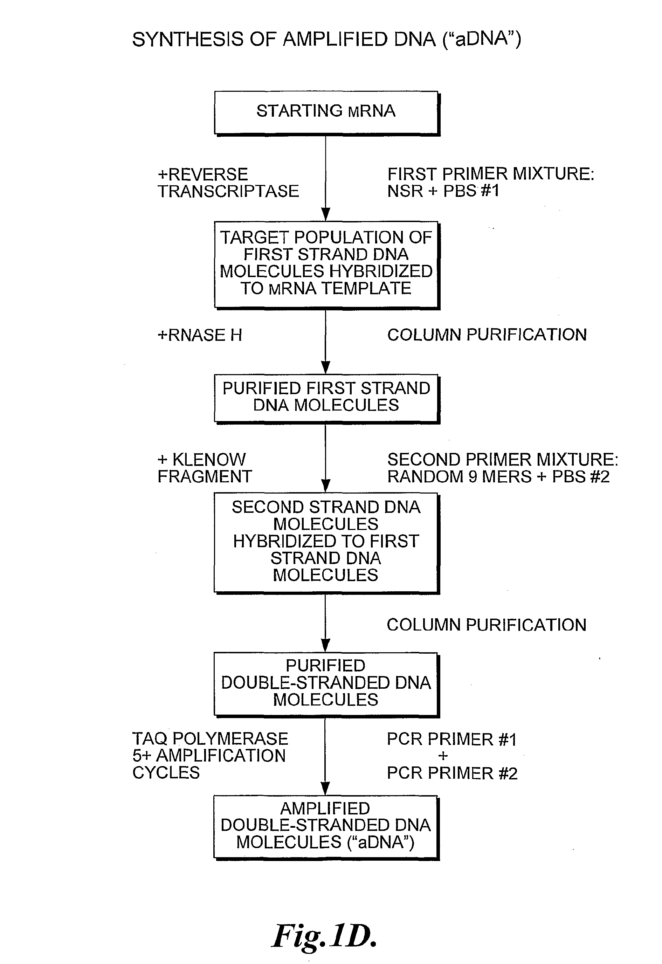

In another example, as described in FIGURE 1D, each oligonucleotide can include a defined sequence comprising a primer binding site located 5' to the sequence of the member of the selected subpopulation of oligonucleotides. The primer binding site is incorporated into the amplified nucleic acids which can then be used as a PCR primer binding site for the generation of double-stranded amplified DNA products from the cDNA. The primer binding site may be a portion of a transcriptional promoter sequence, as shown for example in TABLE 35. Alternatively, the primer binding site may not include a portion of a transcriptional promoter sequence, (e.g., SEQ ID NO:956, as described in Example 11).

For example, one embodiment of the present invention provides a population of oligonucleotides wherein each oligonucleotide of the population includes: (a) a sequence of a 6 nucleic acid oligonucleotide that is a member of a subpopulation of oligonucleotides (SEQ ID NOS:1-933), wherein the subpopulation of oligonucleotides hybridizes to all, or substantially all, mRNAs expressed in mammalian blood cells, but does not hybridize to globin mRNAs or to ribosomal RNAs; and (b) a T7 transcriptional promoter sequence (SEQ ID NO:934) located 5' to the sequence of the 6 nucleic acid oligonucleotide. In one embodiment, the population of oligonucleotides includes all of the 6 nucleotide sequences set forth in SEQ ID NOS: 1-933. In another embodiment, the population of oligonucleotides includes at least 10% (such as at least 20%, 30%, 40%, 50%, 60%, 70%, 80%, 85%, 90%, 95%, or 99%) of the 6 nucleotide sequences set forth in SEQ ID NOS:1-933.

Optionally, a spacer portion is located between the defined sequence portion and the hybridizing portion. The spacer portion is typically from 1 to 12 nucleotides long (e.g., from 1 to 6 nucleotides long) and can include any combination of nucleotides. The spacer portion can, for example, be composed of a random selection of nucleotides. All or part of the spacer portion may, or may not, hybridize to the same target nucleic acid sequence as the hybridizing portion. If all, or part, of the spacer portion hybridizes to the same target nucleic acid sequence as the hybridizing portion, then the effect is to enhance the efficiency of cDNA synthesis primed by the oligonucleotide that includes the hybridizing portion and the hybridizing spacer portion.

Hybridization conditions. In the practice of the present invention, a subpopulation of oligonucleotides is selected from a population of oligonucleotides based on the ability of the members of the subpopulation of oligonucleotides to hybridize, under defined conditions, to a first subpopulation of a target nucleic acid population, but not hybridize under the same defined conditions to a second subpopulation of the target nucleic acid population. The defined hybridization conditions permit the oligonucleotides to specifically hybridize to all nucleic acid molecules that are present in the sample except for globin mRNAs or ribosomal RNAs. Typically, hybridization conditions are no more than 25°C to 30°C (for example, 10°C) below the melting temperature (Tm) of the native duplex. Tm for nucleic acid molecules greater than about 100 bases can be calculated by the formula Tm = 81.5 + 0.41%(G+C) - log(Na+), wherein (G+C) is the guanosine and cytosine content of the nucleic acid molecule. For oligonucleotide molecules less than 100 bases in length, exemplary hybridization conditions are 5 to 10°C below Tm. On average, the Tm of a short oligonucleotide duplex is reduced by approximately (500/oligonucleotide length)°C. In some embodiments of the present invention, the hybridization temperature is in the range of from 40°C to 50°C. The appropriate hybridization conditions may also be identified empirically, without undue experimentation.

Amplification Conditions. In the practice of the present invention, the amplification of the first subpopulation of a target nucleic acid population occurs under defined amplification conditions. Hybridization conditions can be chosen as described, *supra.* Typically, the defined amplification conditions include first strand cDNA synthesis using a reverse transcriptase enzyme. The reverse transcription reaction is performed in the presence of defined concentrations of deoxynucleoside triphosphates (dNTPs). In some embodiments, the dNTP concentration is in a range from about 1000 to about 2000 microMolar in order to enrich the amplified product for target genes, as described in Examples 5-9.

Composition and synthesis of oligonucleotides. An oligonucleotide primer useful in the practice of the present invention can be DNA, RNA, PNA, chimeric mixtures, or derivatives or modified versions thereof, so long as it is still capable of priming the desired reaction. The oligonucleotide primer can be modified at the base moiety, sugar moiety, or phosphate backbone, and may include other appending groups or labels, so long as it is still capable of priming the desired amplification reaction.

For example, an oligonucleotide primer may comprise at least one modified base moiety which is selected from the group including but not limited to 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5N-methoxycarboxymethyluracil, 5-methoxyuracil, 2-methylthio-N6-isopentenyladenine, uracil-5-oxyacetic acid, pseudouracil, queosine, 2-thiocytosine, 5-methyl-2-thiouracil, 2-thiouracil, 4-thiouracil, 5-methyluracil, uracil-5-oxyacetic acid methylester, 5-methyl-2-thiouracil, 3-(3-amino-3-N-2-carboxypropyl) uracil, and 2,6-diaminopurine.

Again by way of example, an oligonucleotide primer can include at least one modified sugar moiety selected from the group including, but not limited to, arabinose, 2-fluoroarabinose, xylulose, and hexose.

By way of further example, an oligonucleotide primer can include at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal, or analog thereof.

An oligonucleotide primer for use in the methods of the present invention may be derived by cleavage of a larger nucleic acid fragment using non-specific nucleic acid cleaving chemicals or enzymes, or site-specific restriction endonucleases, or by synthesis by standard methods known in the art, for example, by use of an automated DNA synthesizer (such as are commercially available from Biosearch, Applied Biosystems, etc.) and standard phosphoramidite chemistry. As examples, phosphorothioate oligonucleotides may be synthesized by the method of Stein et al. (Nucl. Acids Res. 16:3209-3221, 1988) and methylphosphonate oligonucleotides can be prepared by use of controlled pore glass polymer supports (Sarin et al., Proc. Natl. Acad. Sci. U.S.A. 85:7448-7451, 1988).

Once the desired oligonucleotide is synthesized, it is cleaved from the solid support on which it was synthesized, and treated, by methods known in the art, to remove any protecting groups present. The oligonucleotide may then be purified by any method known in the art, including extraction and gel purification. The concentration and purity of the oligonucleotide may be determined by examining an oligonucleotide that has been separated on an acrylamide gel, or by measuring the optical density at 260 nm in a spectrophotometer.

The methods of this aspect of the invention can be used, for example, to selectively amplify coding regions of mRNAs, introns, alternatively spliced forms of a gene, and non-coding RNAs that regulate gene expression.

In another aspect, the present invention provides populations of oligonucleotides comprising at least 10% (such as at least 20%, 30%, 40%, 50%, 60%, 70%, 80%, 85%, 90%, 95%, or 99%) of the nucleic acid sequences set forth in SEQ ID NOS:1-933. These oligonucleotides (SEQ ID NOS:1-933) can be used, for example, to prime the synthesis of cDNA molecules complementary to mRNA molecules isolated from mammalian blood without priming the synthesis of cDNA molecules complementary to globin mRNA or ribosomal RNA molecules. Indeed, these oligonucleotides (SEQ ID NOS:1-933) can be used, for example, to prime the synthesis of cDNA using any population of mRNA molecules as templates, without amplifying a significant amount of globin mRNAs or ribosomal RNAs. For example, the present invention provides populations of oligonucleotides wherein a defined sequence portion, such as a transcriptional promoter, such as the T7 promoter (SEQ ID NO:934), is located 5' to a member of the population of oligonucleotides having the sequences set forth in SEQ ID NOS:1-933. Thus, in some embodiments, the present invention provides populations of oligonucleotides wherein each oligonucleotide consists of the T7 promoter (SEQ ID NO:934) located 5' to a different member of the population of oligonucleotides having the sequences set forth in SEQ ID NOS:1-933. In some embodiments, the population of oligonucleotides includes at least 10% (such as 20%, 30%, 40%, 50%, 60%, 70%, 80%, 90%, 95%, or 99%) of the 6 nucleotide sequences set forth in SEQ ID NOS:1-933.

In another aspect, the present invention provides a reagent for selectively amplifying a target population of nucleic acid molecules. The reagent can be used, for example, to prime the synthesis of cDNA molecules complementary to mRNA molecules isolated from mammalian blood cells without priming the synthesis of cDNA molecules complementary to globin mRNA or ribosomal RNA molecules. The reagent of the present invention comprises a population of oligonucleotides comprising at least 10% of the nucleic acid sequences set forth in SEQ ID NOS:1-933. In some embodiments, the present invention provides a reagent comprising a population of oligonucleotides that includes at least 10% (such as 20%, 30%, 40%, 50%, 60%, 70%, 80%, 85%, 90%, 95%, or 99%) of the 6 nucleotide sequences set forth in SEQ ID NOS:1-933. In some embodiments, the population of oligonucleotides is selected to hybridize to substantially all nucleic acid molecules that are present in a sample except for globin mRNAs or ribosomal RNAs. In other embodiments, the population of oligonucleotides is selected to hybridize to a subset of nucleic acid molecules that are present in a sample, wherein the subset of nucleic acid molecules does not include globin mRNAs or ribosomal RNAs.

In another embodiment, the present invention provides a reagent that comprises a population of oligonucleotides wherein a defined sequence portion comprising a transcriptional promoter, such as the T7 promoter, is located 5' to a member of the population of oligonucleotides having the sequences set forth in SEQ ID NOS:1-933. Thus, in some embodiments, the present invention provides a reagent comprising populations of oligonucleotides wherein each oligonucleotide consists of the T7 promoter (SEQ ID NO:934) located 5' to a different member of the population of oligonucleotides having the sequences set forth in SEQ ID NOS:1-933.

In another embodiment, the present invention provides a reagent that comprises a population of oligonucleotides wherein a defined sequence portion comprising a primer binding site is located 5' to a member of the population of oligonucleotides having the sequences set forth in SEQ ID NOS:1-933. Thus, in some embodiments, the present invention provides a reagent comprising populations of oligonucleotides wherein each oligonucleotide consists of the primer binding site (SEQ ID NO:956) located 5' to a different member of the population of oligonucleotides having the sequences set forth in SEQ ID NOS: 1-933.The reagent of the present invention can be provided as an aqueous solution, or an aqueous solution with the water removed, or a lyophilized solid.

In a further embodiment, the reagent of the present invention may include one or more of the following components for the production of double-stranded cDNA: a reverse transcriptase, a DNA polymerase, a DNA ligase, a RNase H enzyme, a Tris buffer, a potassium salt, a magnesium salt, an ammonium salt, a reducing agent, deoxynucleoside triphosphates (dNTPs), [beta]-nicotinamide adenine dinucleotide (β-NAD+), and a ribonuclease inhibitor. For example, the reagent may include components optimized for first strand cDNA synthesis, such as a reverse transcriptase with reduced RNase H activity and increased thermal stability (e.g., SuperScript™ III Reverse Transcriptase, Invitrogen), and a final concentration of dNTPs in the range of from 50 to 5000 microMolar, or more preferably in the range of from 1000 to 2000 microMolar.

In another aspect, the present invention provides kits for selectively amplifying a target population of nucleic acid molecules. In some embodiments, the kits comprise a reagent that comprises a population of oligonucleotides wherein a defined sequence portion, such as a transcriptional promoter, (e.g., the T7 promoter), is located 5' to a member of the population of oligonucleotides having the sequences set forth in SEQ ID NOS:1-933. Thus, in some embodiments, the present invention provides kits containing a reagent comprising populations of oligonucleotides wherein each oligonucleotide consists of the T7 promoter (SEQ ID NO:934) located 5' to a different member of the population of oligonucleotides having the sequences set forth in SEQ ID NOS:1-933. The kits according to this embodiment are useful for producing *in vitro* transcribed RNA from cDNA generated using the not-so-random primers of the invention.

In some embodiments, the kits contain a reagent comprising populations of oligonucleotides wherein each oligonucleotide consists of a defined sequence suitable for use as a primer binding site located 5' to a different member of the population of oligonucleotides having the sequences set forth in SEQ ID NOS:1-933. The kits according to this embodiment are useful for producing double-stranded DNA generated from PCR amplification of cDNA generated using the not-so-random primers of the invention.

The kits of the invention may be designed to detect any subpopulation of a target nucleic acid population, for example, all mRNAs expressed in a cell or tissue except for the most abundantly expressed mRNAs, in accordance with the methods described herein. Nonlimiting examples of mammalian mRNA target molecules include all or substantially all of the mRNA molecules from mammalian blood cells. Nonlimiting examples of exemplary oligonucleotide primers include SEQ ID NOS: 1-933. Nonlimiting examples of the transcription promoter are set forth as SEQ ID NOS:934-936. Nonlimiting examples of primer binding regions are set forth as SEQ ID NO:946, 955, and 956.

The spacer portion may include any combination of nucleotides, including nucleotides that hybridize to the target mRNA.

In certain embodiments, the kit comprises a reagent comprising oligonucleotide primers with hybridizing portions of 6, 7, or 8 nucleotides.

In certain embodiments, the kit comprises a reagent comprising a population of oligonucleotide primers that may be used to detect a plurality of mammalian mRNA targets.

In certain embodiments, the kit comprises oligonucleotides that hybridize in the temperature range of from 40°C to 50°C.

In another embodiment, the kit comprises a subpopulation of oligonucleotides that do not detect rRNA or globin mRNA. Exemplary oligonucleotides for use in accordance with this embodiment of the kit are provided in SEQ ID NOS:1-933.

In some embodiments, the kits comprises a reagent comprising a population of oligonucleotides comprising at least 10% (such as at least 20%, 30%, 40%, 50%, 60%, 70%, 80%, 85%, 90%, 95%, or 99%) of the 6 nucleotide sequences set forth in SEQ ID NOS:1-933.

In certain embodiments, the kit includes oligonucleotides wherein the transcription promoter comprises the T7 promoter (SEQ ID NO:934), the SP6 promoter (SEQ ID NO:935), or the T3 promoter (SEQ ID NO:936).

In another embodiment, the kit may comprise oligonucleotides with a spacer portion of from 1 to 12 nucleotides that comprises any combination of nucleotides.

In some embodiments of the present invention, the kit may further comprise one or more of the following components for the production of cDNA—a reverse transcriptase enzyme, a DNA polymerase enzyme, a DNA ligase enzyme, a RNase H enzyme, a Tris buffer, a potassium salt (e.g., potassium chloride), a magnesium salt (e.g., magnesium chloride), an ammonium salt (e.g., ammonium sulfate), a reducing agent (e.g., dithiothreitol), deoxynucleoside triphosphates (dNTPs), [beta]-nicotinamide adenine dinucleotide (β-NAD+), and a ribonuclease inhibitor. For example, the kit may include components optimized for first strand cDNA synthesis, such as a reverse transcriptase with reduced RNase H activity and increased thermal stability (e.g., SuperScript™ III Reverse Transcriptase, Invitrogen), and a dNTP stock solution to provide a final concentration of dNTPs in the range of from 50 to 5,000 microMolar, or more preferably in the range of from 1000 to 2000 microMolar.

In various embodiments, the kit may include a detection reagent such as SYBR green dye or BEBO dye that preferentially or exclusively binds to double stranded DNA during a PCR amplification step. In other embodiments, the kit may include a forward and/or reverse primer that includes a fluorophore and quencher to measure the amount of the PCR amplification products.

A kit of the invention can also provide reagents for *in vitro* transcription of the amplified cDNAs. For example, in some embodiments, the kit may further include one or more of the following components—a RNA polymerase enzyme, an IPPase (Inositol polyphosphate 1-phosphatase) enzyme, a transcription buffer, a Tris buffer, a sodium salt (e.g., sodium chloride), a magnesium salt (e.g., magnesium chloride), spermidine, a reducing agent (e.g., dithiothreitol), nucleoside triphosphates (ATP, CTP, GTP, UTP), and amino-allyl-UTP.

In another embodiment, the kit may include reagents for labeling the *in vitro* transcription products with Cy3 or Cy5 dye for use in hybridizing the labeled cDNA samples to microarrays.

The kit optionally includes instructions for using the kit in the selective amplification of mRNA targets. The kit can also be optionally provided with instructions for *in vitro* transcription of the amplified cDNA molecules, and with instructions for labeling and hybridizing the *in vitro* transcription products to microarrays.

In another aspect, the present invention provides methods of selectively amplifying a target population of nucleic acid molecules to generate amplified RNA molecules. The method comprises: (a) providing a population of oligonucleotides, wherein each oligonucleotide comprises a hybridizing portion and transcriptional promoter portion located 5' to the hybridizing portion, wherein the hybridizing portion is a member of the population of oligonucleotides comprising SEQ ID NOS:1-933, (b) annealing the population of oligonucleotides to a sample comprising mRNA isolated from a mammalian subject, (c) synthesizing cDNA from the mRNA using a reverse transcriptase enzyme, (d)synthesizing double stranded cDNA using a DNA polymerase; and (e) transcribing the double-stranded cDNA into RNA using an RNA polymerase that binds to the transcriptional promoter portion of each oligonucleotide to generate amplified RNA. FIGURE 1C shows a representative embodiment of the methods according to this aspect of the invention. The methods and reagents described herein are useful in the practice of this aspect of the invention.

In another aspect, the present invention provides methods of selectively amplifying a target population of nucleic acid molecules to generate amplified DNA molecules. The method comprises: (a) providing a first population of oligonucleotides, wherein each oligonucleotide comprises a hybridizing portion and a first PCR primer binding site located 5' to the hybridizing portion, wherein the hybridizing portion is a member of the population of oligonucleotides comprising SEQ ID NOS:1-933, (b) annealing the population of oligonucleotides to a sample comprising mRNA isolated from a mammalian subject, (c)synthesizing cDNA from the mRNA using a reverse transcriptase enzyme, (d) synthesizing double stranded cDNA using a DNA polymerase and a second population of oligonucleotides, wherein each oligonucleotide comprises a random hybridizing portion and a second PCR binding site located 5' to the hybridizing portion, and (e) PCR amplifying the double stranded cDNA using thermostable DNA polymerase, a first PCR primer that binds to the first PCR primer binding site and a second PCR primer that binds to the second PCR primer binding site to generate amplified double stranded DNA. FIGURE 1D illustrates a representative embodiment of the methods of this aspect of the invention.

In accordance with this aspect of the invention, any DNA-dependent DNA polymerase may be utilized to synthesize second-strand DNA molecules from the first strand cDNA. For example, the Klenow fragment of DNA Polymerase I can be utilized to synthesize the second-strand DNA molecules. The synthesis of second-strand DNA molecules is primed using a second population of oligonucleotides comprising a hybridizing portion consisting of from 6 to 9 random nucleotides and further comprising a defined sequence portion 5' to the hybridizing portion. The defined sequence portion may include any suitable sequence, provided that the sequence differs from the defined sequence contained in the first population of oligonucleotides. Depending on the choice of primer sequence, these defined sequence portions can be used, for example, to selectively direct DNA-dependent RNA synthesis from the second DNA molecule and/or to amplify the double-stranded cDNA template via DNA-dependent DNA synthesis.

Purification of double-stranded DNA molecules: Synthesis of the second DNA molecules yields a population of double-stranded DNA molecules wherein the first DNA molecules are hybridized to the second DNA molecules, as shown in FIGURE 1D. Typically, the double-stranded DNA molecules are purified to remove substantially all nucleic acid molecules shorter than 50 base pairs, including all, or substantially all (i.e., typically more than 99%), of the second primers. Preferably, the purification method selectively purifies DNA molecules that are substantially double-stranded and removes substantially all unpaired, single-stranded nucleic acid molecules, such as single-stranded primers. Purification can be achieved by any art-recognized means, such as by elution through a size-fractionation column. The purified, second DNA molecules can then, for example, be precipitated and redissolved in a suitable buffer for the next step of the methods of this aspect of the invention.

Amplification of the double-stranded DNA molecules. In the practice of the methods of this aspect of the invention, the double-stranded DNA molecules are utilized as templates that are enzymatically amplified using the polymerase chain reaction. Any suitable primers can be used to prime the polymerase chain reaction. Typically two primers are used, one primer hybridizes to the defined portion of the first primer sequence (or to the complement thereof); and the other primer hybridizes to the defined portion of the second primer sequence (or to the complement thereof).

PCR Amplification Conditions. In general, the greater the number of amplification cycles during the polymerase chain reaction, the greater the amount of amplified DNA that is obtained. On the other hand, too many amplification cycles may result in randomly biased amplification of the double-stranded DNA. Thus, in some embodiments, a desirable number of amplification cycles is between 5 and 40 amplification cycles, such as from five to 35, such as from 10 to 30 amplification cycles.

With regard to temperature conditions, typically a cycle comprises a melting temperature, such as 95°C; an annealing temperature, which varies from about 40°C to 70°C; and an elongation temperature, which is typically about 72°C. With regard to the annealing temperature, in some embodiments the annealing temperature is from about 55°C to 65°C, more preferably about 60°C.

In one embodiment, amplification conditions for use in this aspect of the invention comprise 10 cycles of (95°C, 30 sec; 60°C, 30 sec; 72°C, 60 sec), then 20 cycles of (95°C, 30 sec; 60°C, 30 sec, 72°C, 60 sec (+ 10 sec added to the elongation step with each cycle)).

With regard to PCR reaction components for use in the methods of this aspect of the invention, dNTPs are typically present in the reaction in a range from 50 µM to 2000 µM dNTPs, and more preferably from 800 to 1000 µM. MgCl₂ is typically present in the reaction in a range from 0.25 mM to 10 mM, and more preferably about 4 mM. The forward and reverse PCR primers are typically present in the reaction from about 50 nM to 2000 nM, and more preferably present at a concentration of about 1000 nM.

DNA Labeling. Optionally, the amplified DNA molecules can be labeled with a dye molecule to facilitate use as a probe in a hybridization experiment, such as a probe used to screen a DNA chip. Any suitable dye molecules can be utilized, such as fluorophores and chemiluminescers. An exemplary method for attaching the dye molecules to the amplified DNA molecules is provided in EXAMPLE 15.

The methods according this aspect of the invention may be used, for example, for transcriptome profiling in a biological sample containing total RNA, such as whole blood. In some embodiments, the amplified DNA produced in accordance with the methods of this aspect of the invention is labeled for use in gene expression experiments, thereby providing a hybridization based reagent that typically produces a lower level of background than amplified RNA generated from NSR-primed cDNA.

In some embodiments of this aspect of the invention, the defined sequence portion of the first and/or second primer binding regions further includes one or more restriction enzyme sites, thereby generating a population of amplifed double-stranded DNA products having one or more restriction enzyme sites flanking the amplified portions. These amplified products may be used directly for sequence analysis, or may be released by digestion with restriction enzymes and subcloned into any desired vector, such as an expression vector for further analysis.

The following examples merely illustrate the best mode now contemplated for practicing the invention, but should not be construed to limit the invention.

### EXAMPLE 1

This Example describes the selection of a population of 933 6-mer oligonucleotides (SEQ ID NOS:1-933) that hybridizes to all, or substantially all, mRNA molecules expressed in blood cells, but that does not hybridize to globin mRNA or to ribosomal RNA.

All 4096 possible 6-mer oligonucleotides were computed, wherein each nucleotide was A, T (or U), C, or G. The reverse complement of each 6-mer oligonucleotide was compared to the nucleotide sequences of 18S and 28S rRNAs, and to the nucleotide sequences of the following six hemoglobin genes, selected based on their high level of expression in blood samples:

**TABLE 1.**

| **Gene Symbol** | **NCBI Reference Sequence Transcript Identifier** |
|---|---|
| HBA1 | NM_000558.3 |
| HBA2 | NM_000517.3 |
| HBB | NM_000518.4 |
| HBD | NM_000519.2 |
| HBG1 | NM_000559.2 |
| HBG2 | NM_000184.2 |

Reverse-complement 6-mer oligonucleotides having perfect matches to any of the eight transcript sequences were eliminated. The reverse complements of 933 6-mers (SEQ ID NOS:1-933) did not perfectly match any portion of the globin or rRNA transcripts. The 933 6-mer oligonucleotides (SEQ ID NOS:1-933) that do not have a perfect match to any portion of the globin or rRNA genes are referred to as "Not-So-Random" ("NSR") primers.

### EXAMPLE 2

This Example shows that the population of oligonucleotides having the nucleic acid sequences set forth in SEQ ID NOS: 1-933 hybridize to every 4 to 5 nucleotides on a nucleic acid sequence within the RefSeq database accessible at the website of the National Center for Biotechnology Information (NCBI), U.S. National Library of Medicine, 8600 Rockville Pike, Bethesda, MD 20894, U.S.A. NCBI's reference sequence transcript database (RefSeq) contains what is considered a gold-standard of human protein coding transcripts.

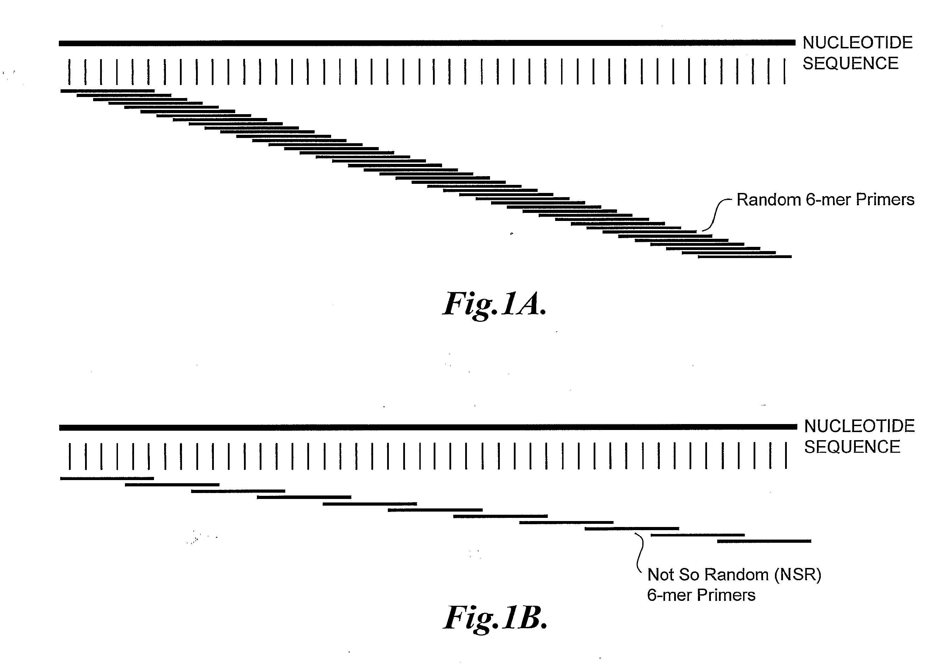

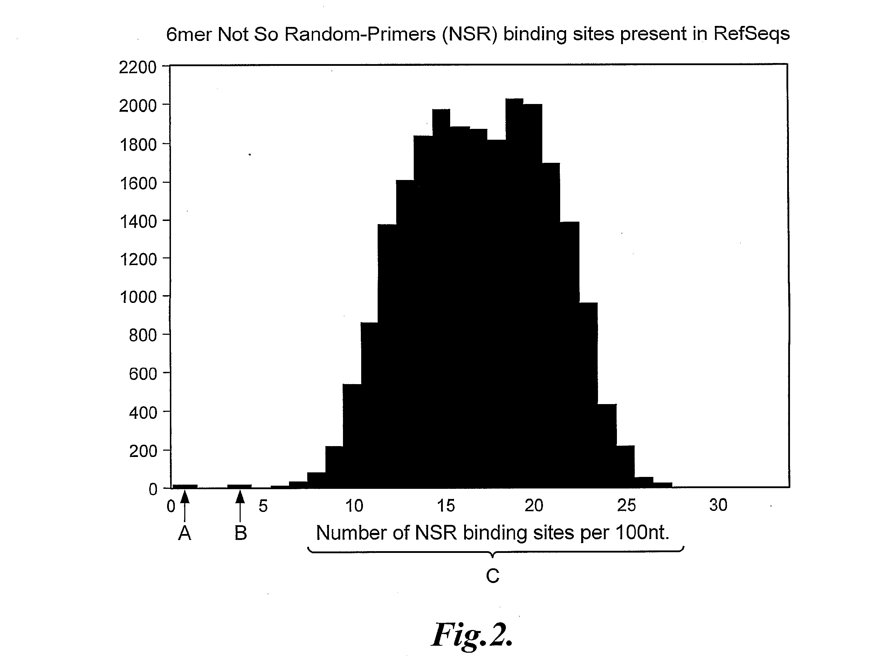

Random 6-mers (N6) can anneal at every nucleotide position on a transcript sequence from the RefSeq database (represented as "nucleotide sequence"), as shown in FIGURE 1A. After subtracting out the 6-mers whose reverse complements are a perfect match to ribosomal RNAs and globin mRNAs, the remaining NSR oligonucleotides (SEQ ID NOS:1-933) show a perfect match to every 4 to 5 nucleotides on nucleic acid sequences within the RefSeq database (represented as "nucleotide sequence"), as shown in FIGURE 1B. As shown in FIGURE 2, NSR oligonucleotide binding sites are not present in the hemoglobin genes (represented as "A" in FIGURE 2). One atypical gene family (represented as "B" in FIGURE 2) consisting of 3 genes (LCE1A, CLE1D, LCE1F) contains only four NSR 6-mer binding sites per 100 nucleotides). However, RefSeq transcripts typically have anywhere from 5 to 30 NSR oligonucleotide (SEQ ID NOS:1-933) binding sites per 100 nucleotides, with most transcripts having 15-20 NSR binding sites per 100 nucleotides (represented as "C" in FIGURE 2).

Thus, the population of 933 6-mers (SEQ ID NOS:1-933) is capable of amplifying all transcripts except 18S, 28S, and hemoglobin transcripts.

### EXAMPLE 3

This Example shows that PCR amplification of an actin reporter mRNA using the 933 6-mers (SEQ ID NOS:1-933) (that each have the T7 promoter (SEQ ID NO:934) covalently attached at the 5' end) selectively reduces priming of globin mRNA and rRNA.

Total RNA was extracted from individual donors of human whole blood using the PAXgene Blood RNA Kit (Qiagen, Inc., Valencia, CA) according to the manufacturer's instructions. MMLV (Moloney Murine Leukemia Virus) reverse transcriptase (Epicentre Biotechnologies, Madison, WI) was used to synthesize cDNA from 100 ng of template RNA with 5 µM 6-mers (SEQ ID NOS:1-933) (T7-NSR6) or random 9-mers with the T7 promoter covalently attached at the 5' end (T7-N9). Prior to reverse transcription, 5 µL of water containing primers and template were denatured at 65°C for 5 min, snap cooled at 4°C and equilibrated to 23°C. For reverse transcription, 5 µL of RT master mix containing 1 µl of water, 2 µl of 5X First Strand Buffer (250 mM Tris-HCl, pH 8.3; 375 mM KCl; 15 mM MgCl₂; Invitrogen Corporation, Carlsbad, CA), 0.5 µl of 100 mM DTT, 0.5 µl of 10 mM dNTPs and 1.0 µl of MMLV reverse transcriptase (50 units/µl) was added to the sample mix. The 10 µl reaction was incubated at 40°C for 120 min, 95°C for 5 min, cooled to room temperature, and diluted 5-fold with water.

Following reverse transcription, duplicate measurements of 2 µl of cDNA were made in 10 µl final reaction volumes by quantitative PCR (qPCR) in a 384-well optical PCR plate using a 7900 HT PCR instrument (Applied Biosystems, Foster City, CA). qPCR was performed using ABI TaqMan® assays (eukaryotic 18S rRNA, ABI Catalog Hs99999901 s1; human beta actin, ABI Catalog No. 4310881E; human beta hemoglobin, ABI Catalog No. Hs00747223_gl; ABI Assay-by-Design alpha globin; (fwd 5'-GCACGCGCACAAGCT-3' (SEQ ID NO:937), reverse 5'-GGGTCACCAGCAGGCA-3' (SEQ ID NO:938), FAM probe 5'-ACTTCAAGCTCCTAAGCCAC-3' (SEQ ID NO:939)) using the manufacturer's recommended conditions. Following PCR, the results table was exported to Excel (Microsoft Corp., Redmond, WA) and quantitative analysis for samples was regressed from the raw data (abundance = 10[(Ct-5)/-3.4]).

Reverse transcription of human whole blood total RNA with the 933 NSR 6-mers (SEQ ID NOS: 1-933) resulted in a significant reduction of globin and rRNA content in cDNA as determined by qPCR. Specifically, T7-NSR 6 primed cDNA showed reduced abundance of alpha globin (58% reduction), beta globin (85% reduction) and 18S rRNA (75% reduction) as compared to cDNA generated with random 9-mers (T7-N9).

As shown below in TABLE 2, T7-NSR6 primed cDNA showed reduced abundance of beta globin (HBB) (94% reduction), and 18S rRNA (91% reduction), as compared to cDNA generated with random 9-mers. In contrast, beta actin cDNA levels for the two primer pools (T7-N9 and T7-NSR6) were comparable after amplification of RNA, a result that was confirmed using three independent donors (data not shown).

**TABLE 2. Subtractive Amplification of total RNA from whole blood.**

| | **microarray signal** | | |
|---|---|---|---|
| **Primer** | **hemoglobin mRNA (HBB)** | **ribosomal 18S rRNA** | **Actin mRNA** |
| T7-dT | not determined | 6,332,672 | not determined |
| T7-N9 | 2,072,315 | 2,627,237 | 188 |
| NSR-6-mer | 111,954 | 215,776 | 102 |

### EXAMPLE 4

This Example shows that the 933 6-mers (SEQ ID NOS:1-933) (that each have the T7 promoter (SEQ ID NO:934) covalently attached at the 5' end) prime the amplification of a substantial fraction of the transcriptome.

To assess cDNA complexity, 933 6-mers (SEQ ID NOS:1-933) were used to amplify total RNA from two diverse cell lines (Jurkat and K562) using a two-step reverse transcription and *in vitro* transcription (IVT) approach. The amplification products were labeled and hybridized to a DNA microarray containing probes for ∼5000 human transcripts known to be expressed in either Jurkat or K562 cells. Nearly 82% (4244/5198) of the transcripts reported above background signal intensity (log intensity ≥-1.5) in either cell line. Moreover, the Jurkat/K562 expression profile from RNA amplified using the 933 6-mers (SEQ ID NOS:1-933) showed a high degree of similarity to a control profile amplified from random-primed Jurkat and K562 mRNA (for probes with a p-value < 0.01 in either experiment; correlation coefficient = 0.72).

The amplification experiment was conducted as follows. Total RNA and mRNA were obtained from Ambion, Inc. (Austin, TX) for the cell lines Jurkat (T lymphocyte, ATCC No. TIB-152) and K562 (chronic myelogenous leukemia, ATCC No. CCL-243). A two-step amplification approach using reverse transcription and *in vitro* transcription was used to generate amplified RNA (aRNA) for microarray hybridizations. NSR cDNA was synthesized from 1 µg of total RNA and 5 µM primer in a 20 µL reaction volume as described in Example 3, but with a prolonged incubation step at 40°C for 6 hrs. cDNA was synthesized from 100 ng of mRNA using 10 µM random 9-mer in a 20 µL reaction volume as described in Example 3. The 933 6-mers (SEQ ID NOS:1-933) and the random 9-mers were covalently linked to the T7 promoter sequence (SEQ ID NO:934) at the 5' end.

For *in vitro* transcription, the 20 µL RT reaction was added to 60 µL of IVT pre-mix containing 16 µL of 5X Transcription Buffer (0.2 M Tris-HCl, pH 7.5, 50 mM NaCl, 30 mM MgCl₂, and 10 mM spermidine; Epicentre Biotechnologies, Madison, WI), 6 µL of 100 mM DTT, 3.3 µL of 200 mM MgCl₂, 8 µL of NTP (25 mM ATP, 25 mM CTP, 25 mM GTP, 6 mM UTP), 2 µL of 75 mM amino allyl-UTP, 0.6 µL of IPPase (2 U/µL), 0.08 µL of T7 RNA polymerase (2.5 kU/µL), 24 µL of water and incubated for 16 hrs at 40°C, 5 min at 70°C, and cooled to room temperature. The final product was coupled to Cy3 or Cy5 dye in 1 M bicarbonate buffer for 1 h. Reactions were finished with the addition of 4 M hydroxylamine followed by purification with RNAeasy Spin Columns. The percentage dye incorporation and total cDNA yield were determined spectrophotometrically. Pairs of Cy3/Cy5-labeled cDNA samples were combined and hybridized to microarrays as described previously (Roberts et al., "Signaling and Circuitry of Multiple MAPK Pathways Revealed by a Matrix of Global Gene Expression Profiles," Science 287:873-880, 2000). Arrays were hybridized for 48 hrs then washed and scanned on Agilent Microarray Scanners.

Custom high density oligonucleotide arrays were obtained from Agilent Technologies (Palo Alto, CA). A total of 5198 transcripts were chosen for array probe design from previously described experiments (Hughes et al., "Expression profiling using microarrays fabricated by an ink-jet oligonucleotide synthesizer," Nat. Biotechnol. 19(4):342-347, 2001). Probe sequences (60-mers) were selected on the basis of nucleotide composition and cross-hybridization potential, and positioned at least 500 bp upstream of the known polyadenylation site for each transcript. Data analysis was carried out as previously described (Hughes et al., *supra*).

### EXAMPLE 5

This example shows that increasing the concentration of deoxyribonucleoside triphosphates (dNTPs) in the first strand synthesis reaction increases the amplification of IGF1R and GAPDH reporter mRNA using NSR primers (comprising SEQ ID NOS:1-933) (that each have the T7 promoter (SEQ ID NO:934) covalently attached at the 5' end), relative to globin mRNA and rRNA.

### Methods:

NSR primers were used in this Example as follows:
The 933 6-mers (SEQ ID NOS:1-933) plus a single random nucleotide inserted between the 6-mer sequence and the T7 promoter (SEQ ID NO:934), referred to as "NSR7."
Total RNA was extracted from human whole blood as described in Example 3. MMLV reverse transcriptase (SuperScript III™ ("SSIII"), Invitrogen Corporation, Carlsbad, CA) was used to synthesize cDNA from 200 ng of template RNA with 10 µM 6-mers (SEQ ID NOS:1-933) or random 8-mers (N8). First strand synthesis was performed as described in Example 3, except that the reaction volume was 20 µL, 2 µL SSIII reverse transcriptase (200 units/µl) was added to the reaction mix, and the concentration of dNTPs varied from 500 to 5,000 µM. The reaction was incubated at 40°C for 120 min, 70°C for 15 min, cooled to room temperature, and diluted two-fold with 10 mM Tris pH 7.6, 0.1 mM EDTA.

Following reverse transcription, qPCR was performed as described in Example 3, except that ABI TaqMan assays for human insulin growth factor one receptor (IGFR1R) (ABI Catalog # Hs00181385) and human glyceraldehyde-3-phosphate dehydrogenase (GAPDH) (ABI Catalog # 43100884E-0507028) were substituted for human beta actin as reporters. Following PCR, the results table was exported to Excel and quantitative analysis for samples was regressed from the raw data as described in Example 3.

Reverse transcription of human whole blood total RNA with the NSR7 primers (comprising SEQ ID NOS:1-933) and increasing concentrations of dNTPs resulted in increased specificity of the NSR7-primed reaction. The data from two independent experiments is shown in Tables 1-5 below. The raw abundance values, expressed as 10 raised to the (Cts x log10(1/2) +10) power (Tables 3 and 4), were normalized to the highest N8 value for a given target gene (Tables 5 and 6).

**TABLE 3. Raw abundance values vs. dNTP concentration. Values expressed as 10 raised to the (Cts x log10(1/2) +10) power.**

| | **[dNTPs]µM** | **500** | **1000** | **1500** | **2000** | **2500** | **3000** | **4000** | **5000** |
|---|---|---|---|---|---|---|---|---|---|
| **Target Gene** | **Primer set** | | | | | | | | |
| IGF1R | NSR | 30 | 36 | 28 | 21 | 14 | 9 | 2 | 0 |
| | N8 | 82 | 68 | 76 | 61 | 49 | 29 | 15 | 5 |
| GAPDH | NSR | 428 | 519 | 399 | 361 | 281 | 263 | 56 | 1 |
| | N8 | 1009 | 857 | 1000 | 649 | 567 | 302 | 167 | 6 |
| 18S | NSR | 22576 | 19812 | 7255 | 1843 | 408 | 30 | 0 | 0 |
| | N8 | 377536 | 466002 | 584331 | 291882 | 103635 | 9602 | 26 | 0 |
| 28S | NSR | 162393 | 27816 | 9910 | 1516 | 243 | 40 | 2 | 2 |
| | N8 | 613070 | 148408 | 110193 | 59667 | 12571 | 2558 | 33 | 1 |
| HBA | NSR | 1689 | 1450 | 759 | 607 | 341 | 226 | 168 | 37 |
| | N8 | 8963 | 7271 | 11116 | 12151 | 7088 | 6578 | 1815 | 1241 |
| HBB | NSR | 1473 | 1128 | 588 | 528 | 423 | 373 | 270 | 0 |
| | N8 | 2838 | 3574 | 6312 | 4588 | 3276 | 2513 | 962 | 255 |

**TABLE 4. Raw abundance values vs. dNTP concentration. Values expressed as 10 raised to the (Cts x log10(1/2) +10) power.**

| | **[dNTPs] µM** | **500** | **1000** | **1500** | **2000** | **2500** | **3000** | **4000** | **5000** |
|---|---|---|---|---|---|---|---|---|---|
| **Target Gene** | **Primer set** | | | | | | | | |
| IGF1R | NSR | 201 | 177 | 154 | 110 | 71 | 45 | 7 | 1 |
| | N8 | 495 | 230 | 259 | 211 | 215 | 152 | 73 | 25 |
| GAPDH | NSR | 288 | 405 | 350 | 270 | 229 | 221 | 61 | 7 |
| | N8 | 530 | 490 | 424 | 359 | 329 | 265 | 93 | 16 |
| 18S | NSR | 605722 | 393759 | 170366 | 60940 | 26468 | 4076 | 8 | 1 |
| | N8 | 3338794 | 2685092 | 2257580 | 1149677 | 589425 | 46427 | 137 | 0 |
| 28S | NSR | 622085 | 71670 | 34636 | 19885 | 6064 | 581 | 4 | 0 |
| | N8 | 1346589 | 1310318 | 1744481 | 970265 | 412149 | 63991 | 1236 | 1 |
| HBA | NSR | 5047 | 2114 | 1125 | 1395 | 884 | 531 | 235 | 80 |
| | N8 | 44790 | 35305 | 95774 | 158007 | 447716 | 540325 | 214874 | 73825 |
| HBB | NSR | 5420 | 1937 | 1484 | 1774 | 1672 | 1577 | 2448 | 4182 |
| | N8 | 29146 | 24501 | 22769 | 24853 | 21620 | 11380 | 1710 | 477 |

**TABLE 5. Abundance values normalized to N8.**

| | **[dNTPs] µM** | **500** | **1000** | **1500** | **2000** | **2500** | **3000** | **4000** | **5000** |
|---|---|---|---|---|---|---|---|---|---|
| **Target RNA** | **Primer set** | | | | | | | | |
| IGF1R | NSR | 44 | 53 | 41 | 31 | 20 | 13 | 2 | 0 |
| | N8 | 120 | 100 | 112 | 90 | 72 | 43 | 22 | 7 |
| GAPDH | NSR | 50 | 61 | 47 | 42 | 33 | 31 | 7 | 0 |
| | N8 | 118 | 100 | 117 | 76 | 66 | 35 | 20 | 1 |
| 18S | NSR | 5 | 4 | 2 | 0 | 0 | 0 | 0 | 0 |
| | N8 | 81 | 100 | 125 | 63 | 22 | 2 | 0 | 0 |
| 28S | NSR | 109 | 19 | 7 | 1 | 0 | 0 | 0 | 0 |
| | N8 | 413 | 100 | 74 | 40 | 8 | 2 | 0 | 0 |
| HBA | NSR | 23 | 20 | 10 | 8 | 5 | 3 | 2 | 1 |
| | N8 | 123 | 100 | 153 | 167 | 97 | 90 | 25 | 17 |
| HBB | NSR | 41 | 32 | 16 | 15 | 12 | 10 | 8 | 0 |
| | N8 | 79 | 100 | 177 | 128 | 92 | 70 | 27 | 7 |

**TABLE 6. Abundance normalized to N8.**

| **[dNTPs]** | | **500** | **1000** | **1500** | **2000** | **2500** | **3000** | **4000** | **5000** |
|---|---|---|---|---|---|---|---|---|---|
| IGF1R | NSR | 41 | 36 | 31 | 22 | 14 | 9 | 1 | 0 |
| | N8 | 100 | 46 | 52 | 43 | 44 | 31 | 15 | 5 |
| GAPDH | NSR | 54 | 77 | 66 | 51 | 43 | 42 | 11 | 1 |
| | N8 | 100 | 93 | 80 | 68 | 62 | 50 | 17 | 3 |
| 18S | NSR | 18 | 12 | 5 | 2 | 1 | 0 | 0 | 0 |
| | N8 | 100 | 80 | 68 | 34 | 18 | 1 | 0 | 0 |
| 28S | NSR | 46 | 5 | 3 | 1 | 0 | 0 | 0 | 0 |
| | N8 | 100 | 97 | 130 | 72 | 31 | 5 | 0 | 0 |
| HBA | NSR | 11 | 5 | 3 | 3 | 2 | 1 | 1 | 0 |
| | N8 | 100 | 79 | 214 | 353 | 1000 | 1206 | 480 | 165 |
| HBB | NSR | 19 | 7 | 5 | 6 | 6 | 5 | 8 | 14 |
| | N8 | 100 | 84 | 78 | 85 | 74 | 39 | 6 | 2 |

The ratios of reporters to backgrounds (reporter/background), which is the ratio of IGF1R and GAPDH reporters to 18S rRNA, 28S rRNA, alpha globin (HBA) and beta globin (HBB) background values, were then calculated for the NSR7 and N8 primer pools. As shown in Tables 7 and 8, the NSR7-primed reporter/background ratio increased dramatically with increasing dNTP concentrations.

**TABLE 7. Ratios of Reporters to Background.**

| | **[dNTPs] µM** | **500** | **1000** | **1500** | **2000** | **2500** | **3000** | **4000** | **5000** |
|---|---|---|---|---|---|---|---|---|---|
| **Reporter/ Background** | **Primer set** | | | | | | | | |
| IGF1R/18S | NSR | 9 | 12 | 26 | 78 | 228 | 1963 | 39729 | 2974 |
| | N8 | 1 | 1 | 1 | 1 | 3 | 21 | 3872 | 72108 |
| IGF1R/28S | NSR | 0 | 3 | 6 | 30 | 122 | 463 | 1701 | 101 |
| | N8 | 0 | 1 | 2 | 2 | 9 | 25 | 986 | 13040 |
| IGF1R/HBA | NSR | 2 | 3 | 4 | 4 | 4 | 4 | 1 | 0 |
| | N8 | 1 | 1 | 1 | 1 | 1 | 0 | 1 | 0 |
| IGF1R/HBB | NSR | 1 | 2 | 2 | 2 | 2 | 1 | 0 | 12 |
| | N8 | 2 | 1 | 1 | 1 | 1 | 1 | 1 | 1 |
| GAPDH/18S | NSR | 10 | 14 | 30 | 107 | 374 | 4786 | 111747 | 1211 |
| | N8 | 1 | 1 | 1 | 1 | 3 | 17 | 3436 | 7399 |
| GAPDH/28S | NSR | 0 | 3 | 7 | 41 | 200 | 1129 | 4783 | 41 |
| | N8 | 0 | 1 | 2 | 2 | 8 | 20 | 875 | 1338 |
| GAPDH/HBA | NSR | 2 | 3 | 4 | 5 | 7 | 10 | 3 | 0 |
| | N8 | 1 | 1 | 1 | 0 | 1 | 0 | 1 | 0 |
| GAPDH/HBB | NSR | 1 | 2 | 3 | 3 | 3 | 3 | 1 | 5 |
| | N8 | 1 | 1 | 1 | 1 | 1 | 1 | 1 | 0 |

**TABLE 8: Ratio of reporters to backgrounds.**

| | **[dNTPs] µM** | **500** | **1000** | **1500** | **2000** | **2500** | **3000** | **4000** | **5000** |
|---|---|---|---|---|---|---|---|---|---|
| IGF1R/18S | NSR | 2 | 3 | 6 | 12 | 18 | 74 | 6090 | 5194 |
| | N8 | 1 | 1 | 1 | 1 | 2 | 22 | 3594 | 2356335 |
| IGF1R/28S | NSR | 1 | 7 | 12 | 15 | 32 | 211 | 5197 | 6174 |
| | N8 | 1 | 0 | 0 | 1 | 1 | 6 | 160 | 51368 |
| IGF1R/HBA | NSR | 4 | 8 | 12 | 7 | 7 | 8 | 3 | 1 |
| | N8 | 1 | 1 | 0 | 0 | 0 | 0 | 0 | 0 |
| IGF1R/HBB | NSR | 2 | 5 | 6 | 4 | 3 | 2 | 0 | 0 |
| | N8 | 1 | 1 | 1 | 0 | 1 | 1 | 3 | 3 |
| GAPDH/18S | NSR | 3 | 6 | 13 | 28 | 55 | 342 | 49034 | 42162 |
| | N8 | 1 | 1 | 1 | 2 | 4 | 36 | 4275 | 1374635 |
| GAPDH/28S | NSR | 1 | 14 | 26 | 35 | 96 | 967 | 41850 | 50116 |
| | N8 | 1 | 1 | 1 | 1 | 2 | 11 | 191 | 29967 |
| GAPDH/HBA | NSR | 5 | 16 | 26 | 16 | 22 | 35 | 22 | 7 |
| | N8 | 1 | 1 | 0 | 0 | 0 | 0 | 0 | 0 |
| GAPDH/HBB | NSR | 3 | 12 | 13 | 8 | 8 | 8 | 1 | 0 |
| | N8 | 1 | 1 | 1 | 1 | 1 | 1 | 3 | 2 |

The ratios of ratios is a comparison of reporter to background ratios for the NSR7 and N8 primer pools, and measures the specificity of the NSR-primed reactions. As shown in Table 9 and Table 10, the ratios of ratios increased dramatically at higher dNTP concentrations.

**TABLE 9. Ratios of Ratios (Reporter/Background versus NSR7/N8).**

| | **[dNTPs] µM** | **500** | **1000** | **1500** | **2000** | **2500** | **3000** | **4000** | **5000** |
|---|---|---|---|---|---|---|---|---|---|
| **Reporter/ Background** | **Primer Ratio** | | | | | | | | |
| IGF1R/18S | NSR/N8 | 6 | 12 | 29 | 54 | 70 | 94 | 10 | 0 |
| IGF1R/28S | NSR/N8 | 1 | 3 | 4 | 14 | 14 | 19 | 2 | 0 |
| IGF1R/HBA | NSR/N8 | 2 | 3 | 5 | 7 | 6 | 9 | 1 | 1 |
| IGF1R/HBB | NSR/N8 | 1 | 2 | 4 | 3 | 2 | 2 | 0 | 13 |
| GAPDH/18S | NSR/N8 | 7 | 14 | 32 | 88 | 126 | 280 | 33 | 0 |
| GAPDH/28S | NSR/N8 | 2 | 3 | 4 | 22 | 26 | 55 | 5 | 0 |
| GAPDH/HBA | NSR/N8 | 2 | 3 | 6 | 11 | 10 | 25 | 4 | 3 |
| GAPDH/HBB | NSR/N8 | 1 | 2 | 4 | 5 | 4 | 6 | 1 | 51 |

**TABLE 10. Ratios of Ratios (Reporter/Background versus NSR/N8).**

| **[dNTPs] µM** | **500** | **1000** | **1500** | **2000** | **2500** | **3000** | **4000** | **5000** |
|---|---|---|---|---|---|---|---|---|
| IGF1R/18S NSR/N8 | 2 | 5 | 8 | 10 | 7 | 3 | 2 | 0 |
| IGF1R/28S NSR/N8 | 1 | 14 | 30 | 26 | 22 | 32 | 32 | 0 |
| IGF1R/HBA NSR/N8 | 4 | 13 | 51 | 59 | 167 | 300 | 88 | 28 |
| IGF1R/HBB NSR/N8 | 2 | 10 | 9 | 7 | 4 | 2 | 0 | 0 |
| GAPDH/18S NSR/N8 | 3 | 6 | 11 | 14 | 16 | 10 | 11 | 0 |
| GAPDH/28S NSR/N8 | 1 | 15 | 42 | 37 | 47 | 92 | 220 | 2 |
| GAPDH/HBA NSR/N8 | 5 | 14 | 70 | 85 | 353 | 850 | 599 | 392 |
| GAPDH/HBB NSR/N8 | 3 | 10 | 13 | 11 | 9 | 6 | 0 | 0 |

The yield ratio measures the output of cDNA conversion primed by NSR7 versus N8, expressed as a percentage (NSR divided by N8). As shown in Table 11 and Table 12, the NSR to N8 yield ratio was substantially higher for the IGF1R and GAPDH reporters than for the rRNA and globin mRNA. The ratio was relatively constant across dNTP concentrations for the IGF1R and GAPDH reporters, but decreased with increasing dNTP concentrations for the rRNA and globin genes, consistent with enrichment of reporters at higher dNTP concentrations.

**TABLE 11. Yield Ratios of NSR/N8.**

| | **[dNTPs] µM** | **500** | **1000** | **1500** | **2000** | **2500** | **3000** | **4000** | **5000** |
|---|---|---|---|---|---|---|---|---|---|
| **Gene** | | | | | | | | | |
| IGF1R | NSR/N8 | 37 | 53 | 36 | 34 | 28 | 29 | 11 | 2 |
| GAPDH | NSR/N8 | 42 | 61 | 40 | 56 | 49 | 87 | 34 | 9 |
| 18S | NSR/N8 | 6 | 4 | 1 | 1 | 0 | 0 | 1 | 57 |
| 28S | NSR/N8 | 26 | 19 | 9 | 3 | 2 | 2 | 6 | 306 |
| HBA | NSR/N8 | 19 | 20 | 7 | 5 | 5 | 3 | 9 | 3 |
| HBB | NSR/N8 | 52 | 32 | 9 | 11 | 13 | 15 | 28 | 0 |

**TABLE 12: Yields of NSR/N8.**

| **[dNTPs] µM** | **500** | **1000** | **1500** | **2000** | **2500** | **3000** | **4000** | **5000** |
|---|---|---|---|---|---|---|---|---|
| IGF1R NSR/N8 | 41 | 77 | 59 | 52 | 33 | 29 | 10 | 3 |
| GAPDH NSR/N8 | 54 | 83 | 83 | 75 | 70 | 83 | 66 | 43 |
| 18S NSR/N8 | 18 | 15 | 8 | 5 | 4 | 9 | 6 | 1393 |
| 28S NSR/N8 | 46 | 5 | 2 | 2 | 1 | 1 | 0 | 26 |
| HBA NSR/N8 | 11 | 6 | 1 | 1 | 0 | 0 | 0 | 0 |
| HBB NSR/N8 | 19 | 8 | 7 | 7 | 8 | 14 | 143 | 877 |

In summary, this example shows that the absolute yields of cDNA primed with the NSR7 primers (comprising SEQ ID NOS:1-933) were highest at low dNTP concentrations, whereas the specificity relative to random 8-mers increased at higher dNTP concentrations. Based on this data, it appears that the optimal concentration of dNTPs to achieve high yield and high specificity is in the range of about 1000 to about 2000 µM dNTPs.

### EXAMPLE 6

This Example shows that NSR primers (comprising SEQ ID NOS:1-933), that each have the T7 promoter (SEQ ID NO:934) covalently attached at the 5' end, maintain specificity when low amounts of template RNA is reverse transcribed and amplified by *in vitro* transcription (IVT).

In initial experiments, it was determined that the NSR7-primed specificity was lost during the second strand synthesis and IVT amplification steps when low amounts of template RNA were used. The IVT amplification step is required to produce enough RNA for the microarray experiments disclosed above.

It was observed that the NSR7-primed specificity from low amounts of template RNA was improved by using the SuperScript™ RNA Amplification System (Invitrogen, Catalog No. L1016-01), as follows:
Methods: First strand cDNA synthesis was performed as described in Example 5 using 1000 µM dNTPs, and 50 ng, 100 ng, and 200 ng of whole blood total RNA template with the NSR7 primer pool. 100 ng of RNA template was used with random 7-mers that each have the T7 promoter (SEQ ID NO:934) covalently attached at the 5' end (T7-N7) and random 8-mer (N8) primers. For second strand cDNA synthesis, 20 µL of the first strand reaction product was added to 130 µL of second strand mix containing 91 µL water, 30 µL 5X Second-Strand Reaction Buffer (100 mM Tris-HCl (pH 6.9), 450 mM KCl, 23 mM MgCl₂, 0.75 mM β-NAD⁺, 50 mM (NH₄)₂SO₄) 3 µL 10 mM dNTPs, 4 µL DNA Polymerase I (10 units/µl), 1 µL DNA ligase (10 units/µl) and 1 µL RNase H (2 units/µl). The reaction was incubated at 16°C for 120 min. The double stranded cDNA was purified using the Spin Cartridge and buffers supplied in the kit (Invitrogen), and the volume of the eluted cDNA was reduced to less than 20 µL by centrifugation under vacuum with low to moderate heat.

The double stranded cDNA template was amplified by *in vitro* transcription using T7 polymerase to transcribe antisense RNA (aRNA) complementary to the original mRNA targets. For IVT, the volume of the purified cDNA was adjusted to 23 µL with water, added to 17 µL of IVT reaction mix containing 4 µL 10X T7 Reaction Buffer (Invitrogen), 7 µL of T7 Enzyme Mix (includes T7 RNA polymerase), 8 µL NTP (25 mM each of ATP, CTP, GTP, and UTP), and incubated at 37°C for 6 to 16 hours. The resulting amplified aRNA was purified using the Spin Cartridges and aRNA Binding and Wash Buffers supplied by Invitrogen, and the yield of aRNA was quantified using Nanodrop Technologies.

The aRNA was reverse transcribed with random 8-mers (N8) to produce single stranded cDNA. For reverse transcription, 500 ng or 1000 ng of aRNA was added to 20 µl of RT mix containing 4 µl 5X first strand buffer (250 mM Tris-HCl, pH 8.3; 375 mM KCl; 15 mM MgCl₂), 2 µl 10 mM dNTPs, 1 µl 100 mM DTT, 1 µl RNAseOUT™ (40 units/µl), 2 µl SuperScript III Reverse Transcriptase (200 units/µl), and incubated at 40°C for 60 min, 70°C for 10 min, and cooled on ice.

Following reverse transcription, qPCR was performed as described in Example 3 using ABI Taqman^{®} assays for IGFR1 (catalog #Hs00181385), GAPDH (catalog #43100884E-0507028), GUSB (catalog #4310888E), ACTIN (catalog #4310881E), eukaryotic 18S rRNA (catalog #Hs99999901s1), and human hemoglobin beta (HBB) (catalog #Hs00747223 gl)

For eukaryotic 28S rRNA and human hemoglobin alpha (HBA) custom primers and probes (containing a 5'-FAM (6-carboxyfluorescein) reporter dye and a no fluorescent quencher (NFQ) at the 3' end of the probe) were used as follows:
Eukaryotic 28S rRNA:
Forward primer: 5' ACGGTGGCCATGGAAGTC 3' (SEQ ID NO:940);
Reverse primer: 5' TCGGCAGGTGAGTTGTTACAC 3' (SEQ ID NO:941);
FAM Probe: 5' ACTCCTTAGCGGATTCC 3' (SEQ ID NO:942)
Human hemoglobin alpha (HBA):
Forward primer: 5' GCACGCGCACAAGCT 3' (SEQ ID NO:943)
Reverse primer: 5' GGGTCACCAGCAGGCA 3' (SEQ ID NO:944);
FAM Probe 5' ACTTCAAGCTCCTAAGCCAC 3' (SEQ ID NO:945)

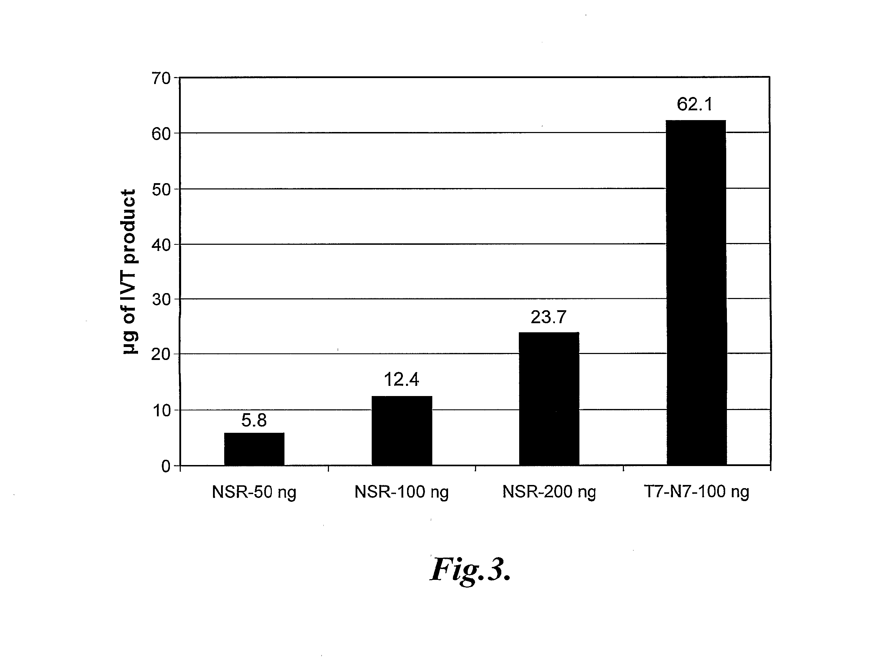

Reverse transcription of human whole blood total RNA with the NSR7 (comprising SEQ ID NOS: 1-933) followed by IVT produced high yields of aRNA that were directly proportional to the amount of input RNA template as shown in FIGURE 3.

Quantitative PCR (qPCR) analysis of the cDNA and reverse transcribed IVT products (IVT-RT) from varying amounts of RNA template is shown in Table 13 (raw Ct values) and Table 14 (absolute abundance). Primer pool indicates the primers used for first strand cDNA synthesis. Product indicates either the cDNA or IVT-RT product was the template for qPCR.

**TABLE 13. Raw Ct values for SSIII reverse transcribed cDNA (left side columns, cDNA) followed by IVT-RT (right side columns, IVT).**

| **RNA template (ng)** | **50** | **100** | **200** | **100** | **100** | **100** | **50** | **100** | **200** | **100** |
|---|---|---|---|---|---|---|---|---|---|---|
| **Primer pool** | **NSR** | **NSR** | **NSR** | **T7-N7** | **N8** | **no primer** | **NSR** | **NSR** | **NSR** | **T7-N7** |
| product | cDNA | cDNA | cDNA | cDNA | cDNA | cDNA | IVT | IVT | IVT | IVT |
| IGF1^{®} | 29 | 27 | 27 | 26 | 25 | 33 | 21 | 21 | 21 | 23 |
| GAPDH | 29 | 28 | 26 | 27 | 26 | 31 | 21 | 21 | 21 | 23 |
| GUSB | 34 | 33 | 32 | 32 | 29 | 33 | 25 | 25 | 25 | 26 |
| ACTIN | 29 | 28 | 26 | 26 | 23 | 31 | 21 | 22 | 21 | 22 |
| 18S | 19 | 17 | 17 | 16 | 15 | 21 | 12 | 12 | 12 | 13 |
| 28S | 17 | 15 | 14 | 15 | 14 | 16 | 14 | 14 | 14 | 14 |
| HBA | 21 | 20 | 19 | 18 | 16 | 24 | 17 | 16 | 16 | 17 |
| HBB | 21 | 20 | 19 | 18 | 17 | 23 | 17 | 16 | 16 | 16 |

**TABLE 14. Absolute abundance values of SSIII reverse transcribed cDNA (left side columns, cDNA) followed by IVT-RT (right side columns, IVT).**

| **RNA template (ng)** | **50** | **100** | **200** | **100** | **100** | **100** | **50** | **100** | **200** | **100** |
|---|---|---|---|---|---|---|---|---|---|---|
| **Primer pool** | **NSR** | **NSR** | **NSR** | **T7-N7** | **N8** | **no primer** | **NSR** | **NSR** | **NSR** | **T7-N7** |
| Product | cDNA | cDNA | cDNA | cDNA | cDNA | cDNA | IVT | IVT | IVT | IVT |
| IGF1^{®} | 22 | 53 | 97 | 111 | 226 | 1 | 3641 | 3824 | 4344 | 1676 |
| GAPDH | 13 | 26 | 138 | 59 | 178 | 5 | 4290 | 4966 | 5375 | 1043 |
| GUSB | 0 | 1 | 3 | 2 | 21 | 1 | 328 | 354 | 384 | 200 |
| ACTIN | 16 | 42 | 123 | 120 | 891 | 4 | 3465 | 3154 | 3518 | 2319 |
| 18S | 14933 | 61683 | 75134 | 124343 | 237330 | 3556 | 2071662 | 2497065 | 2881157 | 1015695 |
| 28S | 99635 | 233965 | 434367 | 339446 | 780775 | 141364 | 499476 | 633708 | 663349 | 816928 |
| HBA | 5441 | 9387 | 17205 | 42639 | 115379 | 564 | 101594 | 132380 | 119639 | 72935 |
| HBB | 4712 | 9505 | 19323 | 36727 | 66940 | 967 | 97505 | 119805 | 127175 | 169351 |

The ratio of reporter to background RNA is shown in Table 15. There was little substantial NSR7 specificity relative to T7-N7 observed during the initial first strand cDNA step (left columns). Importantly, the ratio of reporter to background RNAs increased going from the first strand synthesis step to the IVT step (right columns) for both the NSR7 and T7-N7 primers (the data was normalized to N8).

**TABLE 15. Reporter/background ratios for varying amounts of RNA template after first strand cDNA synthesis (left columns) and IVT-RT (right columns) (normalized to N8).**

| **RNA template (ng)** | **50** | **100** | **200** | **100** | **100** | **100** | **50** | **100** | **200** | **100** |
|---|---|---|---|---|---|---|---|---|---|---|
| **Primer pool** | **NSR** | **NSR** | **NSR** | **T7-N7** | **N8** | **no primer** | **NSR** | **NSR** | **NSR** | **T7-N7** |
| **product** | cDNA | cDNA | cDNA | cDNA | cDNA | cDNA | IVT | IVT | IVT | IVT |
| IGF1R/18S | 1.55 | 0.90 | 1.36 | 0.94 | 1.00 | 0.36 | 1.84 | 1.60 | 1.58 | 1.73 |
| IGF1R/28S | 0.76 | 0.78 | 0.77 | 1.13 | 1.00 | 0.03 | 25.13 | 20.81 | 22.58 | 7.07 |
| IGF1R/HBA | 2.07 | 2.88 | 2.88 | 1.33 | 1.00 | 1.09 | 18.26 | 14.72 | 18.50 | 11.71 |
| IGF1R/HBB | 1.39 | 1.65 | 1.49 | 0.89 | 1.00 | 0.37 | 11.04 | 9.44 | 10.10 | 2.92 |
| APDH/18S | 1.19 | 0.57 | 2.45 | 0.64 | 1.00 | 1.96 | 2.76 | 2.65 | 2.49 | 1.37 |
| GAPDH/28S | 0.59 | 0.49 | 1.39 | 0.77 | 1.00 | 0.16 | 37.66 | 34.36 | 35.53 | 5.60 |
| GAPDH/HBA | 1.59 | 1.82 | 5.19 | 0.90 | 1.00 | 6.00 | 27.36 | 24.30 | 29.11 | 9.27 |
| GAPDH/HBB | 1.06 | 1.04 | 2.68 | 0.61 | 1.00 | 2.03 | 16.54 | 15.58 | 15.89 | 2.32 |
| GUSB/18S | 0.36 | 0.22 | 0.48 | 0.16 | 1.00 | 2.78 | 1.80 | 1.61 | 1.52 | 2.24 |
| GUSB/28S | 0.18 | 0.19 | 0.27 | 0.19 | 1.00 | 0.23 | 24.59 | 20.90 | 21.65 | 9.15 |
| GUSB/HBA | 0.48 | 0.69 | 1.02 | 0.22 | 1.00 | 8.53 | 17.86 | 14.78 | 17.74 | 15.14 |
| GUSB/HBB | 0.32 | 0.40 | 0.53 | 0.15 | 1.00 | 2.89 | 10.80 | 9.48 | 9.68 | 3.78 |
| ACTIN/18S | 0.28 | 0.18 | 0.44 | 0.26 | 1.00 | 0.33 | 0.45 | 0.34 | 0.33 | 0.61 |
| ACTIN/28S | 0.14 | 0.16 | 0.25 | 0.31 | 1.00 | 0.03 | 6.08 | 4.36 | 4.65 | 2.49 |
| ACTIN/HBA | 0.38 | 0.58 | 0.93 | 0.36 | 1.00 | 1.02 | 4.42 | 3.09 | 3.81 | 4.12 |
| ACTIN/HBB | 0.25 | 0.33 | 0.48 | 0.25 | 1.00 | 0.35 | 2.67 | 1.98 | 2.08 | 1.03 |

Quantitative PCR analysis of the IVT products generated from NSR7-primed cDNA showed that the composition of each sample was approximately the same at three different total RNA input concentrations (50 ng, 100 ng, and 200 ng). Composition refers to normalized yields of reporter and background IVT products. As shown in Table 16, the normalized yields were very similar for both the reporter and background genes across a 4-fold range of input RNA. This data suggests that the IVT products were amplified from the appropriate target RNA and the reaction was not overwhelmed with nonspecific transcript material.

**TABLE 16. qPCR analysis of the NSR7 IVT-RT products at varying RNA template amounts.**

| | **NSR-50 ng** | **NSR-100 ng** | **NSR-200 ng** | **average** |
|---|---|---|---|---|
| IGF1R | 3641 | 3824 | 4344 | 3936 |
| GAPDH | 4290 | 4966 | 5375 | 4877 |
| GUSB | 328 | 354 | 384 | 355 |
| ACTIN | 3465 | 3154 | 3518 | 3379 |
| 18S | 2071662 | 2497065 | 2881157 | 2483294 |
| 28S | 499476 | 633708 | 663349 | 598844 |
| HBA | 101594 | 132380 | 119639 | 117871 |
| HBB | 97505 | 119805 | 127175 | 114828 |

### EXAMPLE 7

This Example shows that NSR7 primers (comprising SEQ ID NOS:1-933), that each have the T7 promoter (SEQ ID NO:934) covalently attached at the 5' end, coupled with high dNTP concentrations, generate first strand RT products with high specificity, and that these products can be amplified with the SuperScript™ RNA Amplification System (Invitrogen) to produce high yields of IVT product substantially enriched for target genes.

### Methods:

400 ng of whole blood total RNA was reverse transcribed using the NSR7, T7-N7, and N8 primers in 40 µl reaction volume, as described in Example 5. 20 µl (200 ng whole blood total RNA) was used to assess IGF1R, GAPDH, rRNA and globin abundance by qPCR, and the best performing set of primers was then subjected to IVT. Two primer concentrations (10 µM and 25 µM) and two dNTP concentrations (1000 µM and 2500 µM) were evaluated. The raw Ct and absolute abundance values following first strand reverse transcription are shown in Table 17 and Table 18.

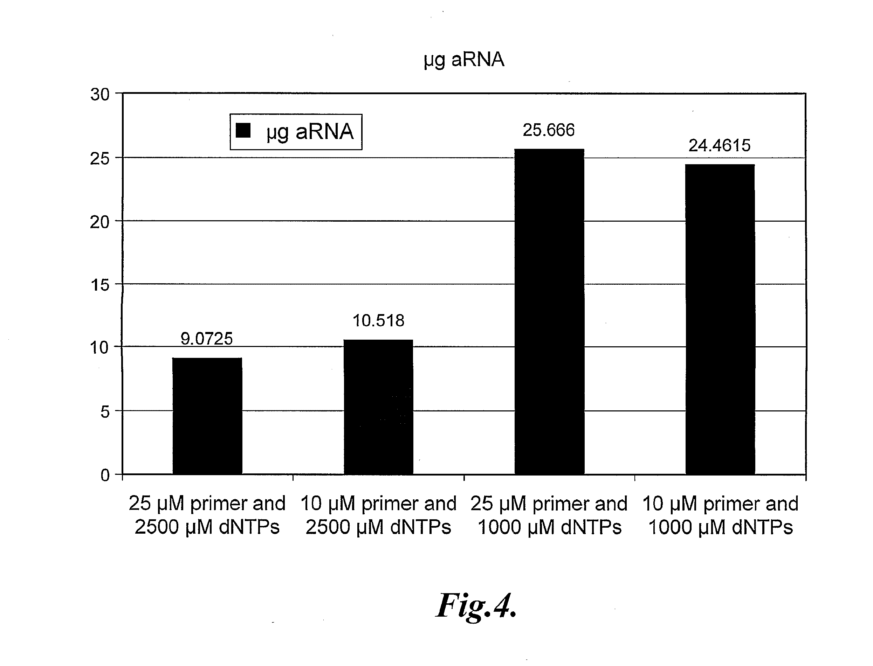

One µg of aRNA from the IVT reaction was converted back into cDNA by reverse transcription for qPCR analysis, as described in Example 6. FIGURE 4 shows that the specific activity of the reporter genes IGF1R and GAPDH was increased in the presence of high dNTP concentrations, whereas the specific activity of background rRNA and globin mRNA was decreased in the presence of high dNTP concentrations.

The raw Ct and abundance values following qPCR of the IVT-RT reaction products is shown in Tables 19 and 20. The reporter to background ratios for first strand NSR7 and IVT-RT are shown in Table 21.

The ratio of ratios data allows a comparison of first strand synthesis yields with IVT yields following conversion to cDNA and qPCR. Comparison of reporter to background ratios and NSR7 to N8 ratios shows that increasing the dNTP concentration from 1000 mM to 2500 mM resulted in a pronounced increase in NSR7-mediated specificity, as shown in Table 22. The primer concentration had a less noticeable impact on the NSR7-mediated specificity. In addition, comparing the ratios of NSR7-primed cDNA synthesis yields to the IVT yields showed a dramatic relative reduction in rRNAs (18S and 28S), suggesting that the rRNA cDNAs observed in the first round of reverse transcription fail to be transcribed during IVT (Table 23). The relative reduction in background globin mRNA yields between first strand synthesis and IVT was not observed with the NSR7 primers. When first strand synthesis was primed with random 8-mers, the reporter genes IGF1R and GAPDH were enriched relative to both rRNA and globin mRNA following IVT (Table 24).

In summary, this Example shows that high dNTP concentrations in combination with the NSR7 primers (comprising SEQ ID NOS:1-933) generate first strand reverse transcription products with high NSR7 specificity. The first strand products can then be amplified using the SuperScript™ RNA Amplification System (Invitrogen) to produce high yields of IVT product that is substantially enriched for target genes.

**TABLE 17. Raw Ct values from the first strand cDNA reactions.**

| First strand Primer pool | **NSR RT** | | | | **N8 RT** | | | |
|---|---|---|---|---|---|---|---|---|
| Primer concentration (mM) | **25 and 2500** | **10 and 2500** | **25 and 1000** | **10 and 1000** | **25 and 2500** | **10 and 2500** | **25 and 1000** | **10 and 1000** |
| IGF1R | 27 | 27 | 26 | 26 | 25 | 26 | 25 | 25 |
| GAPDH | 24 | 24 | 24 | 24 | 23 | 23 | 23 | 23 |
| 18S | 18 | 18 | 14 | 14 | 13 | 14 | 12 | 12 |
| 28S | 20 | 20 | 15 | 15 | 13 | 14 | 12 | 12 |
| HBA | 22 | 23 | 19 | 19 | 16 | 16 | 17 | 17 |
| HBB | 23 | 24 | 21 | 21 | 16 | 16 | 16 | 16 |

Key to the table headings:
25 and 2500 means 25 µM primer and 2500 µM dNTPs;
10 and 2500 means 10 µM primer and 2500 µM dNTPs;
25 and 1000 means 25 µM primer and 1000 µM dNTPs;
10 and 1000 means 10 µM primer and 1000 µM dNTPs;

**TABLE 18. Absolute abundance values from the first strand cDNA reactions.**

| | **NSR** | | | | **N8** | | | |
|---|---|---|---|---|---|---|---|---|
| Primer pool | **25 and 2500** | **10 and 2500** | **25 and 1000** | **10 and 1000** | **25 and 2500** | **10 and 2500** | **25 and 1000** | **10 and 1000** |
| IGF1R | 98 | 80 | 171 | 176 | 242 | 190 | 409 | 363 |
| GAPDH | 613 | 624 | 501 | 740 | 1215 | 966 | 1504 | 1497 |
| 18S | 48666 | 45494 | 548592 | 467387 | 1168629 | 831556 | 2699215 | 2001591 |
| 28S | 9154 | 9053 | 289871 | 381748 | 950029 | 765345 | 2754112 | 2747920 |
| HBA | 1971 | 886 | 15374 | 14544 | 197577 | 158464 | 89286 | 54844 |
| HBB | 1136 | 424 | 5406 | 4152 | 152329 | 176372 | 155202 | 135652 |

**Table 19. Raw Ct values for the IVT-RT reactions. NTC = No template control.**

| **Primer pool** | **NSR** | | | | |
|---|---|---|---|---|---|
| | **25 and 2500** | **10 and 2500** | **25 and 1000** | **10 and 1000** | **NTC** |
| IGF1R | 22 | 21 | 22 | 22 | 40 |
| GAPDH | 21 | 20 | 22 | 21 | 40 |
| 18S | 19 | 17 | 15 | 14 | 40 |
| 28S | 21 | 20 | 16 | 16 | 36 |
| HBA | 18 | 17 | 17 | 17 | 40 |
| HBB | 20 | 19 | 19 | 18 | 40 |

**Table 20. Absolute abundance values for the IVT-RT reactions.**

| **Primer pool** | **NSR** | | | |
|---|---|---|---|---|
| | **25 and 2500** | **10 and 2500** | **25 and 1000** | **10 and 1000** |
| IGF1R | 3174 | 3642 | 2586 | 2190 |
| GAPDH | 5218 | 12093 | 2592 | 5350 |
| 18S | 25303 | 55054 | 295696 | 503082 |
| 28S | 6332 | 9545 | 125816 | 137191 |
| HBA | 49703 | 79131 | 55051 | 82026 |
| HBB | 10622 | 19903 | 26634 | 46817 |

**TABLE 21. Reporter to background ratios (* 1000) calculated from the abundance data in Tables 17 and 19.**

| | | **NSR-IVT RT** | | | |
|---|---|---|---|---|---|
| | | **25 and 2500** | **10 and 2500** | **25 and 1000** | **10 and 1000** |
| IGF1R/18S | IVT | 125 | 66 | 9 | 4 |
| | NSR7 | 2 | 2 | 0 | 0 |
| | N8 | 0 | 0 | 0 | 0 |
| IGF1R/28S | IVT | 501 | 382 | 21 | 16 |
| | NSR | 11 | 9 | 1 | 0 |
| | N8 | 0 | 0 | 0 | 0 |
| IGF1R/HBA | IVT | 64 | 46 | 47 | 27 |
| | NSR | 50 | 90 | 11 | 12 |
| | N8 | 1 | 1 | 5 | 7 |
| IGF1R/HBB | IVT | 299 | 183 | 97 | 47 |
| | NSR | 86 | 188 | 32 | 42 |
| | N8 | 2 | 1 | 3 | 3 |
| GAPDH/18S | IVT | 206 | 220 | 9 | 11 |
| | NSR | 13 | 14 | 1 | 2 |
| | N8 | 1 | 1 | 1 | 1 |
| GAPDH/28S | IVT | 824 | 1267 | 21 | 39 |
| | NSR | 67 | 69 | 2 | 2 |
| | N8 | 1 | 1 | 1 | 1 |
| GAPDH/HBA | IVT | 105 | 153 | 47 | 65 |
| | NSR | 311 | 704 | 33 | 51 |
| | N8 | 6 | 6 | 17 | 27 |
| GAPDH/HBB | IVT | 491 | 608 | 97 | 114 |
| | NSR | 540 | 1473 | 93 | 178 |
| | N8 | 8 | 5 | 10 | 11 |

**TABLE 22. Ratios of ratios comparing NSR7 and N8 primed first strand synthesis after IVT-RT.**

| | **NSR IVT RT** | | | |
|---|---|---|---|---|
| ratios of ratios | **25 and 2500** | **10 and 2500** | **25 and 1000** | **10 and 1000** |
| IGF1R/18S NSR/N8 | 10 | 8 | 2 | 2 |
| IGF1R/28S NSR/N8 | 42 | 35 | 4 | 3 |
| IGF1R/HBA NSR/N8 | 41 | 75 | 2 | 2 |
| IGF1R/HBB NSR/N8 | 54 | 174 | 12 | 16 |
| GAPDH/18S NSR/N8 | 12 | 12 | 2 | 2 |
| GAPDH/28S NSR/N8 | 52 | 55 | 3 | 4 |
| GAPDH/HBA NSR/N8 | 51 | 116 | 2 | 2 |
| GAPDH/HBB NSR/N8 | 68 | 269 | 10 | 16 |

**TABLE 23. Ratios of ratios comparing NSR7 primed first strand synthesis before and after IVT-RT.**

| | **NSR IVT RT** | | | |
|---|---|---|---|---|
| | **25 and 2500** | **10 and 2500** | **25 and 1000** | **10 and 1000** |
| IGF1R/18S IVT/NSR | 62 | 38 | 28 | 12 |
| IGF1R/28S IVT/NSR | 47 | 43 | 35 | 35 |
| IGF1R/HBA IVT/NSR | 1.3 | 0.5 | 4 | 2 |
| IGF1R/HBB IVT/NSR | 3 | 1.0 | 3 | 1.1 |
| GAPDH/18S IVT/NSR | 16 | 16 | 10 | 7 |
| GAPDH/28S IVT/NSR | 12 | 18 | 12 | 20 |
| GAPDH/HBA IVT/NSR | 0.3 | 0.2 | 1.4 | 1.3 |
| GAPDH/HBB IVT/NSR | 0.9 | 0.4 | 1.1 | 0.6 |

**TABLE 24. Ratios of ratios comparing N8 primed first strand synthesis before and after IVT-RT.**

| | **NSR-IVT RT** | | | |
|---|---|---|---|---|
| | **25 and 2500** | **10 and 2500** | **25 and 1000** | **10 and 1000** |
| IGF1R/18S IVT/N8 | 607 | 289 | 58 | 24 |
| IGF1R/28S IVT/N8 | 1971 | 1533 | 138 | 121 |
| IGF1R/HBA IVT/N8 | 52 | 38 | 10 | 4 |
| IGF1R/HBB IVT/N8 | 188 | 169 | 37 | 17 |
| GAPDH/18S IVT/N8 | 198 | 189 | 16 | 14 |
| GAPDH/28S IVT/N8 | 644 | 1004 | 38 | 72 |
| GAPDH/HBA IVT/N8 | 17 | 25 | 3 | 2 |
| GAPDH/HBB IVT/N8 | 62 | 111 | 10 | 10 |

### EXAMPLE 8

This Example shows that selective enhancement of IGF1R and GAPDH reporter mRNAs relative to rRNA and globin mRNA using the NSR7 primers (comprising SEQ ID NOS:1-933), that each have the T7 promoter (SEQ ID NO:934) covalently attached at the 5' end, with high concentrations of dNTPs occurs over a wide range of whole blood total RNA template concentrations.

Total human whole blood RNA was reverse transcribed and analyzed by qPCR as described in Example 5. The amount of template RNA was 20 ng, 50 ng, 100 ng, 500 ng, 1000 ng, 2000 ng, and 5000 ng. The data show that the NSR7 mediated enrichment of IGF1R and GAPDH reporters relative to rRNA and globin mRNA (as determined by the ratio of ratios for NSR7 versus N8) is observed at all template amounts (Table 25 and Table 26). The reporter/background ratio of ratios for NSR versus N8 increases at higher template amounts, but the overall yield of first strand cDNA product is compromised (Table 27). One microgram (1000 ng) of input template RNA represents an optimal compromise between cDNA yields and NSR7-mediated amplification specificity. At higher RNA template amounts (2000 ng and 5000 ng) the reverse transcription reaction failed to fully convert RNA into cDNA. The cDNA yields in Table 28 were determined by multiplying the absolute yields of product (from Table 29) by the dilution factor relative to 5000 ng of template RNA, then normalizing to the N8 yield from 20 ng of template RNA which consistently produced the highest yield values. The normalized cDNA yields of IGF1R and GAPDH were similar across all template amounts for both NSR7 and N8, with a decreased yield at the highest RNA template concentrations. In contrast, the yield of 18S, 28S, alpha globin, and beta globin products was substantially higher when primed with N8 than when primed with NSR7.

In summary, the "NSR effect," defined as a high percentage of conversion by the NSR7 primers (comprising SEQ ID NOS: 1-933) of reporter mRNA to cDNA relative to N8, but a low percentage of conversion by the NSR7 primers of background RNAs to cDNA relative to N8, is conserved across all template concentrations tested. This provides the advantage that reaction conditions do not need to be customized to template amounts.

**TABLE 25. Ratio of ratios for reporter/background and NSR/N8 at different RNA template amounts.**

| | **ng total RNA** | **5000** | **2000** | **1000** | **500** | **200** | **100** | **50** | **20** |
|---|---|---|---|---|---|---|---|---|---|
| IGF1R/18S | NSR/N8 | 26 | 30 | 25 | 16 | 10 | 8 | 7 | 11 |
| IGF1R/28S | NSR/N8 | 7 | 8 | 9 | 7 | 8 | 7 | 7 | 9 |
| IGF1R/HBA | NSR/N8 | 26 | 34 | 30 | 19 | 11 | 7 | 5 | 8 |
| IGF1R/HBB | NSR/N8 | 14 | 16 | 19 | 15 | 10 | 6 | 5 | 8 |
| GAPDH/18S | NSR/N8 | 40 | 44 | 37 | 24 | 13 | 10 | 9 | 11 |
| GAPDH/28S | NSR/N8 | 11 | 12 | 14 | 11 | 10 | 9 | 8 | 9 |
| GAPDH/HBA | NSR/N8 | 42 | 50 | 45 | 27 | 15 | 9 | 7 | 8 |
| GAPDH/HBB | NSR/N8 | 22 | 23 | 28 | 22 | 13 | 8 | 7 | 9 |

**TABLE 26. Percent reduction of background RNAs for the NSR/N8 ratio at different RNA template amounts.**

| | **ng total RNA** | **5000** | **2000** | **1000** | **500** | **200** | **100** | **50** | **20** |
|---|---|---|---|---|---|---|---|---|---|
| IGF1R/18S | NSR/N8 | 96 | 97 | 96 | 94 | 90 | 87 | 86 | 91 |
| IGF1R/28S | NSR/N8 | 86 | 88 | 89 | 86 | 87 | 86 | 85 | 89 |
| IGF1R/HBA | NSR/N8 | 96 | 97 | 97 | 95 | 91 | 86 | 82 | 88 |
| IGF1R/HBB | NSR/N8 | 93 | 94 | 95 | 93 | 90 | 84 | 81 | 88 |
| GAPDH/18S | NSR/N8 | 98 | 98 | 97 | 96 | 93 | 90 | 89 | 91 |
| GAPDH/28S | NSR/N8 | 91 | 92 | 93 | 91 | 90 | 89 | 88 | 89 |
| GAPDH/HBA | NSR/N8 | 98 | 98 | 98 | 96 | 93 | 89 | 85 | 88 |
| GAPDH/HBB | NSR/N8 | 95 | 96 | 96 | 95 | 92 | 88 | 85 | 89 |

**TABLE 27: cDNA yields normalized to N8.**

| | **ng total RNA** | **5000** | **2000** | **1000** | **500** | **200** | **100** | **50** | **20** |
|---|---|---|---|---|---|---|---|---|---|
| IGF1R | NSR | 15 | 25 | 36 | 35 | 41 | 36 | 37 | 40 |
| | N8 | 33 | 54 | 70 | 82 | 90 | 90 | 96 | 100 |
| GAPDH | NSR | 18 | 32 | 43 | 43 | 48 | 43 | 40 | 42 |
| | N8 | 27 | 47 | 56 | 67 | 81 | 83 | 86 | 100 |
| 18S | NSR | 0 | 1 | 2 | 2 | 4 | 4 | 4 | 4 |
| | N8 | 18 | 62 | 97 | 93 | 93 | 86 | 84 | 100 |
| 28S | NSR | 1 | 2 | 3 | 4 | 5 | 5 | 5 | 5 |
| | N8 | 8 | 27 | 50 | 67 | 87 | 84 | 87 | 100 |
| HBA | NSR | 0 | 1 | 1 | 1 | 3 | 4 | 5 | 5 |
| | N8 | 16 | 41 | 63 | 63 | 74 | 72 | 72 | 100 |
| HBB | NSR | 0 | 1 | 1 | 2 | 4 | 5 | 6 | 5 |
| | N8 | 10 | 22 | 44 | 58 | 80 | 80 | 79 | 100 |

**TABLE 28. Ratios of NSR synthesis to N8 synthesis across all template conditions expressed as a % of N8.**

| | **ng total RNA** | **5000** | **2000** | **1000** | **500** | **200** | **100** | **50** | **20** |
|---|---|---|---|---|---|---|---|---|---|
| IGF1R | NSR/N8 | 44 | 47 | 51 | 43 | 45 | 40 | 38 | 40 |
| GAPDH | NSR/N8 | 69 | 69 | 77 | 64 | 59 | 51 | 47 | 42 |
| 18S | NSR/N8 | 2 | 2 | 2 | 3 | 4 | 5 | 5 | 4 |
| 28S | NSR/N8 | 6 | 6 | 5 | 6 | 6 | 6 | 6 | 5 |
| HBA | NSR/N8 | 2 | 1 | 2 | 2 | 4 | 6 | 7 | 5 |
| HBB | NSR/N8 | 3 | 3 | 3 | 3 | 5 | 6 | 7 | 5 |

**TABLE 29. qPCR of NSR and N8 primed first strand reverse transcription products across a range of RNA template amounts. Raw Ct data.**

| | **ng total RNA** | **5000** | **2000** | **1000** | **500** | **200** | **100** | **50** | **20** |
|---|---|---|---|---|---|---|---|---|---|
| IGF1R | NSR | 25.21 | 25.73 | 26.22 | 27.26 | 28.37 | 29.55 | 30.52 | 31.71 |
| | N8 | 24.02 | 24.65 | 25.26 | 26.04 | 27.22 | 28.22 | 29.13 | 30.40 |
| GAPDH | NSR | 21.05 | 21.55 | 22.14 | 23.15 | 24.30 | 25.47 | 26.55 | 27.83 |
| | N8 | 20.50 | 21.02 | 21.75 | 22.50 | 23.55 | 24.51 | 25.46 | 26.56 |
| 18S | NSR | 20.53 | 20.19 | 20.17 | 20.86 | 21.45 | 22.33 | 23.32 | 24.93 |
| | N8 | 14.65 | 14.20 | 14.56 | 15.61 | 16.94 | 18.05 | 19.08 | 20.16 |
| 28S | NSR | 19.21 | 18.96 | 19.12 | 19.60 | 20.57 | 21.63 | 22.57 | 24.02 |
| | N8 | 15.19 | 14.84 | 14.93 | 15.51 | 16.46 | 17.51 | 18.46 | 19.58 |
| HBA | NSR | 21.73 | 21.93 | 22.00 | 22.55 | 22.85 | 23.40 | 24.09 | 25.42 |
| | N8 | 15.81 | 15.74 | 16.13 | 17.12 | 18.22 | 19.26 | 20.26 | 21.11 |
| HBB | NSR | 21.30 | 21.50 | 21.65 | 22.17 | 22.35 | 22.90 | 23.75 | 25.31 |
| | N8 | 16.30 | 16.45 | 16.48 | 17.07 | 17.92 | 18.92 | 19.94 | 20.92 |

**TABLE 30. qPCR of NSR and N8 primed first strand reverse transcription products across a range of RNA template amounts. Absolute abundance data.**

| | **ng total RNA** | **5000** | **2000** | **1000** | **500** | **200** | **100** | **50** | **20** |
|---|---|---|---|---|---|---|---|---|---|
| IGF1R | NSR | 257.04 | 179.85 | 127.89 | 62.30 | 28.88 | 12.70 | 6.51 | 2.85 |
| | N8 | 589.66 | 380.40 | 249.07 | 144.45 | 63.77 | 31.92 | 17.06 | 7.08 |
| GAPDH | NSR | 4615.40 | 3253.99 | 2167.62 | 1076.00 | 483.73 | 215.10 | 101.95 | 41.96 |
| | N8 | 6720.73 | 4697.05 | 2825.73 | 1691.11 | 816.01 | 419.57 | 217.38 | 100.91 |
| 18S | NSR | 6613.12 | 8365.89 | 8469.08 | 5242.78 | 3501.13 | 1893.76 | 954.04 | 313.53 |
| | N8 | 388405.53 | 531139.00 | 412768.53 | 199313.85 | 79602.24 | 36801.88 | 17994.25 | 8554.29 |
| 28S | NSR | 16479.87 | 19626.05 | 17496.69 | 12591.15 | 6419.05 | 3076.32 | 1602.46 | 589.43 |
| | N8 | 268154.87 | 339917.11 | 320454.27 | 214361.60 | 110770.23 | 53752.24 | 27678.38 | 12728.46 |
| HBA | NSR | 2869.10 | 2505.03 | 2381.69 | 1625.43 | 1326.05 | 902.22 | 558.24 | 222.34 |
| | N8 | 173954.32 | 182225.26 | 139416.35 | 70042.65 | 32706.21 | 15922.00 | 7984.30 | 4424.14 |
| HBB | NSR | 3868.82 | 3382.72 | 3033.17 | 2121.01 | 1869.38 | 1279.18 | 711.20 | 240.02 |
| | N8 | 123516.47 | 112017.08 | 109557.90 | 72684.35 | 40199.75 | 20201.73 | 9951.70 | 5035.32 |

### EXAMPLE 9

This Example shows that the NSR7 primers (comprising SEQ ID NOS:1-933), that each have the T7 promoter (SEQ ID NO:934) covalently attached at the 5' end, combined with high dNTP concentrations show increased specificity when compared to random 7-mers (T7-N7), and that this specificity is maintained across a range of RNA template amounts.

First-strand cDNA synthesis, second-strand synthesis, IVT, reverse transcription of the IVT, and qPCR of the aRNA were performed as described in Example 6. The RNA template amounts were 100 ng, 200 ng, 500 ng, and 1000 ng of whole blood total RNA. The reaction volumes were 40 µl, and the reactions were incubated at 40°C for 180 min, 70°C for 10 min, and chilled on ice. The dNTP concentration was 2000 µM. Second strand synthesis reaction was for 120 min at 16°C.

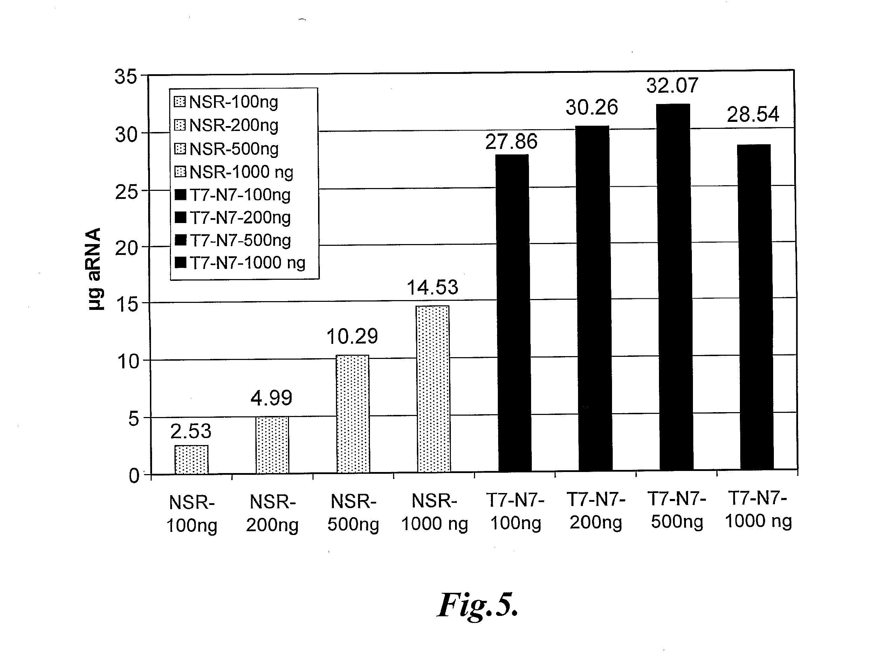

The data from the first-strand synthesis indicated that high dNTP concentrations result in increased levels of IGF1R and GAPDH reporters relative to rRNA and globin background genes when primed with the NSR7 pool, but not when primed with the T7-N7 pool (data normalized to N8) (Table 30). See FIGURE 5. The data in Table 31 further shows that the enrichment of reporters relative to background was also observed following *in vitro* transcription of the NSR7-primed cDNA, the enrichment tended to be more pronounced for the ribosomal rRNAs than the globin mRNAs, and the enrichment was observed across a range of input RNA template amounts. The ratio of ratios shows that reporters are enriched relative to background when comparing NSR7 to T7-N7 for both the first strand and IVT reactions, and this effect was observed at all RNA input amounts tested (Table 32). Table 33 shows the raw qPCR data from the NSR7, T7-N7 and N8-primed first strand cDNA and IVT-RT reactions. Table 34 shows the raw abundance data from the NSR7, T7-N7 and N8-primed first strand cDNA and IVT-RT reactions.

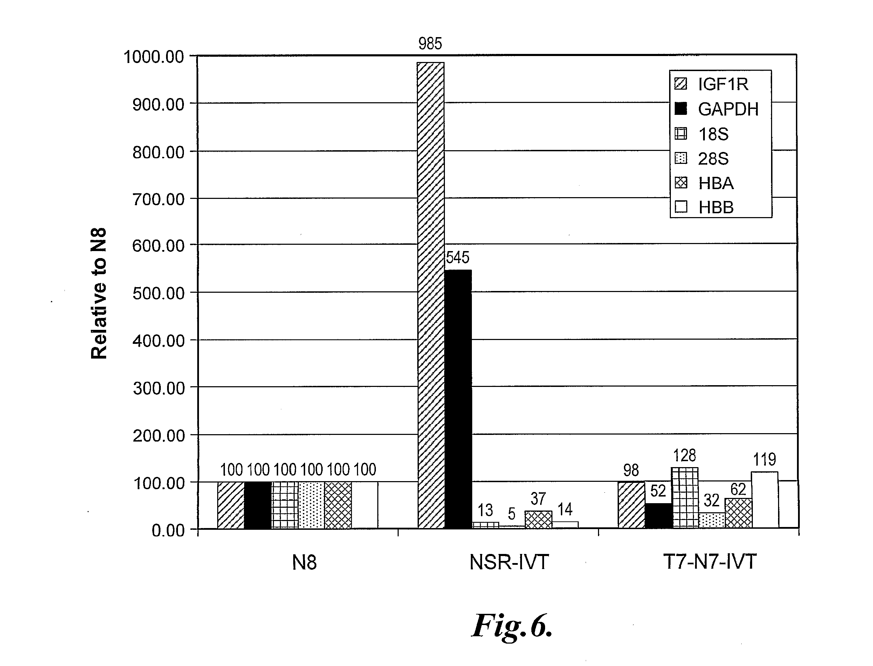

FIGURE 5 is a histogram plot showing the yield of amplified RNA from T7-NSR primed and T7-N7 primed cDNA as a function of the amount of input RNA template. The amount of input RNA tested ranged from 100 ng, 200 ng, 500 ng, and 1000 ng. FIGURE 6 is a histogram plot on a linear scale showing the relative composition of T7-NSR-primed and T7-N7 primed cDNA (normalized to N8) following amplification by *in vitro* transcription (IVT). As shown in FIGURES 5 and 6, the composition of the NSR-primed IVT product is substantially different than the composition of the N8-primed cDNA. As shown in FIGURE 6, following IVT with the NSR primer pool, the IGF1R and GAPDH reporters increased and the rRNA and globin background decreased. In contrast, the IVT product from the T7-N7 primer pool was not substantially different than the starting material when normalized to N8-primed cDNA.

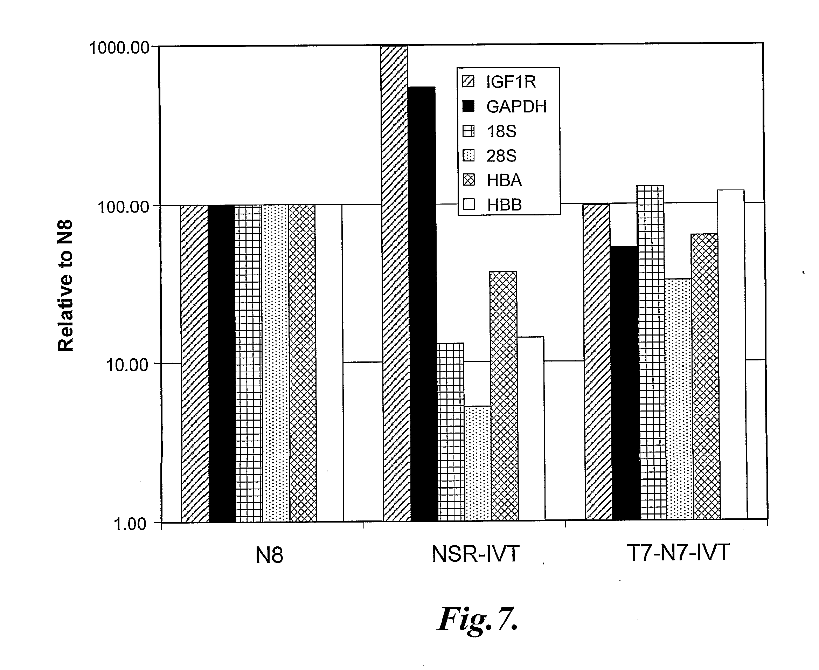

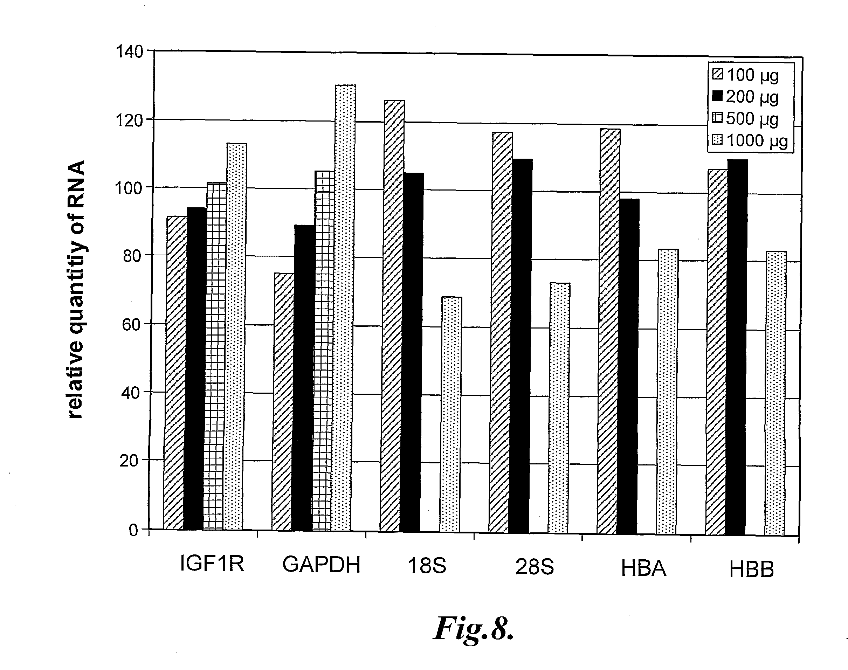

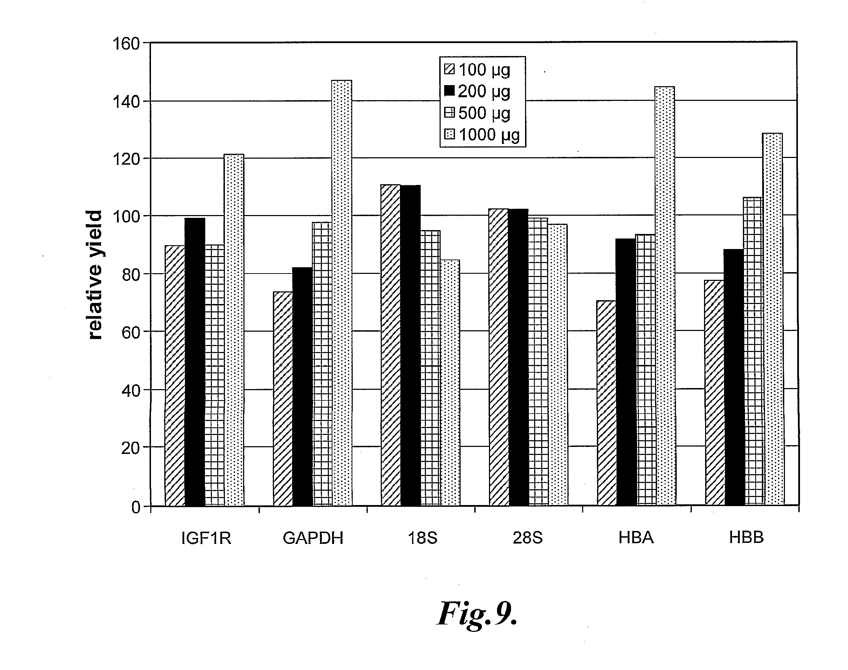

FIGURE 7 is a histogram plot on a logarithmic scale showing the relative composition of T7-NSR primed and T7-N7 primed cDNA (normalized of N8) following amplification by *in vitro* transcription. FIGURE 8 is a histogram plot showing the relative abundance of *in vitro* transcription products from T7-NSR primed cDNA as a function of RNA template amount. FIGURE 9 is a histogram plot showing the relative abundance of *in vitro* transcription products from T7-N7-primed cDNA as a function of RNA template amount.

As shown in FIGURE 7 and FIGURE 8, for the NSR-primed IVT product, there is a trend toward higher specificity with higher RNA template amounts (increase in IGF1R and GAPDH with a corresponding decrease in rRNA and globin aRNA). As shown in FIGURE 9, for the T7-N7 primed IVT product, the IGF1R, GAPDH, and globin levels increased with increasing RNA template, but the rRNAs declined slightly. These results indicate that the NSR-primed IVT product is fairly uniform across a 10-fold range of RNA inputs.

In summary, high dNTP concentrations in combination with the NSR primers result in increased reporter levels and decreased background levels following first strand cDNA synthesis and IVT across a range of RNA template amounts. The composition of the NSR-primed IVT product is substantially different than the T7-N7 and N8 primed product, such that IGF1R and GAPDH levels increased while rRNA and globin levels decreased when primed with NSR, but were not substantially different than the N8-primed starting material when primed with T7-N7. Finally, the composition of the NSR-primed IVT product was relatively uniform across a range of RNA template amounts.

**TABLE 31. Reporter to background ratios for NSR and T7-N7 primed first strand cDNA carried through IVT for different RNA template amounts, normalized to N8.**

| | | **NSR** | | | | **T7-N7** | | | |
|---|---|---|---|---|---|---|---|---|---|
| | | **1000** | **500** | **200** | **100** | **1000** | **500** | **200** | **100** |
| IGF1R/18S | 1st strand/N8 | 20 | 15 | 3 | 0 | 2 | 1 | 0 | 0 |
| | IVT/N8 | 75 | 0 | 9 | 1 | 1 | 0 | 0 | 0 |
| IGF1R/28S | 1st strand/N8 | 7 | 6 | 7 | 6 | 2 | 2 | 2 | 2 |
| | IVT/N8 | 187 | 0 | 148 | 139 | 3 | 3 | 3 | 3 |
| IGF1R/HBA | 1st strand/N8 | 29 | 19 | 14 | 10 | 2 | 1 | 1 | 1 |
| | IVT/N8 | 27 | 0 | 18 | 15 | 2 | 2 | 2 | 2 |
| IGF1R/HBB | 1st strand/N8 | 10 | 10 | 8 | 6 | 1 | 2 | 2 | 2 |
| | IVT/N8 | 69 | 0 | 76 | 85 | 1 | 1 | 2 | 2 |
| | | | | | | | | | |
| GAPDH/18S | 1st strand/N8 | 36 | 23 | 5 | 0 | 3 | 2 | 0 | 0 |
| | IVT/N8 | 41 | 0 | 4 | 0 | 0 | 0 | 0 | 0 |
| GAPDH/28S | 1st strand/N8 | 12 | 10 | 11 | 9 | 3 | 3 | 3 | 2 |
| | IVT/N8 | 104 | 0 | 67 | 55 | 2 | 1 | 1 | 1 |
| GAPDH/HBA | 1st strand/N8 | 50 | 29 | 22 | 15 | 2 | 2 | 1 | 1 |
| | IVT/N8 | 15 | 0 | 8 | 6 | 1 | 1 | 1 | 1 |
| GAPDH/HBB | 1st strand/N8 | 18 | 16 | 12 | 9 | 2 | 2 | 2 | 2 |
| | IVT/N8 | 38 | 0 | 35 | 34 | 0 | 0 | 1 | 1 |

**TABLE 32. Ratio of ratios for NSR versus T7-N7 primed first strand cDNA using the data from Table 24.**

| | | **NSR/T7-N7** | | | |
|---|---|---|---|---|---|
| | | **1000** | **500** | **200** | **100** |
| IGF1R/18S | 1st strand | 10 | 10 | 12 | 13 |
| | IVT | 99 | | 85 | 76 |
| IGF1R/28S | 1st strand | 3 | 3 | 3 | 4 |
| | IVT | 62 | | 44 | 44 |
| IGF1R/HBA | 1st strand | 16 | 15 | 13 | 12 |
| | IVT | 17 | | 9 | 6 |
| IGF1R/HBB | 1st strand | 8 | 6 | 5 | 4 |
| | IVT | 85 | | 44 | 43 |
| | | | | | |
| GAPDH/18S | 1st strand | 13 | 12 | 14 | 14 |
| | IVT | 102 | | 106 | 83 |
| GAPDH/28S | 1st strand | 4 | 4 | 4 | 4 |
| | IVT | 64 | | 55 | 48 |
| GAPDH/HBA | 1st strand | 22 | 18 | 16 | 13 |
| | IVT | 18 | | 12 | 7 |
| GAPDH/HBB | 1st strand | 10 | 8 | 6 | 4 |
| | IVT | 87 | | 55 | 47 |

**TABLE 33. qPCR of NSR, T7-N7 and N8 primed cDNA and IVT-RT products across a range of RNA template amounts. Raw Ct data.**

| | | **NSR** | | | | **T7-N7** | | | |
|---|---|---|---|---|---|---|---|---|---|
| | | **1000** | **500** | **200** | **100** | **1000** | **500** | **200** | **100** |
| | 1st strand | 25 | 26 | 27 | 28 | 25 | 26 | 27 | 28 |
| IGF1R | IVT | 21 | 21 | 21 | 21 | 24 | 25 | 25 | 25 |
| | N8 | 24 | 25 | 26 | 27 | 40 | | | |
| | | | | | | | | | |
| | 1st strand | 22 | 23 | 24 | 25 | 22 | 23 | 24 | 25 |
| GAPDH | IVT | 19 | 20 | 20 | 20 | 23 | 23 | 23 | 24 |
| | N8 | 22 | 22 | 23 | 24 | 40 | | | |
| | | | | | | | | | |
| | 1st strand | 24 | 23 | 24 | 25 | 23 | 22 | 23 | 23 |
| ACTIN | IVT | 25 | 25 | 25 | 26 | 25 | 26 | 27 | 27 |
| | N8 | 21 | 21 | 21 | 22 | 40 | | | |
| | | | | | | | | | |
| | 1st strand | 26 | 26 | 27 | 27 | 24 | 24 | 25 | 26 |
| RPO | IVT | 22 | 22 | 23 | 23 | 23 | 23 | 23 | 23 |
| | N8 | 22 | 23 | 23 | 24 | 40 | | | |
| | | | | | | | | | |
| | 1st strand | 19 | 19 | 20 | 21 | 15 | 16 | 16 | 17 |
| 18S | IVT | 17 | 26 | 16 | 16 | 13 | 13 | 13 | 13 |
| | N8 | 14 | 15 | 18 | 22 | 31 | | | |
| | | | | | | | | | |
| | 1st strand | 18 | 18 | 19 | 20 | 16 | 16 | 17 | 18 |
| 28S | IVT | 19 | 27 | 18 | 18 | 16 | 16 | 16 | 16 |
| | N8 | 14 | 15 | 15 | 16 | 34 | | | |
| | | | | | | | | | |
| | 1st strand | 21 | 21 | 21 | 22 | 16 | 17 | 17 | 18 |
| HBA | IVT | 16 | 24 | 16 | 16 | 16 | 16 | 16 | 17 |
| | N8 | 15 | 16 | 17 | 17 | 40 | | | |
| | | | | | | | | | |
| | 1st strand | 20 | 20 | 21 | 21 | 17 | 17 | 18 | 19 |
| HBB | IVT | 19 | 28 | 18 | 18 | 16 | 16 | 16 | 16 |
| | N8 | 16 | 16 | 17 | 17 | 40 | | | |

**TABLE 34. Raw Abundance Values**

| | | **NSR** | | | | **T7-N7** | | | |
|---|---|---|---|---|---|---|---|---|---|
| | | **1000** | **500** | **200** | **100** | **1000** | **500** | **200** | **100** |
| | 1st strand | 216 | 143 | 72 | 40 | 310 | 183 | 89 | 42 |
| IGF1R | IVT | 4523 | 4053 | 3754 | 3648 | 448 | 333 | 366 | 332 |
| | N8 | 459 | 276 | 149 | 84 | 0 | | | |
| | | | | | | | | | |
| | 1st strand | 2486 | 1423 | 747 | 366 | 2630 | 1505 | 739 | 274 |
| GAPD H | IVT | 16323 | 13161 | 11140 | 9377 | 1570 | 1043 | 876 | 788 |
| | N8 | 2994 | 1780 | 973 | 538 | 0 | | | |
| | | | | | | | | | |
| | 1st strand | 717 | 903 | 711 | 417 | 1681 | 2037 | 1572 | 903 |
| ACTIN | IVT | 394 | 391 | 212 | 205 | 232 | 108 | 95 | 73 |
| | N8 | 3567 | 5356 | 4174 | 2450 | 0 | | | |
| | | | | | | | | | |
| | 1st strand | 175 | 173 | 99 | 74 | 756 | 575 | 291 | 114 |
| RPO | IVT | 2414 | 1749 | 1393 | 1568 | 1484 | 1661 | 1225 | 1481 |
| | N8 | 2488 | 1515 | 891 | 534 | 0 | | | |
| | | | | | | | | | |
| | 1st strand | 17434 | 14200 | 7952 | 4526 | 243626 | 186097 | 113212 | 63707 |
| 18S | IVT | 99195 | | 151603 | 182491 | 969080 | 1084588 | 1263635 | 1266322 |
| | N8 | 754809 | 406684 | 52542 | 3134 | 5 | | | |
| | | | | | | | | | |
| | 1st strand | 34977 | 29249 | 16352 | 10477 | 147574 | 116224 | 70189 | 41750 |
| 28S | IVT | 26417 | | 39545 | 42413 | 162609 | 166203 | 171356 | 171603 |
| | N8 | 501934 | 352785 | 232936 | 135033 | 0 | | | |
| | | | | | | | | | |
| | 1st strand | 5534 | 5188 | 3643 | 2714 | 127644 | 99869 | 58543 | 34664 |
| HBA | IVT | 123460 | | 145322 | 176027 | 208179 | 134168 | 132001 | 101438 |
| | N8 | 335396 | 189786 | 103744 | 58823 | 0 | | | |
| | | | | | | | | | |
| | 1st strand | 7939 | 7090 | 6180 | 4696 | 87383 | 58730 | 38326 | 20360 |
| HBB | IVT | 24468 | | 32431 | 31560 | 205020 | 169267 | 140599 | 123574 |
| | N8 | 171594 | 140251 | 97796 | 61407 | 0 | | | |

### EXAMPLE 10

This Example describes an exemplary method of using NSR-primed first strand reverse transcription, followed by second strand synthesis and *in vitro* transcription for the preparation of microarray samples.

### Methods:

### First Strand Reverse Transcription

### First Strand Reverse Transcription is carried out as follows:

### Combine:

• 1 µg total RNA template
• 2 µl of 100 µM stock NSR primer
• x µl of water to a final volume of 10 µl
• Mix and incubate at 65°C for 5 minutes, snap chill on ice.

Add 10 µl of RT cocktail (prepared on ice) containing:
• 4 µl 5X FS buffer
• 1.6 µl of 25 mM dNTPs
• 1.4 µl water
• 1 µl stock DTT
• 1 µl RNAs OUT
• 1 µl SSIII

The sample is mixed, centrifuged and transferred to a 40°C pre-warmed thermal cycler (to provide a "hot start"), and the sample is incubated at 40°C for three hours, 70°C for 10 minutes and chilled to 4°C.

### Second Strand Synthesis

A second strand synthesis cocktail is prepared using the components of the SuperScript™ RNA Amplification System (Invitrogen, Catalog No. L1016-01) as follows:
• 91 µl DEPC-treated water
• 30 µl 5X Second-strand Buffer
• 3 µl 10 mM dNTP Mix
• 4 µl DNA Polymerase I (10 units/µl)
• 1 µl DNA Ligase (10 units/µl)
• 1 µl RNAse H (2 units/µl)

130 µl of the second strand synthesis cocktail is added to the 20 µl first strand reaction described above, mixed and the sample is incubated at 16°C for two hours.

### Purification of Double-Stranded cDNA

1. 500 µl of cDNA Loading Buffer is added to the reaction tube from Second-Strand cDNA Synthesis. The total volume in the tube should be 650 µl. Mix thoroughly by pipetting up and down.
2. Each Spin Cartridge is pre-inserted into a collection tube. Load the cDNA/buffer solution directly onto the Spin Cartridge.
3. Centrifuge at 12,000 × g at room temperature in a microcentrifuge for 1 minute. Remove the collection tube and discard the flow-through.
4. Place the Spin Cartridge in the same collection tube and add 700 µl of cDNA Wash Buffer.
5. Centrifuge at 12,000 × g at room temperature for 2 minutes. Remove the collection tube and discard the flow-through.
6. Place the Spin Cartridge in the same collection tube and centrifuge at 12,000 × g at room temperature for an additional 4 minutes. Remove the collection tube and discard the flow-through.
7. Place the Spin Cartridge into a new Recovery Tube.
8. Add 28 µl of DEPC-treated water to the center of the Spin Cartridge and incubate at room temperature for 2 minutes.
9. Centrifuge at 12,000 × g at room temperature for 1 minute.
10. Pipette cDNA back onto column, let sit 1 min, and recentrifuge.

### In Vitro Transcription Reaction

An *In Vitro* Transcription Cocktail is prepared as follows (18 µl volume)

| | | |
|---|---|---|
| • | 1.5 µl | 100 mM ATP |
| • | 1.5 µl | 100 mM CTP |
| • | 1.5 µl | 100 mM GTP |
| • | 0.75 µl | 100 mM UTP |
| • | 1.75 µl | Amino-allyl UTP (50 mM) |
| • | 4.0 µl | 10X T7 Reaction Buffer |
| • | 7.0 µl | T7 Enzyme Mix |

22 µl of purified cDNA is added to the cocktail, and the sample is incubated at 37°C overnight.

### Purification of aRNA

The following procedure is used to purify the aRNA.
1. Add 160 µl of aRNA Binding Buffer to the reaction tube. The total volume should be 200 µl. Mix thoroughly by pipetting up and down.
2. Add 100 µl of 100% ethanol to the reaction tube. Mix thoroughly by pipetting up and down.
3. Each Spin Cartridge is pre-inserted into a collection tube. Load the entire aRNA/buffer solution directly onto the Spin Cartridge.
4. Centrifuge at 12,000 × g in a microcentrifuge for 15 seconds at room temperature. Remove the collection tube and discard the flow-through.
5. Place the Spin Cartridge in the same collection tube and add 500 µl of aRNA Wash Buffer.
6. Centrifuge at 12,000 × g for 15 seconds at room temperature. Remove the collection tube and discard the flow-through.
7. Repeat Steps 5-6.
8. Place the Spin Cartridge in the same collection tube and centrifuge at full speed for an additional 2 minutes to dry the column. Remove the collection tube and discard the flow-through.
9. Place the Spin Cartridge into a new Recovery Tube.
10. Add 100 µl of DEPC-treated water to the center of the Spin Cartridge and incubate at room temperature for 1 minute.
11. Centrifuge at 12,000 × g for 2 minutes at room temperature to collect the purified aRNA in the eluate.
12. Quantify aRNA yield using Nanodrop.

### EXAMPLE 11

This Example shows that cDNA generated using the 933 6-mers (SEQ ID NOS:1-933 either with or without T7 promoter (SEQ ID NO:934) covalently attached) can be converted into aDNA by PCR.

### Methods:

NSR primers used in this Example were as follows:
The 933 6-mers (SEQ ID NOS:1-933) plus a single random nucleotide inserted between the 6-mer sequence and the T7 promoter (SEQ ID NO:934), referred to as "NSR7."

Total RNA for the cell line Jurkat (T lymphocyte, ATCC No. TIB-152) was obtained from Ambion, Inc. (Austin, TX).

The protocol involves a three-step amplification approach. (1) first strand cDNA is generated from RNA using reverse transcription that is primed with NSR primers comprising a first primer binding site to generate NSR primed first strand cDNA, (2) second strand cDNA synthesis that is primed with random primers (e.g., 9-mers) comprising a second primer binding site; and (3) PCR amplification of the cDNA that is primed with forward and reverse primers that bind to the first and second primer binding sites to generate amplified DNA (aDNA).

**TABLE 35: Representative Primer Sets for use with NSR primed first strand reverse transcription followed by second strand synthesis and PCR amplification using NSR7 primers including the T7 promoter (comprising 933 6-mers (SEQ ID NOS: 1-933) plus a single random nucleotide inserted between the 6-mer sequence and the T7 promoter (SEQ ID NO:934))**

| *Primer Set 1* | | |
|---|---|---|
| T7 primer 1 | CGCAATTAATACGACTCACTATAGG | SEQ ID NO:946 |
| R RT 1 | TGCATTGAGAGGGTGTAATTTGNNNNNNNNN | SEQ ID NO:947 |
| R PCR 1 | TGCATTGAGAGGGTGTAATTTG | SEQ ID NO:948 |

| *Primer Set 2* | | |
|---|---|---|
| T7 primer 1 | cgcAATTAATACGACTCACTATAGG | SEQ ID NO:946 |
| RRT2 | GTATCTTGGGCCTGTTGACTTCNNNNNNNNN | SEQ ID NO:949 |
| R PCR 2 | GTATCTTGGGCCTGTTGACTTC | SEQ ID NO:950 |

| *Primer Set 3* | | |
|---|---|---|
| T7 Primer 1 | cgcAATTAATACGACTCACTATAGG | SEQ ID NO:946 |
| R RT 3 | ATTGTTCCTTCAGCTGTTCCATNNNNNNNNN | SEQ ID NO:951 |
| R PCR 3 | ATTGTTCCTTCAGCTGTTCCAT | SEQ ID NO:952 |

| *Primer Set 4* | | |
|---|---|---|
| T7 Primer 1 | cgcAATTAATACGACTCACTATAGG | SEQ ID NO:946 |
| R RT 4 | CCTCCACACTCCTAAGGTCATCNNNNNNNNN | SEQ ID NO:953 |
| R PCR 4 | CCTCCACACTCCTAAGGTCATC | SEQ ID NO:954 |

| *Primer Set 5* | | |
|---|---|---|
| T7 Primer 2 | gcgcAATTAATACGACTCACTA | SEQ ID NO:955 |
| R RT 1 | TGCATTGAGAGGGTGTAATTTGNNNNNNNNN | SEQ ID NO:947 |
| R PCR 1 | TGCATTGAGAGGGTGTAATTTG | SEQ ID NO:948 |

| *Primer Set 6* | | |
|---|---|---|
| T7 Primer 2 | gcgcAATTAATACGACTCACTA | SEQ ID NO:955 |
| RRT2 | GTATCTTGGGCCTGTTGACTTCNNNNNNNNN | SEQ ID NO:949 |
| R PCR 2 | GTATCTTGGGCCTGTTGACTTC | SEQ ID NO:950 |

| *Primer Set 7* | | |
|---|---|---|
| T7 primer 2 | gcgcAATTAATACGACTCACTA | SEQ ID NO:955 |
| R RT 3 | ATTGTTCCTTCAGCTGTTCCATNNNNNNNNN | SEQ ID NO:951 |
| R PCR 3 | ATTGTTCCTTCAGCTGTTCCAT | SEQ ID NO:952 |

| *Primer Set 8* | | |
|---|---|---|
| T7 Primer 2 | gcgcAATTAATACGACTCACTA | SEQ ID NO:955 |
| R RT 4 | CCTCCACACTCCTAAGGTCATCNNNNNNNNN | SEQ ID NO:953 |
| R PCR 4 | CCTCCACACTCCTAAGGTCATC | SEQ ID NO:954 |

| | | |
|---|---|---|
| Note: the protocol described below was carried out with all the primers shown above in TABLE 35, however it is briefly described with reference to Primer Set 1 in order to clearly present the three method steps: *First Strand cDNA synthesis:* was primed with the NSR7 primer pool comprising the T7 promoter (SEQ ID NO:934) plus one random nucleotide (N) plus the 933 6-mers (SEQ ID NOS:1-933) *Second Strand cDNA synthesis:was* carried out using the following primer pool: 5' TGCATTGAGAGGGTGTAATTTGNNNNNNNNN 3' (SEQ ID NO:947) (referred to as R RT 1 comprising random 9-mers plus second PCR primer binding site, or "tail"). | | |

*PCR Amplification* was primed using the following PCR Primers:
Forward PCR 1: 5' CGCAATTAATACGACTCACTATAGG 3' (SEQ ID NO:946) (binds to T7 promoter (first primer binding site))
Reverse PCR 1: 5' TGCATTGAGAGGGTGTAATTTG 3' (SEQ ID NO:947)
(binds to second PCR primer binding site)

### Additional Primer Sets Useful for generating aDNA:

Although the primer sets described above in Table 35 are used with NSR7 including the T7 promoter, it will be understood that the method of producing aDNA may be practiced using a pool of not-so-random primers (NSR) tailed with any defined sequence containing at least one primer binding sequence. For example, a primer pool was used comprising a pool of NSR primers that included the 933 6-mers (SEQ ID NOS:1-933) plus a single random nucleotide inserted between the 6-mer sequence and the first primer binding sequence, SEQ ID NO:956: 5' CCGAACTACCCACTTGCATT 3'
*First strand synthesis* was carried out using the NSR primer pool comprising the first primer binding sequence (SEQ ID NO:956) plus one random nucleotide (N) plus the 933 6-mers (SEQ ID NOS:1-933).
*Second strand synthesis* was carried out using the following primer pool:
5' CCACTCCATTTGTTCGTGTGNNNNNNNNN 3' (SEQ ID NO:957) (comprising random 9-mers plus a second PCR primer binding site.)
   *PCR amplification* of the double-stranded cDNA was then carried out using the following PCR primers:
   Forward PCR Primer: 5' CCGAACTACCCACTTGCATT 3' (SEQ ID NO:958) (binds to first PCR primer binding site)
   Reverse PCR Primer: 5' CCACTCCATTTGTTCGTGTG 3' (SEQ ID NO:959) (binds to second PCR primer binding site)

### Detailed Methods

*Test Samples:* Test samples 1-8 were carried out using Primer sets 1-8 shown above in TABLE 35 and were tested in parallel as described for primer set #1 below.

Control samples were included as follows:
1. A first strand reverse transcriptase reaction into second strand Klenow, but no N9 primer, followed by PCR amplification.
2. A first strand reverse transcriptase reaction, no second strand Klenow, followed by PCR amplification.

### First Strand Reverse Transcription:

### First strand reverse transcription was carried out as follows:

### Combine:

• 1 µl of 1µg/µl Jurkat total RNA template (obtained from Ambion, Inc. (Austin, TX)).
• 2 µl of 100 µM stock NSR7 primer pool
• 7 µl H₂O to a final volume of 10 µl.

Mixed and incubated at 65°C for 5 minutes, snap chilled on ice.

Added 10 µl of RT cocktail (prepared on ice) containing:
• 4 ul 5X First Strand Buffer (250 mM Tris-HCL, pH 8.3, 375 mM KCl, 15 mM MgCl₂)
• 2 µl 20 mM dNTP
• 1 µl H₂O
• 1 µl 0.1 M DTT
• 1 µl RNAse OUT (Invitrogen)
• 1 µl MMLV reverse transcriptase (200 units/µl) (SuperScript III™ (SSIII), Invitrogen Corporation, Carlsbad, CA)

The sample was mixed and transferred to a 40°C pre-warmed thermal cycler (to provide a "hot start"), and the sample was then incubated at 40°C for two hours, 70°C for 10 minutes and chilled to 4°C.

1 µl of RNAse H (1-4 units/µl) was then added and the sample was incubated at 37°C for 20 minutes, then heated to 95°C for 5 minutes and snap chilled at 4°C.

### Second Strand Synthesis

A second strand synthesis cocktail was prepared as follows:
• 10 µl 10X Klenow Buffer
• 10 µl N9 Reverse RT 1 Primer (SEQ ID NO:947)
• 2.5 µl 20 mM dNTPs
• 57.5 µl H₂O
• 0.33 µl Klenow enzyme (50 U/µl of NEB exo-)

80 µl of the second strand synthesis cocktail was added to the 20 µl first strand template reaction mixture, mixed and incubated at 37°C for 30 minutes.

### cDNA Purification

The resulting double stranded cDNA was purified using Spin Cartridges obtained from Invitrogen and buffers supplied in the kit according to the manufacturer's directions. A total volume of 30 µl was eluted from the column, of which 20 µl was used for follow-on PCR.

### PCR Amplification

The following mixture was added to the 20 µl of purified cDNA template:
• 20 µl 5X Roche Expand Plus PCR Buffer
• 2.5 µl 20 mM dNTPS
• 4 µl Forward PCR Primer (10 mM stock) (SEQ ID NO:946)
• 4 µl Reverse PCR Primer (10 mM stock) (SEQ ID NO:947)
• 1 µl Expand Enzyme (5U/µl) (Roche)
• 40 µl H₂O
• 10 µl 25 mM MgCl₂

### PCR Amplification Conditions:

25 total PCR cycles:
10 cycles of:
   • 94°C for 30 seconds
   • 55°C or 60°C for 30 seconds
   • 72°C for 1 minute
15 cycles of:
   • 94°C for 30 seconds
   • 55°C or 60°C for 30 seconds
   • 72°C for 1 minute (plus 10 seconds added to the elongation step with each cycle)
   • 72° for 7 minutes to polish and chilled at 4°C.

Results: The results were analyzed in terms of (1) measuring the level of amplification of selected reporter genes by qPCR, (2) measuring amplified DNA "aDNA" yield, and (3) evaluation of an aliquot of the aDNA on an agarose gel to confirm that the population of species in the cDNA were equally represented.

The data in TABLE 36 shows the raw abundance values (adjusted for dilution) for reporter genes GAPDH, GUSB, hPO and Actin following qPCR of the double-stranded cDNA and aDNA products for samples 1-10.

**TABLE 36**

| qPCR Values (raw values adjusted for dilution) | | | | | | | | | | | |
|---|---|---|---|---|---|---|---|---|---|---|---|
| | | **1** | **2** | **3** | **4** | **5** | **6** | **7** | **8** | **9** | **10** |
| GAPDH | ds-cDNA | 7380 | 6881 | 6122 | 9532 | 8173 | 7594 | 839 | 3585 | 7570 | 323 |
| | PCR-aDNA | 11990 | 19366 | 25184 | 35564 | 9926 | 17131 | 11701 | 15143 | 10564 | 34 |
| GUSB | ds-cDNA | 20 | 20 | 18 | 25 | 16 | 23 | 2 | 14 | 20 | 1 |
| | PCR-aDNA | 430 | 898 | 262 | 507 | 368 | 938 | 117 | 275 | 500 | 3 |
| hPO | ds-cDNA | 2365 | 2300 | 1578 | 2095 | 2490 | 2214 | 207 | 795 | 1097 | 54 |
| | PCR-aDNA | 32525 | 34686 | 14460 | 25778 | 20979 | 36267 | 5086 | 17396 | 4685 | 17 |
| Actin | ds-cDNA | 3088 | 2553 | 2362 | 3412 | 2829 | 3385 | 171 | 1417 | 2702 | 254 |
| | PCR-aDNA | 16551 | 11425 | 7172 | 9476 | 14503 | 13920 | 3305 | 4479 | 36772 | 174 |

The raw data shown above in Table 36 was then adjusted based on the following amplification factor:

### (PCR/cDNA) * (cDNA/average cDNA)

This amplification factor was introduced to weight amplification in favor of samples that had robust cDNA synthesis levels and robust PCR yields (e.g., sample 2), over samples with poor cDNA and PCR yields, but high levels of cDNA to PCR amplification (e.g., sample 7). The abundance values, adjusted based on the amplification factor described above, are shown below in TABLE 37.

**TABLE 37: qPCR results (amplification factor)**

| Sample | 1 | 2 | 3 | 4 | 5 | 6 | 7 | 8 | Control #1: No N9 Primer | Control #2: No Klenow step |
|---|---|---|---|---|---|---|---|---|---|---|
| µg PCR yield | 4.1 | 4.2 | 3.1 | 3.3 | 2.5 | 3.4 | 2.3 | 2.1 | 3.3 | 0.4 |
| GAPDH | 2.1 | 3.3 | 4.3 | 6.1 | 1.7 | 3.0 | 2.0 | 2.6 | 1.8 | 0.0 |
| GUSB | 27.0 | 56.3 | 16.4 | 31.8 | 23.1 | 58.8 | 7.3 | 17.3 | 31.3 | 0.2 |
| hPO | 21.4 | 22.8 | 9.5 | 17.0 | 13.8 | 23.9 | 3.3 | 11.4 | 3.1 | 0.0 |
| Actin | 7.5 | 5.2 | 3.2 | 4.3 | 6.5 | 6.3 | 1.5 | 2.0 | 16.6 | 0.1 |

As shown above in TABLE 37, PCR yield was generally good, especially with Primer Sets 1 and 2. Amplification was observed for all reporter genes tested. An aliquot of the aDNA from samples 1-8 and controls 1-2 were run on 1.6% agarose gels (not shown) and each test sample showed a smear of amplified material, indicating that the amplified products were representative of mRNA species present in the starting total RNA.

A second set of reactions were run with the same samples using the methods described above, but with the PCR annealing temperature raised to 60°C with a total of 30 cycles of PCR. qPCR was performed on the resulting aDNA using qPCR for the eight reporter genes shown in TABLE 38 that are known to be highly expressed in Jurkat cells.

**TABLE 38 : Reporter Genes for Jurkat Cells**

| **Assay #** | **Gene Symbol** | **Gene Name** |
|---|---|---|
| 1 | STMN1 | stathmin 1/oncoprotein 18 |
| 2 | CDCA7 | cell division cycle associated 7 |
| 3 | PPIA | peptidylprolyl isomerase A (cyclophilin A) |
| 4 | EIF3S3 | eukaryotic translation initiation factor 3, subunit 3 gamma, 40 kDa |
| 5 | NUCB2 | nucleobindin 2 |
| 6 | SRP14 | signal recognition particle 14 kDa (homologous Alu RNA binding protein) |
| 7 | MAPRE2 | microtubule-associated protein, RP/EB family member 2 |
| 8 | NDUFV2 | NADH dehydrogenase (ubiquinone) flavoprotein 2, 24 kDa |
| 9 | GAPDH | |
| 10 | GUSB | |
| 11 | hPO | |
| 12 | Actin | |

As shown in TABLE 39, the overall yield of aDNA obtained using an annealing temperature at 60°C during PCR amplification is greater than that obtained with 55°C for seven out of the eight primer sets tested.

**TABLE 39: PCR yield measured bv Nanodrop**

| **PCR Primer Set** | **1** | **2** | **3** | **4** | **5** | **6** | **7** | **8** |
|---|---|---|---|---|---|---|---|---|
| **55°C** | 3.1 | 2.6 | 1.6 | 2.8 | 1.1 | 2.6 | 2.1 | 2.1 |
| **60°C** | 3.8 | 3.2 | 2.1 | 3.2 | 1.7 | 2.7 | 2.0 | 2.5 |
| **total PCR yield (µg)** | 6.9 | 5.8 | 3.7 | 6.0 | 2.9 | 5.2 | 4.1 | 4.7 |

**TABLE 40: PCR Specificity as measured by qPCR, expressed as the ratio of aDNA to cDNA**

| **Gene ID** | **temp** | **Primer set 1** | **2** | **3** | **4** | **5** | **6** | **7** | **8** |
|---|---|---|---|---|---|---|---|---|---|
| 1 | 55 | 97 | 25 | 15 | 21 | 11 | 25 | 9 | 43 |
| 1 | 60 | 188 | 49 | 66 | 62 | 28 | 40 | 18 | 97 |
| 2 | 55 | 9 | 6 | 3 | 3 | 3 | 5 | 2 | 2 |
| 2 | 60 | 17 | 9 | 5 | 4 | 7 | 7 | 3 | 2 |
| 3 | 55 | 101 | 55 | 31 | 68 | 13 | 51 | 23 | 44 |
| 3 | 60 | 105 | 68 | 32 | 89 | 18 | 49 | 34 | 53 |
| 4 | 55 | 38 | 18 | 8 | 18 | 8 | 15 | 7 | 11 |
| 4 | 60 | 38 | 19 | 9 | 26 | 13 | 14 | 10 | 13 |
| 5 | 55 | 35 | 20 | 4 | 5 | 4 | 15 | 3 | 9 |
| 5 | 60 | 75 | 48 | 24 | 18 | 11 | 27 | 6 | 25 |
| 6 | 55 | 10 | 5 | 1 | 1 | 1 | 5 | 2 | 2 |
| 6 | 60 | 21 | 12 | 7 | 3 | 2 | 4 | 3 | 2 |
| 7 | 55 | 39 | 20 | 7 | 14 | 9 | 18 | 5 | 9 |
| 7 | 60 | 53 | 36 | 7 | 20 | 24 | 23 | 6 | 10 |
| 8 | 55 | 61 | 46 | 13 | 28 | 13 | 42 | 8 | 12 |
| 8 | 60 | 67 | 59 | 12 | 37 | 17 | 39 | 12 | 13 |
| 9 | 55 | 1 | 1 | 1 | 2 | 0 | 2 | 1 | 4 |
| 9 | 60 | 3 | 2 | 5 | 5 | 0 | 1 | 1 | 4 |
| 10 | 55 | 16 | 11 | 3 | 4 | 2 | 16 | 3 | 10 |
| 10 | 60 | 20 | 26 | 13 | 17 | 12 | 23 | 6 | 19 |
| 11 | 55 | 10 | 12 | 3 | 9 | 1 | 9 | 3 | 6 |
| 11 | 60 | 10 | 15 | 4 | 11 | 1 | 6 | 3 | 5 |
| 12 | 55 | 4 | 3 | 2 | 2 | 1 | 3 | 3 | 2 |
| 12 | 60 | 3 | 5 | 3 | 3 | 0 | 2 | 2 | 1 |

As shown in TABLE 40, quantitative PCR (qPCR) analysis of the aDNA from PCR reactions annealed at 55°C or 60°C showed that primer pair 1 at a 60°C annealing temperature provided the best PCR specificity as measured by the ratio of aDNA to cDNA. It was further determined that the specific activity of reporter genes tracked closely with aDNA yields over the range of PCR cycles tested (4 cycles to 24 cycles) (data not shown).

### EXAMPLE 12

This Example demonstrates that aDNA amplified from double stranded cDNA templates generated using the 933 6-mers (SEQ ID NOS:1-933), as described in EXAMPLE 11, preserved the expression ratios observed in the double-stranded cDNA templates.

### Methods:

First strand cDNA synthesis was performed as described in Example 11 using 1µg of total RNA template from Jurkat or K562 cells (obtained from Ambion, Inc. Austin TX) with the NSR7 primer pool. Two Jurkat samples were run in parallel. For second strand synthesis and cDNA purification were also carried out as described in Example 11.

### PCR Amplification of cDNA

The cDNA template was diluted 10-fold and 100-fold prior to use in PCR reaction, and either 10 µl purified, or 10 µl diluted cDNA was used.

Add to the 10 µl of cDNA template:
• 20 µl 5X Roche Expand Plus PCR Buffer
• 2.0 µl 10 mM dNTPS
• 4 µl Forward PCR Primer (10 mM stock) (SEQ ID NO:946)
• 4 µl Reverse PCR Primer (10 mM stock) (SEQ ID NO:947)
• 1 µl Expand Enzyme (5U/µl) (Roche)
• 60 µl H₂O
• 10 µl 25 mM MgCl₂

### PCR Amplification Conditions:

Perform 30 total PCR cycles:
10 cycles of:
   • 94°C for 30 seconds
   • 60°C for 30 seconds
   • 72°C for 1 minute
20 cycles of:
   • 94°C for 30 seconds
   • 60°C for 30 seconds
   • 72°C for 1 minute (plus 10 seconds added to the elongation step with each cycle)
   • 72°C for 7 minutes to polish and chill at 4°C.

Post-PCR clean-up: 100 µl PCR reaction was diluted with 500 µl buffer and purified using Spin Cartridges obtained from Invitrogen and buffers supplied in the kit according to the manufacturer's directions. A total volume of 55 µl was eluted from the column.

Results: The results were analyzed in terms of total aDNA yield, evaluation of aDNA on agarose gels to verify the presence of the expected smear indicating amplification of substantially all species in the transcriptome, and by measuring a panel of reporter genes by qPCR.

**TABLE 41: Yield of PCR product (aDNA) measured by Nanodrop**

| **PCR Sample** | **ug total aDNA in PCR Reaction (nanodrop)** |
|---|---|
| Jurkat #1 (10 µl) | 2.6 |
| Jurkat #2 (10 µl) | 2.5 |
| K562 #1 (10 µl) | 1.7 |
| Jurkat #1 (1 µl) | 2.2 |
| Jurkat #2 (1 µl) | 2.1 |
| K562 #1 (1 µl) | 1.9 |
| Jurkat #1 (0.1 µl) | 2.0 |
| Jurkat #2 (0.1 µl) | 1.9 |
| K562 #1 (0.1 µl) | 2.0 |
| no template control | 0.5 |

Results: As shown in TABLE 41, the yields of aDNA were consistent across the range of template inputs. These results indicate that the PCR reaction was saturated at the lower template input. The agarose gel analysis showed the expected smear indicating good amplification across the transcriptome (not shown).

In order to determine if the aDNA generated from the cDNA preserved the target genes expression levels present in the cDNA, quantitative PCR analysis was conducted with 54 randomly chosen TaqMan reagents, detecting Gene IDS #1-34. As shown in TABLE 42, of the 54 randomly chosen target genes, measurable signal was measured for 34 genes in both NSR7-primed cDNA and aDNA generated therefrom (from 10 µl cDNA template input). The gene expression signal shown below in Table 42 was calculated as power (10 (log 10(1/2) * Ct +10)).

**TABLE 42: qPCR abundance values. Values expressed as 10 raised to the (Cts x log10(1/2) + 10) power**

| | **cDNA** | | | **aDNA (from 10 ul input Template)** | | |
|---|---|---|---|---|---|---|
| **Gene ID** | **Jurkat #1** | **Jurkat #2** | **K562 #1** | **Jurkat #1** | **Jurkat #2** | **K562 #1** |
| 1 | 10 | 7 | 5 | 57 | 50 | 98 |
| 2 | 12 | 14 | 8 | 87 | 109 | 8 |
| 3 | 15 | 54 | 0 | 339 | 619 | 24 |
| 4 | 20 | 28 | 47 | 245 | 277 | 500 |
| 5 | 447 | 954 | 160 | 16840 | 27201 | 7321 |
| 6 | 1726 | 5528 | 1458 | 349878 | 455053 | 202163 |
| 7 | 0 | 1 | 0 | 3 | 30 | 9 |
| 8 | 93 | 273 | 17 | 7315 | 10373 | 1010 |
| 9 | 166 | 417 | 594 | 11335 | 11122 | 16157 |
| 10 | 255 | 522 | 238 | 17105 | 19957 | 8002 |
| 11 | 1 | 3 | 0 | 79 | 107 | 5 |
| 12 | 7130 | 11637 | 1062 | 635546 | 790841 | 177300 |
| 13 | 281 | 642 | 76 | 11218 | 13634 | 2909 |
| 14 | 364 | 1141 | 83 | 21810 | 26586 | 3022 |
| 15 | 40 | 97 | 0 | 3499 | 3570 | 20 |
| 16 | 1 | 1 | 0 | 4 | 10 | 1 |
| 17 | 24 | 68 | 24 | 1153 | 1366 | 915 |
| 18 | 2764 | 7517 | 598 | 59021 | 62854 | 14790 |
| 19 | 688 | 1362 | 288 | 24491 | 33524 | 13175 |
| 20 | 65 | 148 | 23 | 3900 | 3834 | 655 |
| 21 | 8 | 19 | 4 | 138 | 189 | 52 |
| 22 | 600 | 1011 | 205 | 118107 | 129372 | 54354 |
| 23 | 0 | 2 | 0 | 14 | 45 | 5 |
| 24 | 1059 | 3831 | 87 | 117411 | 123053 | 4357 |
| 26 | 15 | 33 | 4 | 468 | 673 | 12 |
| 27 | 27 | 94 | 68 | 5051 | 5570 | 5316 |
| 29 | 184 | 236 | 0 | 932 | 1105 | 5 |
| 30 | 29 | 58 | 28 | 3259 | 3095 | 1580 |
| 31 | 201 | 816 | 64 | 5142 | 5825 | 1826 |
| 32 | 2 | 5 | 2 | 72 | 49 | 103 |
| 33 | 105 | 258 | 82 | 1978 | 2770 | 2260 |
| 34 | 836 | 1560 | 513 | 3312 | 3906 | 2202 |

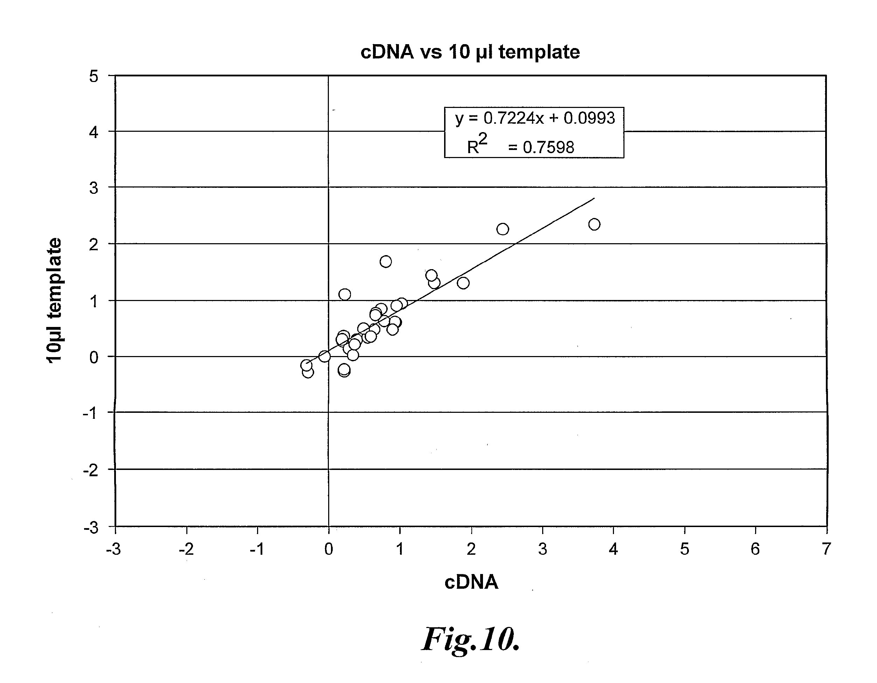

As shown in TABLE 42, the results of the Jurkat and K562 experiments show a good correlation in expression ratios between the cDNA and the aDNA derived from the cDNA. FIGURE 10 shows a plot of the expression values from TABLE 42 measured in the cDNA (x-axis) versus the aDNA (y-axis). Importantly, the results shown in FIGURE 10 demonstrate the preservation of expression ratios from the actual sample (cDNA) to the PCR amplified material (aDNA), such that gene x is y-fold amplified by the NSR-PCR regardless of whether its expression is high or low. Overall, these experiments indicates that NSR PCR aDNA may be used to amplify total RNA to aDNA while preserving expression ratios.

### EXAMPLE 13

This Example demonstrates that aDNA amplified from double stranded cDNA templates generated using the 933 6-mers (SEQ ID NOS:1-933), as described in EXAMPLE 11, preserved the enrichment of target genes relative to rRNA and globin.

Rationale: To determine whether the enrichment of target genes relative to rRNA and globin previously observed from in-vitro transcription products generated from NSR-primer cDNA is preserved in amplified DNA product generated from PCR amplification of the NSR-primed cDNA.

Methods: In order to determine whether the enrichment of target genes relative to rRNA and globin previously observed from in-vitro transcription products generated from NSR-primer cDNA is preserved in amplified DNA product generated from PCR amplification of the NSR-primed cDNA, the amplified aDNA product obtained from cDNA generated using NSR7 primers was compared to the amplified aDNA product obtained from cDNA generated using random 8-mers in the following experiment.

First strand cDNA synthesis was performed on 1µg of total RNA isolated from whole blood, as described in Example 11 with duplicate samples generated with either (1) the NSR7 primer pool (comprising SEQ ID NOS: 1-933 that each had the T7 promoter (SEQ ID NO: 934) covalently attached at the 5' end); or (2) a random 8-mer (N8) primer pool with the T7 promoter (SEQ ID NO: 934) covalently attached at the 5' end.

Second strand synthesis and cDNA purification was performed as described in Example 11, with either SEQ ID NO: 947 for both the NSR7 primed samples, and the random 8-mers (N8) primed samples.

### PCR Amplification of cDNA

Either 1 µl + 9 µl H20 or 10 µl cDNA template was used in the follow-on PCR reaction as follows:
Add to the 10 µl of cDNA template:
   • 20 µl 5X Roche Expand Plus PCR Buffer (includes 1.5mM MgCl₂)
   • 1.0 µl 20 mM dNTPS
   • 4 µl Forward PCR Primer (10 mM stock) (SEQ ID NO:949)
   • 4 µl Reverse PCR Primer (10 mM stock) (SEQ ID NO:948)
   • 1 µl Expand Enzyme (5U/µl) (Roche)
   • 60 µl H₂O

### PCR Amplification Conditions:

Perform 20 total PCR cycles:
10 cycles of:
   • 94°C for 30 seconds
   • 60°C for 30 seconds
   • 72°C for 1 minute
10 cycles of:
   • 94°C for 30 seconds
   • 60°C for 30 seconds
   • 72°C for 1 minute (plus 10 seconds added to the elongation step with each cycle)
   • 72° for 7 minutes to polish and chill at 4°C.

Post-PCR clean-up: 100 µl PCR reaction was diluted with 500 µl buffer and purified using Spin Cartridges obtained from Invitrogen and buffers supplied in the kit according to the manufacturer's directions. A total volume of 50 µl was eluted from the column.

Results: The data in TABLE 43 shows the ratio of qPCR abundance values for NSR-primed to N8-primed cDNA (adjusted for dilution) for the following reporter genes: A= IGF1R; B= GAPDH, C= STMN1, and D= CDCA7 compared to background gene expression measured for 18S, 28S, HBA1, HBA2 or HBB.

**TABLE 43: qPCR results**

| | | cDNA #1 (5 ul) | cDNA #2 (5 ul) | PCR #1 (10 ul) | PCR #1 (1 ul) | PCR #2 (10 ul) | PCR #2 (1 ul) |
|---|---|---|---|---|---|---|---|
| Gene A/18S | NSR/N8 | 79 | 88 | 1162 | 3732 | 1345 | 4677 |
| Gene B/18S | NSR/N8 | 162 | 178 | 103 | 311 | 112 | 310 |
| Gene C/18S | NSR/N8 | 64 | 71 | 732 | 1861 | 846 | 3329 |
| Gene D/18S | NSR/N8 | 87 | 102 | 236 | 937 | 253 | 811 |
| Gene A/28S | NSR/N8 | 23 | 31 | 193 | 300 | 190 | 287 |
| Gene B/28S | NSR/N8 | 46 | 63 | 17 | 25 | 16 | 19 |
| Gene C/28S | NSR/N8 | 18 | 25 | 122 | 150 | 119 | 204 |
| Gene D/28S | NSR/N8 | 25 | 36 | 39 | 75 | 36 | 50 |
| Gene A/HBA | NSR/N8 | 42 | 54 | 159 | 746 | 269 | 532 |
| Gene B/HBA | NSR/N8 | 86 | 111 | 14 | 62 | 22 | 35 |
| Gene C/HBA | NSR/N8 | 34 | 44 | 100 | 372 | 169 | 379 |
| Gene D/HBA | NSR/N8 | 46 | 63 | 32 | 187 | 51 | 92 |
| Gene A/HBB | NSR/N8 | 6 | 7 | 125 | 139 | 155 | 163 |
| Gene B/HBB | NSR/N8 | 13 | 14 | 11 | 12 | 13 | 11 |
| Gene C/HBB | NSR/N8 | 5 | 5 | 79 | 70 | 98 | 116 |
| Gene D/HBB | NSR/N8 | 7 | 8 | 25 | 35 | 29 | 28 |

The data shown above in TABLE 43 shows a dramatic enrichment of target genes over background expression in aDNA amplified from NSR7 primed cDNA. The observed enrichment is consistently better with lower template inputs. Overall, it is clear that the three step amplification method (NSR-PCR) described in EXAMPLE 11 provides a significant enrichment of target genes over rRNAs and globins. Therefore, the NSR-PCR method described in EXAMPLE 11 could be used for full transcriptome profiling of total RNA in a biological sample, such as whole blood.

### EXAMPLE 14

This Example shows that the yield of amplified DNA can be increased in a PCR reaction through the use of an elevated concentration of primers, dNTPs and MgCl₂, thereby allowing for the implementation of NSR-PCR in high-throughput applications.

Rationale: An Experiment was carried out to measure total aDNA yields from a PCR reaction using a cDNA template as a function of primer input concentrations at two different dNTP and Mg++ concentrations as follows.

### Methods:

Template: cDNA was prepared from Jurkat total RNA using NSR7 primers, as described in Example 13.

PCR Primers:
Forward: SEQ ID NO:949; Reverse: SEQ ID NO:948
Primer Concentrations tested: 200, 400, 600, 800, and 1000 nM
dNTP concentrations tested: 200 µM, 1.5 mM
Mg++ concentrations tested: 500 µM, 4 mM

PCR reactions were prepared as described in Example 13.

PCR Amplification Conditions:

Perform 30 total PCR cycles:
10 cycles of:
   • 94°C for 30 seconds
   • 60°C for 30 seconds
   • 72°C for 1 minute
   and

20 cycles of:
   • 94°C for 30 seconds
   • 60°C for 30 seconds
   • 72°C for 1 minute (plus 10 seconds added to the elongation step with each cycle)
   • 72°C for 7 minutes to polish and chill at 4°C.

Post-PCR clean-up: 100 µl PCR reaction was diluted with 500 µl buffer and purified using Spin Cartridges obtained from Invitrogen and buffers supplied in the kit according to the manufacturer's directions. A total volume of 50 µl was eluted from the column.

**TABLE 44: Yield of PCR product (aDNA), raw data**

| | 200 uM | 500 uM | 200 uM | 500 uM |
|---|---|---|---|---|
| | dNTP/1.5 mM | dNTP/4 mM | dNTP/1.5 mM | dNTP/4 mM |
| | Mg++ | Mg++ | Mg++ | Mg++ |
| | ng PCR product/ µl cDNA template | | ng PCR product/100 µl PCR reaction | |
| 200 nM dNTPs | 27.96 | 36.47 | 2.796 | 3.647 |
| 400 nM dNTPs | 48.36 | 60.51 | 4.836 | 6.051 |
| 600 nM dNTPs | 54.72 | 76.72 | 5.472 | 7.672 |
| 800 nM dNTPs | 60.61 | 94.32 | 6.061 | 9.432 |
| 1000 nM dNTPs | 59.62 | 96.87 | 5.962 | 9.687 |

The results shown above in TABLE 44 indicate that the yields of aDNA at 800 nM primer, 500 uM dNTPs and 4 mM Mg++ approach 10 µg per a 100 µl PCR reaction. The aDNA was also analyzed by agarose gel and was confirmed to contain a smear of amplified material, indicating that the amplified products were representative of mRNA species present in the starting total RNA.

In order to further assess the presence of amplified full transcript material versus the amplification of non-specific material, the panel of genes 1-8 shown in TABLE 38 were analyzed by qPCR. Control sample 9 was 18S rRNA and control sample 10 was 28S rRNA?

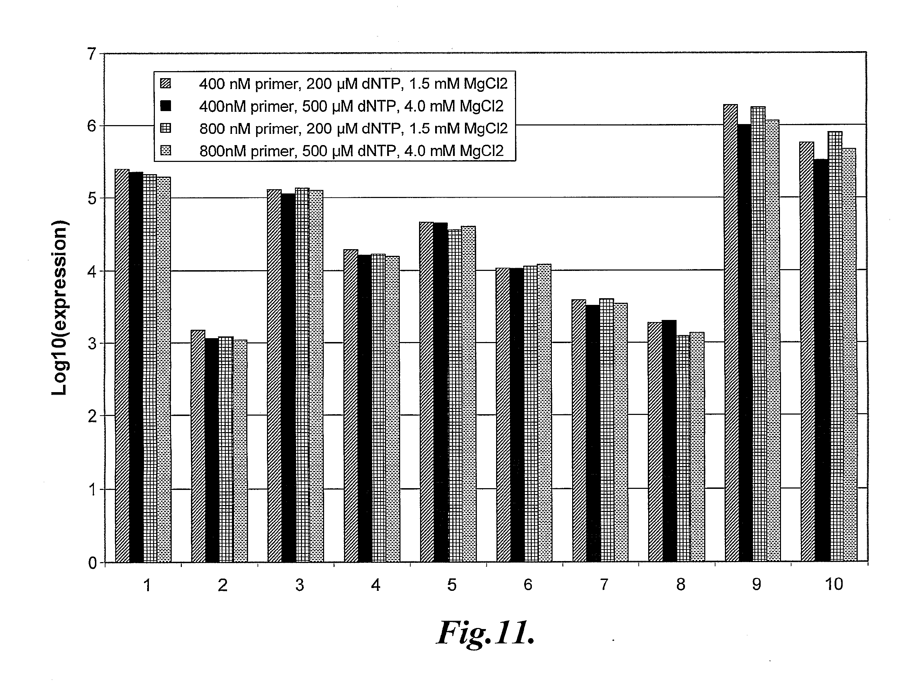

FIGURE 11 shows the results of the qPCR plotted as the log 10 expression of each of the genes. As shown, the amplified aDNA yields produced in the PCR reaction having 800 nM primer, 500 uM dNTP and 4 mM MgCl₂ have the same gene specific activities as aDNA produced at lower primer, dNTP and Mg++ concentrations. Although the values of control sample 9 (18S rRNA) and sample 10 (28S rRNA) shown in FIGURE 11 are higher than some of the reporter genes, it is important to note that these values (18S and 28S RNA) have actually been reduced by approximately 99 fold in comparison to the amount of rRNA present in samples that are randomly primed (e.g., not primed using the NSR primers). Because rRNA constitutes about 98% of total RNA, even when the abundance of the rRNA is reduced to 1% of the original value, the total amount present still represents about 1/3 of the RNA in the sample. Importantly, this 99% decrease in rRNA observed in samples treated in accordance with the methods of the invention has been observed by the present inventors to be more than sufficient to eliminate the problem of rRNA obscured signal (i.e. "cross-talk") typically observed in samples that are randomly primed (data not shown).

These data demonstrate that the high yield of aDNA generated using the optimized PCR conditions contain amplified full transcript material, and the increased yield is not due to the presence of non-specific material generated during the amplification reaction.

### EXAMPLE 15

This Example describes methods that are useful to label the aDNA PCR products for subsequent use in gene expression monitoring applications.

### 1. Direct Chemical Coupling of Fluorescent Label to the PCR Product

Cy3 and Cy5 direct label kits were obtained from Mirus (Madison, WI, kit MIR product numbers 3625 and 3725).

10 µg of PCR product (aDNA), obtained as described in Example 11, was incubated with labeling reagent as described by the manufacturer. The labeling reagents covalently attached Cy3 or Cy5 to the nucleic acid sample, which can then be used in almost any molecular biology application, such as gene expression monitoring. The labeled aDNA was then purified and its fluorescence was measured relative to the starting label.

Results: Four aDNA samples were labeled as described above, and fluorescence was measured. A range of 0.9 to 1.5% of retained label was observed across the four labeled aDNA samples (otherwise referred to as a labeling efficiency of 0.9 to 1.5%). These results fall within the 1% to 3% labeling efficiency typically observed for aaUTP labeled, in vitro translated, amplified RNA.

### 2. Incorporation of aminoallyl modified dUTP (aadUTP) during PCR with an aDNA template using one primer (forward or reverse) to yield aa-labeled, single-stranded aDNA.

Methods: 1 µg of the aDNA PCR product, generated using the NSR7 primer pool as described in Example 11, is added to a PCR reaction mix as follows:
• 100 to 1000 µM aadUTP+dCTP+cATP+dGTP+dUTP (the optimal balance of aadUTP to dUTP may be empirically determined using routine experimentation)
• 4 mM MgCl₂
• 400-1000 nM of only the forward or reverse primer (e.g., Forward: SEQ ID NO:949; or Reverse: SEQ ID NO:948), but not both.

PCR Reaction: 5 to 20 cycles of PCR (94°C 30 seconds, 60°C 30 seconds, 72°C 30 seconds), during which time only one strand of the double-stranded PCR template is synthesized. Each cycle of PCR is expected to produce one copy of the aa-labeled, single-stranded aDNA. This PCR product is then purified and a Cy3 or Cy5 label is incorporated by standard chemical coupling.

### 3. Incorporation of aminoallyl modified dUTP (aadUTP) during PCR with an aDNA template using forward and reverse primers to yield aa-labeled, double-stranded aDNA.

Methods: 1 µg of the aDNA PCR product, generated using the NSR7 primer pool as described in Example 11, is added to a PCR reaction mix as follows:
• 100 to 1000 µM aadUTP+dCTP+cATP+dGTP+dUTP (the optimal balance of aadUTP to dUTP may be empirically determined using routine experimentation)
• 4 mM MgCl₂
• 400-1000 nM of the forward and reverse primer (e.g., Forward: SEQ ID NO:949; or Reverse: SEQ ID NO:948)

PCR Reaction: 5 to 20 cycles of PCR (94°C 30 seconds, 60°C 30 seconds, 72°C 30 seconds), during which time both strands of the double-stranded PCR template are synthesized. The double-stranded, aa-labeled aDNA PCR product is then purified and a Cy3 or Cy5 label is incorporated by standard chemical coupling.

While the preferred embodiment of the invention has been illustrated and described, it will be appreciated that various changes can be made therein without departing from the scope of the invention.

## Claims

1. A method of selectively amplifying a target population of nucleic acid molecules, the method comprising the step of using a population of oligonucleotides to prime the amplification of a target population of nucleic acid molecules within a larger population of nucleic acid molecules, wherein:
the population of oligonucleotides is selected to hybridize under defined conditions to a first subpopulation of the target nucleic acid population, but not hybridize under the defined conditions to a second subpopulation of the target nucleic acid population.

2. The method of Claim 1, wherein each oligonucleotide comprises a hybridizing portion, wherein the hybridizing portion consists of one of 6, 7, or 8 nucleotides

3. The method of Claim 2, wherein each oligonucleotide further comprises a defined sequence portion located 5' to the hybridizing portion.

4. The method of Claim 3, wherein the defined sequence portion comprises a transcriptional promoter, or a primer binding site for PCR amplification.

5. The method of any one of Claims 2 to 4, wherein the population of hybridizing portions is selected from the oligonucleotides comprising SEQ ID NOS:1-933.

6. The method of any preceding claim, wherein the target population of nucleic acid molecules comprises all mRNA molecules obtained from mammalian blood cells.

7. The method of any preceding claim, wherein the second subpopulation of the target nucleic acid population consists essentially of the most abundant nucleic acid molecules in the target population of nucleic acid molecules, wherein the most abundant nucleic acid molecules are selected from the group consisting of ribosomal RNA, ribosomal DNA, globin DNA, or globin RNA.

8. A method of selectively amplifying a target population of nucleic acid molecules, the method comprising the step of using a population of oligonucleotides to prime the amplification of a target population of nucleic acid molecules within a larger population of nucleic acid molecules, wherein each oligonucleotide within the population of oligonucleotides comprises a hybridizing portion and a defined sequence portion located 5' to the hybridizing portion, wherein the hybridizing portion is a member of the population of oligonucleotides comprising SEQ ID NOS:1-933.

9. A population of oligonucleotides comprising at least 10% of the oligonucleotides comprising SEQ ID NOS:1-933.

10. A reagent for selectively amplifying a target population of nucleic acid molecules, the reagent comprising at least 10% of the oligonucleotides comprising SEQ ID NOS:1-933.

11. A reagent for selectively amplifying a target population of nucleic acid molecules, the reagent comprising a population of oligonucleotides to prime the amplification of a target population of nucleic acid molecules, wherein each oligonucleotide comprises a hybridizing portion that consists of 6, 7 or 8 nucleotides.

12. The reagent of Claim 11, wherein each oligonucleotide further comprises a defined sequence portion located 5' to the hybridizing portion, and wherein the hybridizing portion is a member of the population of oligonucleotides comprising SEQ ID NOS:1-933.

13. A kit for selectively amplifying a target population of nucleic acid molecules, the kit comprising a reagent comprising a population of oligonucleotides, wherein each oligonucleotide comprises a hybridizing portion that consists of 6, 7 or 8 nucleotides.

14. A method of selectively amplifying a target population of nucleic acid molecules, the method comprising the steps of:
(a) providing a population of oligonucleotides, wherein each oligonucleotide comprises a hybridizing portion and transcriptional promoter portion located 5' to the hybridizing portion, wherein the hybridizing portion is a member of the population of oligonucleotides comprising SEQ ID NOS:1-933;
(b) annealing the population of oligonucleotides to a sample comprising mRNA isolated from a mammalian subject;
(c) synthesizing cDNA from the mRNA using a reverse transcriptase enzyme;
(d) synthesizing double stranded cDNA using a DNA polymerase; and
(e) transcribing the double-stranded cDNA into RNA using an RNA polymerase that binds to the transcriptional promoter portion of each oligonucleotide to generate amplified RNA.

15. A method of selectively amplifying a target population of nucleic acid molecules, the method comprising the steps of:
(a) providing a first population of oligonucleotides, wherein each oligonucleotide comprises a hybridizing portion and a first PCR primer binding site located 5' to the hybridizing portion, wherein the hybridizing portion is a member of the population of oligonucleotides comprising SEQ ID NOS:1-933;
(b) annealing the population of oligonucleotides to a sample comprising mRNA isolated from a mammalian subject;
(c) synthesizing cDNA from the mRNA using a reverse transcriptase enzyme;
(d) synthesizing double stranded cDNA using a DNA polymerase and a second population of oligonucleotides, wherein each oligonucleotide comprises a random hybridizing portion and a second PCR binding site located 5' to the hybridizing portion; and
(e) PCR amplifying the double stranded cDNA using thermostable DNA polymerase, a first PCR primer that binds to the first PCR primer binding site and a second PCR primer that binds to the second PCR primer binding site to generate amplified double stranded DNA.


SEQUENCE LISTING

<110>  Life Technologies Corporation

<120>  Nucleic Acid Amplification Using Non-Random Primers

<130>  P16571EP-1

<150>  US 60/790,458
<151>  2006-04-07

<150>  US 60/731,529
<151>  2005-10-27

<160> 959

<170> FastSEQ for Windows Version 4.0

<210> 1
<211> 6
<212> DNA
<213> Artificial Sequence

<220>
<223> oligonucleotide 6-mer sequences

<400> 1
cgtttt                                                                 6

<210> 2
<211> 6
<212> DNA
<213> Artificial Sequence

<220>
<223> oligonucleotide 6-mer sequences

<400> 2
agtttt                                                                 6

<210> 3
<211> 6
<212> DNA
<213> Artificial Sequence

<220>
<223> oligonucleotide 6-mer sequences

<400> 3
gcgttt                                                                 6

<210> 4
<211> 6
<212> DNA
<213> Artificial Sequence

<220>
<223> oligonucleotide 6-mer sequences

<400> 4
acgttt                                                                 6

<210> 5
<211> 6

<212> DNA
<213> Artificial Sequence

<220>
<223> oligonucleotide 6-mer sequences

<400> 5
ctcttt 6

<210> 6
<211> 6
<212> DNA
<213> Artificial Sequence

<220>
<223> oligonucleotide 6-mer sequences

<400> 6
tacttt 6

<210> 7
<211> 6
<212> DNA
<213> Artificial Sequence

<220>
<223> oligonucleotide 6-mer sequences

<400> 7
ctattt 6

<210> 8
<211> 6
<212> DNA
<213> Artificial Sequence

<220>
<223> oligonucleotide 6-mer sequences

<400> 8
tcattt 6

<210> 9
<211> 6
<212> DNA
<213> Artificial Sequence

<220>
<223> oligonucleotide 6-mer sequences

<400> 9
caattt 6

<210> 10
<211> 6
<212> DNA
<213> Artificial Sequence

<220>
<223> oligonucleotide 6-mer sequences

<400> 10
aaattt 6

<210> 11

<211> 6
<212> DNA
<213> Artificial Sequence

<220>
<223> oligonucleotide 6-mer sequences

<400> 11
ggtgtt                                                                  6

<210> 12
<211> 6
<212> DNA
<213> Artificial Sequence

<220>
<223> oligonucleotide 6-mer sequences

<400> 12
agtgtt                                                                  6

<210> 13
<211> 6
<212> DNA
<213> Artificial Sequence

<220>
<223> oligonucleotide 6-mer sequences

<400> 13
tatgtt                                                                  6

<210> 14
<211> 6
<212> DNA
<213> Artificial Sequence

<220>
<223> oligonucleotide 6-mer sequences

<400> 14
gatgtt                                                                  6

<210> 15
<211> 6
<212> DNA
<213> Artificial Sequence

<220>
<223> oligonucleotide 6-mer sequences

<400> 15
ccggtt                                                                  6

<210> 16
<211> 6
<212> DNA
<213> Artificial Sequence

<220>
<223> oligonucleotide 6-mer sequences

<400> 16
aaggtt                                                                  6

<210> 17
<211> 6
<212> DNA
<213> Artificial Sequence

<220>
<223> oligonucleotide 6-mer sequences

<400> 17
gtcgtt                                                                 6

<210> 18
<211> 6
<212> DNA
<213> Artificial Sequence

<220>
<223> oligonucleotide 6-mer sequences

<400> 18
agcgtt                                                                 6

<210> 19
<211> 6
<212> DNA
<213> Artificial Sequence

<220>
<223> oligonucleotide 6-mer sequences

<400> 19
tccgtt                                                                 6

<210> 20
<211> 6
<212> DNA
<213> Artificial Sequence

<220>
<223> oligonucleotide 6-mer sequences

<400> 20
tacgtt                                                                 6

<210> 21
<211> 6
<212> DNA
<213> Artificial Sequence

<220>
<223> oligonucleotide 6-mer sequences

<400> 21
aacgtt                                                                 6

<210> 22
<211> 6
<212> DNA
<213> Artificial Sequence

<220>
<223> oligonucleotide 6-mer sequences

<400> 22
cgagtt                                                                 6

<210> 23
<211> 6
<212> DNA
<213> Artificial Sequence

<220>
<223> oligonucleotide 6-mer sequences

<400> 23
tgtctt                                                                  6

<210> 24
<211> 6
<212> DNA
<213> Artificial Sequence

<220>
<223> oligonucleotide 6-mer sequences

<400> 24
actctt                                                                  6

<210> 25
<211> 6
<212> DNA
<213> Artificial Sequence

<220>
<223> oligonucleotide 6-mer sequences

<400> 25
tatctt                                                                  6

<210> 26
<211> 6
<212> DNA
<213> Artificial Sequence

<220>
<223> oligonucleotide 6-mer sequences

<400> 26
gatctt                                                                  6

<210> 27
<211> 6
<212> DNA
<213> Artificial Sequence

<220>
<223> oligonucleotide 6-mer sequences

<400> 27
gtcctt                                                                  6

<210> 28
<211> 6
<212> DNA
<213> Artificial Sequence

<220>
<223> oligonucleotide 6-mer sequences

<400> 28

cccctt                                                                  6

<210> 29
<211> 6
<212> DNA
<213> Artificial Sequence

<220>
<223> oligonucleotide 6-mer sequences

<400> 29
ctactt                                                                  6

<210> 30
<211> 6
<212> DNA
<213> Artificial Sequence

<220>
<223> oligonucleotide 6-mer sequences

<400> 30
acactt                                                                  6

<210> 31
<211> 6
<212> DNA
<213> Artificial Sequence

<220>
<223> oligonucleotide 6-mer sequences

<400> 31
cgtatt                                                                  6

<210> 32
<211> 6
<212> DNA
<213> Artificial Sequence

<220>
<223> oligonucleotide 6-mer sequences

<400> 32
agtatt                                                                  6

<210> 33
<211> 6
<212> DNA
<213> Artificial Sequence

<220>
<223> oligonucleotide 6-mer sequences

<400> 33
tctatt                                                                  6

<210> 34
<211> 6
<212> DNA
<213> Artificial Sequence

<220>
<223> oligonucleotide 6-mer sequences

<400> 34
actatt                                                                 6

<210> 35
<211> 6
<212> DNA
<213> Artificial Sequence

<220>
<223> oligonucleotide 6-mer sequences

<400> 35
tatatt                                                                 6

<210> 36
<211> 6
<212> DNA
<213> Artificial Sequence

<220>
<223> oligonucleotide 6-mer sequences

<400> 36
gatatt                                                                 6

<210> 37
<211> 6
<212> DNA
<213> Artificial Sequence

<220>
<223> oligonucleotide 6-mer sequences


<400> 37
catatt                                                                 6

<210> 38
<211> 6
<212> DNA
<213> Artificial Sequence

<220>
<223> oligonucleotide 6-mer sequences

<400> 38
gtgatt                                                                 6

<210> 39
<211> 6
<212> DNA
<213> Artificial Sequence

<220>
<223> oligonucleotide 6-mer sequences

<400> 39
atgatt                                                                 6

<210> 40
<211> 6
<212> DNA
<213> Artificial Sequence

<220>

<223> oligonucleotide 6-mer sequences

<400> 40
gcgatt                                                                  6

<210> 41
<211> 6
<212> DNA
<213> Artificial Sequence

<220>
<223> oligonucleotide 6-mer sequences

<400> 41
acgatt                                                                  6

<210> 42
<211> 6
<212> DNA
<213> Artificial Sequence

<220>
<223> oligonucleotide 6-mer sequences

<400> 42
cagatt                                                                  6


<210> 43
<211> 6
<212> DNA
<213> Artificial Sequence

<220>
<223> oligonucleotide 6-mer sequences

<400> 43
aagatt                                                                  6

<210> 44
<211> 6
<212> DNA
<213> Artificial Sequence

<220>
<223> oligonucleotide 6-mer sequences

<400> 44
gtcatt                                                                  6

<210> 45
<211> 6
<212> DNA
<213> Artificial Sequence

<220>
<223> oligonucleotide 6-mer sequences

<400> 45
agcatt                                                                  6

<210> 46
<211> 6
<212> DNA
<213> Artificial Sequence

<220>
<223> oligonucleotide 6-mer sequences

<400> 46
accatt                                                                  6

<210> 47

<211> 6
<212> DNA
<213> Artificial Sequence

<220>
<223> oligonucleotide 6-mer sequences

<400> 47
gcaatt                                                                  6

<210> 48
<211> 6
<212> DNA
<213> Artificial Sequence

<220>
<223> oligonucleotide 6-mer sequences

<400> 48
acaatt                                                                  6

<210> 49
<211> 6
<212> DNA
<213> Artificial Sequence

<220>
<223> oligonucleotide 6-mer sequences

<400> 49
aaaatt                                                                  6

<210> 50
<211> 6
<212> DNA
<213> Artificial Sequence

<220>
<223> oligonucleotide 6-mer sequences

<400> 50
ttttgt                                                                  6

<210> 51
<211> 6
<212> DNA
<213> Artificial Sequence

<220>
<223> oligonucleotide 6-mer sequences

<400> 51
gtttgt                                                                  6

<210> 52
<211> 6

<212> DNA
<213> Artificial Sequence

<220>
<223> oligonucleotide 6-mer sequences

<400> 52
tgttgt                                                                 6

<210> 53
<211> 6
<212> DNA
<213> Artificial Sequence

<220>
<223> oligonucleotide 6-mer sequences

<400> 53
cgttgt                                                                 6

<210> 54
<211> 6
<212> DNA
<213> Artificial Sequence

<220>
<223> oligonucleotide 6-mer sequences

<400> 54
gattgt                                                                 6

<210> 55
<211> 6
<212> DNA
<213> Artificial Sequence

<220>
<223> oligonucleotide 6-mer sequences

<400> 55
aattgt                                                                 6

<210> 56
<211> 6
<212> DNA
<213> Artificial Sequence

<220>
<223> oligonucleotide 6-mer sequences

<400> 56
ctgtgt                                                                 6

<210> 57
<211> 6
<212> DNA
<213> Artificial Sequence

<220>
<223> oligonucleotide 6-mer sequences

<400> 57
atgtgt                                                                 6

<210> 58

<211> 6
<212> DNA
<213> Artificial Sequence

<220>
<223> oligonucleotide 6-mer sequences

<400> 58
gggtgt                                                                 6

<210> 59
<211> 6
<212> DNA
<213> Artificial Sequence

<220>
<223> oligonucleotide 6-mer sequences

<400> 59
tcgtgt                                                                 6

<210> 60
<211> 6
<212> DNA
<213> Artificial Sequence

<220>
<223> oligonucleotide 6-mer sequences

<400> 60
gcgtgt                                                                 6

<210> 61
<211> 6
<212> DNA
<213> Artificial Sequence

<220>
<223> oligonucleotide 6-mer sequences

<400> 61
tagtgt                                                                 6

<210> 62
<211> 6
<212> DNA
<213> Artificial Sequence

<220>
<223> oligonucleotide 6-mer sequences

<400> 62
cgctgt                                                                 6

<210> 63
<211> 6
<212> DNA
<213> Artificial Sequence

<220>
<223> oligonucleotide 6-mer sequences

<400> 63
gcctgt                                                                 6

<210> 64
<211> 6
<212> DNA
<213> Artificial Sequence

<220>
<223> oligonucleotide 6-mer sequences

<400> 64
tactgt                                                                 6

<210> 65
<211> 6
<212> DNA
<213> Artificial Sequence

<220>
<223> oligonucleotide 6-mer sequences

<400> 65
gactgt                                                                 6

<210> 66
<211> 6
<212> DNA
<213> Artificial Sequence

<220>
<223> oligonucleotide 6-mer sequences

<400> 66
aactgt                                                                 6

<210> 67
<211> 6
<212> DNA
<213> Artificial Sequence

<220>
<223> oligonucleotide 6-mer sequences

<400> 67
gtatgt                                                                 6

<210> 68
<211> 6
<212> DNA
<213> Artificial Sequence

<220>
<223> oligonucleotide 6-mer sequences

<400> 68
ctatgt                                                                 6

<210> 69
<211> 6
<212> DNA
<213> Artificial Sequence

<220>
<223> oligonucleotide 6-mer sequences

<400> 69
atatgt                                                                 6

<210> 70
<211> 6
<212> DNA
<213> Artificial Sequence

<220>
<223> oligonucleotide 6-mer sequences

<400> 70
tgatgt                                                                  6

<210> 71
<211> 6
<212> DNA
<213> Artificial Sequence

<220>
<223> oligonucleotide 6-mer sequences

<400> 71
cgatgt                                                                  6

<210> 72
<211> 6
<212> DNA
<213> Artificial Sequence

<220>
<223> oligonucleotide 6-mer sequences

<400> 72
agatgt                                                                  6

<210> 73
<211> 6
<212> DNA
<213> Artificial Sequence

<220>
<223> oligonucleotide 6-mer sequences

<400> 73
taatgt                                                                  6

<210> 74
<211> 6
<212> DNA
<213> Artificial Sequence

<220>
<223> oligonucleotide 6-mer sequences

<400> 74
gaatgt                                                                  6

<210> 75
<211> 6
<212> DNA
<213> Artificial Sequence

<220>
<223> oligonucleotide 6-mer sequences

<400> 75

caatgt                                                                  6

<210> 76
<211> 6
<212> DNA
<213> Artificial Sequence

<220>
<223> oligonucleotide 6-mer sequences

<400> 76
attggt                                                                  6

<210> 77
<211> 6
<212> DNA
<213> Artificial Sequence

<220>
<223> oligonucleotide 6-mer sequences

<400> 77
gctggt                                                                  6

<210> 78
<211> 6
<212> DNA
<213> Artificial Sequence

<220>
<223> oligonucleotide 6-mer sequences

<400> 78
ttgggt                                                                  6

<210> 79
<211> 6
<212> DNA
<213> Artificial Sequence

<220>
<223> oligonucleotide 6-mer sequences


<400> 79
gagggt                                                                  6

<210> 80
<211> 6
<212> DNA
<213> Artificial Sequence

<220>
<223> oligonucleotide 6-mer sequences

<400> 80
aagggt                                                                  6

<210> 81
<211> 6
<212> DNA
<213> Artificial Sequence

<220>
<223> oligonucleotide 6-mer sequences

<400> 81
gtcggt                                                                 6

<210> 82
<211> 6
<212> DNA
<213> Artificial Sequence

<220>
<223> oligonucleotide 6-mer sequences

<400> 82
ctcggt                                                                 6

<210> 83
<211> 6
<212> DNA
<213> Artificial Sequence

<220>
<223> oligonucleotide 6-mer sequences

<400> 83

tccggt                                                                 6

<210> 84
<211> 6
<212> DNA
<213> Artificial Sequence

<220>
<223> oligonucleotide 6-mer sequences

<400> 84
accggt                                                                 6

<210> 85
<211> 6
<212> DNA
<213> Artificial Sequence

<220>
<223> oligonucleotide 6-mer sequences

<400> 85
ttaggt                                                                 6

<210> 86
<211> 6
<212> DNA
<213> Artificial Sequence

<220>
<223> oligonucleotide 6-mer sequences

<400> 86
ctaggt                                                                 6

<210> 87
<211> 6
<212> DNA
<213> Artificial Sequence

<220>
<223> oligonucleotide 6-mer sequences

<400> 87
agaggt                                                                  6

<210> 88
<211> 6
<212> DNA
<213> Artificial Sequence

<220>
<223> oligonucleotide 6-mer sequences

<400> 88
taaggt                                                                  6

<210> 89
<211> 6
<212> DNA
<213> Artificial Sequence

<220>
<223> oligonucleotide 6-mer sequences

<400> 89
gaaggt                                                                  6

<210> 90
<211> 6
<212> DNA
<213> Artificial Sequence

<220>
<223> oligonucleotide 6-mer sequences

<400> 90
caaggt                                                                  6

<210> 91
<211> 6
<212> DNA
<213> Artificial Sequence

<220>
<223> oligonucleotide 6-mer sequences

<400> 91
cttcgt                                                                  6

<210> 92
<211> 6
<212> DNA
<213> Artificial Sequence

<220>
<223> oligonucleotide 6-mer sequences

<400> 92
cgtcgt                                                                  6

<210> 93
<211> 6
<212> DNA
<213> Artificial Sequence

<220>
<223> oligonucleotide 6-mer sequences

<400> 93
agtcgt                                                                  6

<210> 94
<211> 6
<212> DNA
<213> Artificial Sequence

<220>
<223> oligonucleotide 6-mer sequences

<400> 94
aatcgt                                                                  6

<210> 95
<211> 6
<212> DNA
<213> Artificial Sequence

<220>
<223> oligonucleotide 6-mer sequences

<400> 95
ttgcgt                                                                  6

<210> 96
<211> 6
<212> DNA
<213> Artificial Sequence

<220>
<223> oligonucleotide 6-mer sequences

<400> 96
atgcgt                                                                  6

<210> 97
<211> 6
<212> DNA
<213> Artificial Sequence

<220>
<223> oligonucleotide 6-mer sequences

<400> 97
ccgcgt                                                                  6

<210> 98
<211> 6
<212> DNA
<213> Artificial Sequence

<220>
<223> oligonucleotide 6-mer sequences

<400> 98
acgcgt                                                                  6

<210> 99
<211> 6
<212> DNA

<213> Artificial Sequence

<220>
<223> oligonucleotide 6-mer sequences

<400> 99
aagcgt                                                                  6

<210> 100
<211> 6
<212> DNA
<213> Artificial Sequence

<220>
<223> oligonucleotide 6-mer sequences

<400> 100
ctccgt                                                                  6

<210> 101
<211> 6
<212> DNA
<213> Artificial Sequence

<220>
<223> oligonucleotide 6-mer sequences

<400> 101
atccgt                                                                  6

<210> 102
<211> 6
<212> DNA
<213> Artificial Sequence

<220>
<223> oligonucleotide 6-mer sequences


<400> 102
aaccgt                                                                  6

<210> 103
<211> 6
<212> DNA
<213> Artificial Sequence

<220>
<223> oligonucleotide 6-mer sequences

<400> 103
ttacgt                                                                  6

<210> 104
<211> 6
<212> DNA
<213> Artificial Sequence

<220>
<223> oligonucleotide 6-mer sequences

<400> 104
gtacgt                                                                  6

<210> 105

<211> 6
<212> DNA
<213> Artificial Sequence

<220>
<223> oligonucleotide 6-mer sequences

<400> 105
atacgt                                                                  6

<210> 106
<211> 6
<212> DNA
<213> Artificial Sequence

<220>
<223> oligonucleotide 6-mer sequences

<400> 106
ggacgt                                                                  6

<210> 107
<211> 6
<212> DNA
<213> Artificial Sequence

<220>
<223> oligonucleotide 6-mer sequences

<400> 107
taacgt                                                                  6

<210> 108
<211> 6
<212> DNA
<213> Artificial Sequence

<220>
<223> oligonucleotide 6-mer sequences

<400> 108
caacgt                                                                  6

<210> 109
<211> 6
<212> DNA
<213> Artificial Sequence

<220>
<223> oligonucleotide 6-mer sequences

<400> 109
aaacgt                                                                  6

<210> 110
<211> 6
<212> DNA
<213> Artificial Sequence

<220>
<223> oligonucleotide 6-mer sequences

<400> 110
tgtagt                                                                  6

<210> 111
<211> 6
<212> DNA
<213> Artificial Sequence

<220>
<223> oligonucleotide 6-mer sequences

<400> 111
cgtagt                                                                 6

<210> 112
<211> 6
<212> DNA
<213> Artificial Sequence

<220>
<223> oligonucleotide 6-mer sequences

<400> 112
tctagt                                                                 6

<210> 113
<211> 6
<212> DNA
<213> Artificial Sequence

<220>
<223> oligonucleotide 6-mer sequences

<400> 113
cctagt                                                                 6

<210> 114
<211> 6
<212> DNA
<213> Artificial Sequence

<220>
<223> oligonucleotide 6-mer sequences

<400> 114
tatagt                                                                 6

<210> 115
<211> 6
<212> DNA
<213> Artificial Sequence

<220>
<223> oligonucleotide 6-mer sequences

<400> 115
aatagt                                                                 6

<210> 116
<211> 6
<212> DNA
<213> Artificial Sequence

<220>
<223> oligonucleotide 6-mer sequences

<400> 116
atgagt                                                                 6

<210> 117
<211> 6
<212> DNA
<213> Artificial Sequence

<220>
<223> oligonucleotide 6-mer sequences

<400> 117
cggagt                                                                 6

<210> 118
<211> 6
<212> DNA
<213> Artificial Sequence

<220>
<223> oligonucleotide 6-mer sequences

<400> 118
tcgagt                                                                 6

<210> 119
<211> 6
<212> DNA
<213> Artificial Sequence

<220>
<223> oligonucleotide 6-mer sequences

<400> 119
ccgagt                                                                 6

<210> 120
<211> 6
<212> DNA
<213> Artificial Sequence

<220>
<223> oligonucleotide 6-mer sequences

<400> 120
acgagt                                                                 6

<210> 121
<211> 6
<212> DNA
<213> Artificial Sequence

<220>
<223> oligonucleotide 6-mer sequences

<400> 121
agcagt                                                                 6

<210> 122
<211> 6
<212> DNA
<213> Artificial Sequence

<220>
<223> oligonucleotide 6-mer sequences

<400> 122
tacagt                                                                  6

<210> 123
<211> 6
<212> DNA
<213> Artificial Sequence

<220>
<223> oligonucleotide 6-mer sequences

<400> 123
gtaagt                                                                  6

<210> 124
<211> 6
<212> DNA
<213> Artificial Sequence

<220>
<223> oligonucleotide 6-mer sequences

<400> 124
ataagt                                                                  6

<210> 125
<211> 6
<212> DNA
<213> Artificial Sequence

<220>
<223> oligonucleotide 6-mer sequences

<400> 125
gcaagt                                                                  6

<210> 126
<211> 6
<212> DNA
<213> Artificial Sequence

<220>
<223> oligonucleotide 6-mer sequences

<400> 126
acaagt                                                                  6

<210> 127
<211> 6
<212> DNA
<213> Artificial Sequence

<220>
<223> oligonucleotide 6-mer sequences

<400> 127
tgttct                                                                  6

<210> 128
<211> 6
<212> DNA
<213> Artificial Sequence

<220>
<223> oligonucleotide 6-mer sequences

<400> 128
ttgtct                                                                  6

<210> 129
<211> 6
<212> DNA
<213> Artificial Sequence

<220>
<223> oligonucleotide 6-mer sequences

<400> 129
aggtct                                                                  6

<210> 130
<211> 6
<212> DNA
<213> Artificial Sequence

<220>
<223> oligonucleotide 6-mer sequences

<400> 130
cagtct                                                                  6

<210> 131
<211> 6
<212> DNA
<213> Artificial Sequence

<220>
<223> oligonucleotide 6-mer sequences

<400> 131
aagtct                                                                  6

<210> 132
<211> 6
<212> DNA
<213> Artificial Sequence

<220>
<223> oligonucleotide 6-mer sequences

<400> 132
gcctct                                                                  6

<210> 133
<211> 6
<212> DNA
<213> Artificial Sequence

<220>
<223> oligonucleotide 6-mer sequences

<400> 133
tactct                                                                  6

<210> 134
<211> 6
<212> DNA
<213> Artificial Sequence

<220>

<223> oligonucleotide 6-mer sequences

<400> 134
gactct                                                                 6

<210> 135
<211> 6
<212> DNA
<213> Artificial Sequence

<220>
<223> oligonucleotide 6-mer sequences

<400> 135
ctatct                                                                 6

<210> 136
<211> 6
<212> DNA
<213> Artificial Sequence

<220>
<223> oligonucleotide 6-mer sequences

<400> 136
atatct                                                                 6

<210> 137
<211> 6
<212> DNA
<213> Artificial Sequence

<220>
<223> oligonucleotide 6-mer sequences

<400> 137
cgatct                                                                 6

<210> 138
<211> 6
<212> DNA
<213> Artificial Sequence

<220>
<223> oligonucleotide 6-mer sequences

<400> 138
tcatct                                                                 6

<210> 139
<211> 6
<212> DNA
<213> Artificial Sequence

<220>
<223> oligonucleotide 6-mer sequences

<400> 139
aaatct                                                                 6

<210> 140
<211> 6
<212> DNA
<213> Artificial Sequence

<220>
<223> oligonucleotide 6-mer sequences

<400> 140
cttgct                                                                 6

<210> 141
<211> 6
<212> DNA
<213> Artificial Sequence

<220>
<223> oligonucleotide 6-mer sequences

<400> 141
tgtgct                                                                 6

<210> 142
<211> 6
<212> DNA
<213> Artificial Sequence

<220>
<223> oligonucleotide 6-mer sequences

<400> 142
catgct                                                                 6

<210> 143
<211> 6
<212> DNA
<213> Artificial Sequence

<220>
<223> oligonucleotide 6-mer sequences

<400> 143
atagct                                                                 6

<210> 144
<211> 6
<212> DNA
<213> Artificial Sequence

<220>
<223> oligonucleotide 6-mer sequences

<400> 144
acagct                                                                 6

<210> 145
<211> 6
<212> DNA
<213> Artificial Sequence

<220>
<223> oligonucleotide 6-mer sequences

<400> 145
taagct                                                                 6

<210> 146
<211> 6
<212> DNA
<213> Artificial Sequence

<220>
<223> oligonucleotide 6-mer sequences

<400> 146
ttgcct                                                                 6

<210> 147
<211> 6
<212> DNA
<213> Artificial Sequence

<220>
<223> oligonucleotide 6-mer sequences

<400> 147
gtgcct                                                                 6

<210> 148
<211> 6
<212> DNA
<213> Artificial Sequence

<220>
<223> oligonucleotide 6-mer sequences

<400> 148
atgcct                                                                 6

<210> 149
<211> 6
<212> DNA
<213> Artificial Sequence

<220>
<223> oligonucleotide 6-mer sequences

<400> 149
acccct                                                                 6

<210> 150
<211> 6
<212> DNA
<213> Artificial Sequence

<220>
<223> oligonucleotide 6-mer sequences

<400> 150
gtacct                                                                 6

<210> 151
<211> 6
<212> DNA
<213> Artificial Sequence

<220>
<223> oligonucleotide 6-mer sequences

<400> 151
atacct                                                                 6

<210> 152
<211> 6
<212> DNA

<213> Artificial Sequence

<220>
<223> oligonucleotide 6-mer sequences

<400> 152
tgacct                                                                 6

<210> 153
<211> 6
<212> DNA
<213> Artificial Sequence

<220>
<223> oligonucleotide 6-mer sequences

<400> 153
caacct                                                                 6

<210> 154
<211> 6
<212> DNA
<213> Artificial Sequence

<220>
<223> oligonucleotide 6-mer sequences

<400> 154
attact                                                                 6

<210> 155
<211> 6
<212> DNA
<213> Artificial Sequence

<220>
<223> oligonucleotide 6-mer sequences

<400> 155
tgtact                                                                 6

<210> 156
<211> 6
<212> DNA
<213> Artificial Sequence

<220>
<223> oligonucleotide 6-mer sequences

<400> 156
agtact                                                                 6

<210> 157
<211> 6
<212> DNA
<213> Artificial Sequence

<220>
<223> oligonucleotide 6-mer sequences

<400> 157
tctact                                                                 6

<210> 158
<211> 6

<212> DNA
<213> Artificial Sequence

<220>
<223> oligonucleotide 6-mer sequences

<400> 158
actact                                                                   6

<210> 159
<211> 6
<212> DNA
<213> Artificial Sequence

<220>
<223> oligonucleotide 6-mer sequences

<400> 159
tatact                                                                   6

<210> 160
<211> 6
<212> DNA
<213> Artificial Sequence

<220>
<223> oligonucleotide 6-mer sequences

<400> 160
aatact                                                                   6

<210> 161
<211> 6
<212> DNA
<213> Artificial Sequence

<220>
<223> oligonucleotide 6-mer sequences

<400> 161
gtgact                                                                   6

<210> 162
<211> 6
<212> DNA
<213> Artificial Sequence

<220>
<223> oligonucleotide 6-mer sequences

<400> 162
atgact                                                                   6

<210> 163
<211> 6
<212> DNA
<213> Artificial Sequence

<220>
<223> oligonucleotide 6-mer sequences

<400> 163
gagact                                                                   6

<210> 164

<211> 6
<212> DNA
<213> Artificial Sequence

<220>
<223> oligonucleotide 6-mer sequences

<400> 164
tacact                                                                 6

<210> 165
<211> 6
<212> DNA
<213> Artificial Sequence

<220>
<223> oligonucleotide 6-mer sequences

<400> 165
ctaact                                                                 6

<210> 166
<211> 6
<212> DNA
<213> Artificial Sequence

<220>
<223> oligonucleotide 6-mer sequences

<400> 166
cgaact                                                                 6

<210> 167
<211> 6
<212> DNA
<213> Artificial Sequence

<220>
<223> oligonucleotide 6-mer sequences

<400> 167
acaact                                                                 6

<210> 168
<211> 6
<212> DNA
<213> Artificial Sequence

<220>
<223> oligonucleotide 6-mer sequences

<400> 168
taaact                                                                 6

<210> 169
<211> 6
<212> DNA
<213> Artificial Sequence

<220>
<223> oligonucleotide 6-mer sequences

<400> 169
atttat                                                                 6

<210> 170
<211> 6
<212> DNA
<213> Artificial Sequence

<220>
<223> oligonucleotide 6-mer sequences

<400> 170
tattat                                                                 6

<210> 171
<211> 6
<212> DNA
<213> Artificial Sequence

<220>
<223> oligonucleotide 6-mer sequences

<400> 171
gattat                                                                 6

<210> 172
<211> 6
<212> DNA
<213> Artificial Sequence

<220>
<223> oligonucleotide 6-mer sequences

<400> 172
aattat                                                                 6

<210> 173
<211> 6
<212> DNA
<213> Artificial Sequence

<220>
<223> oligonucleotide 6-mer sequences

<400> 173
gtgtat                                                                 6

<210> 174
<211> 6
<212> DNA
<213> Artificial Sequence

<220>
<223> oligonucleotide 6-mer sequences

<400> 174
aggtat                                                                 6

<210> 175
<211> 6
<212> DNA
<213> Artificial Sequence

<220>
<223> oligonucleotide 6-mer sequences

<400> 175
tcgtat                                                                 6

<210> 176
<211> 6
<212> DNA
<213> Artificial Sequence

<220>
<223> oligonucleotide 6-mer sequences

<400> 176
gcgtat                                                                 6

<210> 177
<211> 6
<212> DNA
<213> Artificial Sequence

<220>
<223> oligonucleotide 6-mer sequences

<400> 177
acgtat                                                                 6

<210> 178
<211> 6
<212> DNA
<213> Artificial Sequence

<220>
<223> oligonucleotide 6-mer sequences

<400> 178
tagtat                                                                 6

<210> 179
<211> 6
<212> DNA
<213> Artificial Sequence

<220>
<223> oligonucleotide 6-mer sequences

<400> 179
gagtat                                                                 6

<210> 180
<211> 6
<212> DNA
<213> Artificial Sequence

<220>
<223> oligonucleotide 6-mer sequences

<400> 180
cagtat                                                                 6

<210> 181
<211> 6
<212> DNA
<213> Artificial Sequence

<220>
<223> oligonucleotide 6-mer sequences

<400> 181

aagtat                                                                 6

<210> 182
<211> 6
<212> DNA
<213> Artificial Sequence

<220>
<223> oligonucleotide 6-mer sequences

<400> 182
ttctat                                                                 6

<210> 183
<211> 6
<212> DNA
<213> Artificial Sequence

<220>
<223> oligonucleotide 6-mer sequences

<400> 183
ctctat                                                                 6

<210> 184
<211> 6
<212> DNA
<213> Artificial Sequence

<220>
<223> oligonucleotide 6-mer sequences

<400> 184
atctat                                                                 6

<210> 185
<211> 6
<212> DNA
<213> Artificial Sequence

<220>
<223> oligonucleotide 6-mer sequences

<400> 185
tgctat                                                                 6

<210> 186
<211> 6
<212> DNA
<213> Artificial Sequence

<220>
<223> oligonucleotide 6-mer sequences

<400> 186
acctat                                                                 6


<210> 187
<211> 6
<212> DNA
<213> Artificial Sequence

<220>
<223> oligonucleotide 6-mer sequences

<400> 187
tactat                                                                  6

<210> 188
<211> 6
<212> DNA
<213> Artificial Sequence

<220>
<223> oligonucleotide 6-mer sequences

<400> 188
cactat                                                                  6

<210> 189
<211> 6
<212> DNA
<213> Artificial Sequence

<220>
<223> oligonucleotide 6-mer sequences

<400> 189
aactat                                                                  6

<210> 190
<211> 6
<212> DNA
<213> Artificial Sequence

<220>
<223> oligonucleotide 6-mer sequences

<400> 190
ttatat                                                                  6

<210> 191
<211> 6
<212> DNA
<213> Artificial Sequence

<220>
<223> oligonucleotide 6-mer sequences

<400> 191
gtatat                                                                  6

<210> 192
<211> 6
<212> DNA
<213> Artificial Sequence

<220>
<223> oligonucleotide 6-mer sequences

<400> 192
atatat                                                                  6

<210> 193
<211> 6
<212> DNA
<213> Artificial Sequence

<220>

<223> oligonucleotide 6-mer sequences

<400> 193
tgatat                                                                  6

<210> 194
<211> 6
<212> DNA
<213> Artificial Sequence

<220>
<223> oligonucleotide 6-mer sequences

<400> 194
cgatat                                                                  6

<210> 195
<211> 6
<212> DNA
<213> Artificial Sequence

<220>
<223> oligonucleotide 6-mer sequences

<400> 195
agatat                                                                  6

<210> 196
<211> 6
<212> DNA
<213> Artificial Sequence

<220>
<223> oligonucleotide 6-mer sequences

<400> 196
tcatat                                                                  6

<210> 197
<211> 6
<212> DNA
<213> Artificial Sequence

<220>
<223> oligonucleotide 6-mer sequences

<400> 197
ccatat                                                                  6

<210> 198
<211> 6
<212> DNA
<213> Artificial Sequence

<220>
<223> oligonucleotide 6-mer sequences

<400> 198
acatat                                                                  6

<210> 199
<211> 6
<212> DNA
<213> Artificial Sequence

<220>
<223> oligonucleotide 6-mer sequences

<400> 199
caatat                                                                  6

<210> 200
<211> 6
<212> DNA
<213> Artificial Sequence

<220>
<223> oligonucleotide 6-mer sequences

<400> 200
aaatat                                                                  6

<210> 201
<211> 6
<212> DNA
<213> Artificial Sequence

<220>
<223> oligonucleotide 6-mer sequences

<400> 201
ggtgat                                                                  6

<210> 202
<211> 6
<212> DNA
<213> Artificial Sequence

<220>
<223> oligonucleotide 6-mer sequences

<400> 202
cgtgat                                                                  6

<210> 203
<211> 6
<212> DNA
<213> Artificial Sequence

<220>
<223> oligonucleotide 6-mer sequences

<400> 203
tatgat                                                                  6

<210> 204
<211> 6
<212> DNA
<213> Artificial Sequence

<220>
<223> oligonucleotide 6-mer sequences

<400> 204
gatgat                                                                  6

<210> 205
<211> 6
<212> DNA
<213> Artificial Sequence

<220>
<223> oligonucleotide 6-mer sequences

<400> 205
gtggat                                                                  6

<210> 206
<211> 6
<212> DNA
<213> Artificial Sequence

<220>
<223> oligonucleotide 6-mer sequences

<400> 206
tgggat                                                                  6

<210> 207
<211> 6

<212> DNA
<213> Artificial Sequence

<220>
<223> oligonucleotide 6-mer sequences

<400> 207
agggat                                                                  6

<210> 208
<211> 6
<212> DNA
<213> Artificial Sequence

<220>
<223> oligonucleotide 6-mer sequences

<400> 208
taggat                                                                  6

<210> 209
<211> 6
<212> DNA
<213> Artificial Sequence

<220>
<223> oligonucleotide 6-mer sequences

<400> 209
gaggat                                                                  6

<210> 210
<211> 6
<212> DNA
<213> Artificial Sequence

<220>
<223> oligonucleotide 6-mer sequences

<400> 210
gtcgat                                                                  6

<210> 211
<211> 6

<212> DNA
<213> Artificial Sequence

<220>
<223> oligonucleotide 6-mer sequences

<400> 211
atcgat                                                                 6

<210> 212
<211> 6
<212> DNA
<213> Artificial Sequence

<220>
<223> oligonucleotide 6-mer sequences

<400> 212
agcgat                                                                 6

<210> 213
<211> 6
<212> DNA
<213> Artificial Sequence

<220>
<223> oligonucleotide 6-mer sequences

<400> 213
tccgat                                                                 6

<210> 214
<211> 6
<212> DNA
<213> Artificial Sequence

<220>
<223> oligonucleotide 6-mer sequences

<400> 214
tacgat                                                                 6

<210> 215
<211> 6
<212> DNA
<213> Artificial Sequence

<220>
<223> oligonucleotide 6-mer sequences

<400> 215
gacgat                                                                 6

<210> 216
<211> 6
<212> DNA
<213> Artificial Sequence

<220>
<223> oligonucleotide 6-mer sequences

<400> 216
cacgat                                                                 6

<210> 217

<211> 6
<212> DNA
<213> Artificial Sequence

<220>
<223> oligonucleotide 6-mer sequences

<400> 217
gtagat                                                                  6

<210> 218
<211> 6
<212> DNA
<213> Artificial Sequence

<220>
<223> oligonucleotide 6-mer sequences

<400> 218
ggagat                                                                  6

<210> 219
<211> 6
<212> DNA
<213> Artificial Sequence

<220>
<223> oligonucleotide 6-mer sequences

<400> 219
agagat                                                                  6

<210> 220
<211> 6
<212> DNA
<213> Artificial Sequence

<220>
<223> oligonucleotide 6-mer sequences

<400> 220
gcagat                                                                  6

<210> 221
<211> 6
<212> DNA
<213> Artificial Sequence

<220>
<223> oligonucleotide 6-mer sequences

<400> 221
taagat                                                                  6

<210> 222
<211> 6
<212> DNA
<213> Artificial Sequence

<220>
<223> oligonucleotide 6-mer sequences

<400> 222
gaagat                                                                  6

<210> 223
<211> 6
<212> DNA
<213> Artificial Sequence

<220>
<223> oligonucleotide 6-mer sequences

<400> 223
tttcat                                                                 6

<210> 224
<211> 6
<212> DNA
<213> Artificial Sequence

<220>
<223> oligonucleotide 6-mer sequences

<400> 224
tgtcat                                                                 6

<210> 225
<211> 6
<212> DNA
<213> Artificial Sequence

<220>
<223> oligonucleotide 6-mer sequences

<400> 225
cgtcat                                                                 6

<210> 226
<211> 6
<212> DNA
<213> Artificial Sequence

<220>
<223> oligonucleotide 6-mer sequences

<400> 226
gctcat                                                                 6

<210> 227
<211> 6
<212> DNA
<213> Artificial Sequence

<220>
<223> oligonucleotide 6-mer sequences

<400> 227
cctcat                                                                 6

<210> 228
<211> 6
<212> DNA
<213> Artificial Sequence

<220>
<223> oligonucleotide 6-mer sequences

<400> 228
tatcat                                                                 6

<210> 229
<211> 6
<212> DNA
<213> Artificial Sequence

<220>
<223> oligonucleotide 6-mer sequences

<400> 229
ttgcat                                                                  6

<210> 230
<211> 6
<212> DNA
<213> Artificial Sequence

<220>
<223> oligonucleotide 6-mer sequences

<400> 230
gtgcat                                                                  6

<210> 231
<211> 6
<212> DNA
<213> Artificial Sequence

<220>
<223> oligonucleotide 6-mer sequences

<400> 231
gcgcat                                                                  6

<210> 232
<211> 6
<212> DNA
<213> Artificial Sequence

<220>
<223> oligonucleotide 6-mer sequences


<400> 232
ctccat                                                                  6

<210> 233
<211> 6
<212> DNA
<213> Artificial Sequence

<220>
<223> oligonucleotide 6-mer sequences

<400> 233
tcccat                                                                  6

<210> 234
<211> 6
<212> DNA
<213> Artificial Sequence

<220>
<223> oligonucleotide 6-mer sequences

<400> 234
gaccat                                                                  6

<210> 235
<211> 6
<212> DNA
<213> Artificial Sequence

<220>
<223> oligonucleotide 6-mer sequences

<400> 235
ttacat                                                                  6

<210> 236
<211> 6
<212> DNA
<213> Artificial Sequence

<220>
<223> oligonucleotide 6-mer sequences

<400> 236
gtacat                                                                  6

<210> 237
<211> 6
<212> DNA
<213> Artificial Sequence

<220>
<223> oligonucleotide 6-mer sequences

<400> 237
atacat                                                                  6

<210> 238
<211> 6
<212> DNA
<213> Artificial Sequence

<220>
<223> oligonucleotide 6-mer sequences

<400> 238
taacat                                                                  6

<210> 239
<211> 6
<212> DNA
<213> Artificial Sequence

<220>
<223> oligonucleotide 6-mer sequences

<400> 239
gttaat                                                                  6

<210> 240
<211> 6
<212> DNA
<213> Artificial Sequence

<220>
<223> oligonucleotide 6-mer sequences

<400> 240
tgtaat                                                                 6

<210> 241
<211> 6
<212> DNA
<213> Artificial Sequence

<220>
<223> oligonucleotide 6-mer sequences

<400> 241
cgtaat                                                                 6

<210> 242
<211> 6
<212> DNA
<213> Artificial Sequence

<220>
<223> oligonucleotide 6-mer sequences

<400> 242
gctaat                                                                 6

<210> 243
<211> 6
<212> DNA
<213> Artificial Sequence

<220>
<223> oligonucleotide 6-mer sequences

<400> 243
cctaat                                                                 6

<210> 244
<211> 6
<212> DNA
<213> Artificial Sequence

<220>
<223> oligonucleotide 6-mer sequences

<400> 244
tataat                                                                 6

<210> 245
<211> 6
<212> DNA
<213> Artificial Sequence

<220>
<223> oligonucleotide 6-mer sequences

<400> 245
gataat                                                                 6

<210> 246
<211> 6
<212> DNA
<213> Artificial Sequence

<220>

<223> oligonucleotide 6-mer sequences

<400> 246
atgaat                                                                  6

<210> 247
<211> 6
<212> DNA
<213> Artificial Sequence


<220>
<223> oligonucleotide 6-mer sequences

<400> 247
aggaat                                                                  6

<210> 248
<211> 6
<212> DNA
<213> Artificial Sequence

<220>
<223> oligonucleotide 6-mer sequences

<400> 248
ccgaat                                                                  6

<210> 249
<211> 6
<212> DNA
<213> Artificial Sequence

<220>
<223> oligonucleotide 6-mer sequences

<400> 249
ggcaat                                                                  6

<210> 250
<211> 6
<212> DNA
<213> Artificial Sequence

<220>
<223> oligonucleotide 6-mer sequences

<400> 250
agcaat                                                                  6

<210> 251
<211> 6
<212> DNA
<213> Artificial Sequence

<220>
<223> oligonucleotide 6-mer sequences

<400> 251
cccaat                                                                  6

<210> 252
<211> 6
<212> DNA
<213> Artificial Sequence

<220>
<223> oligonucleotide 6-mer sequences

<400> 252
accaat                                                                  6

<210> 253
<211> 6
<212> DNA
<213> Artificial Sequence

<220>
<223> oligonucleotide 6-mer sequences

<400> 253
tacaat                                                                  6

<210> 254
<211> 6
<212> DNA
<213> Artificial Sequence

<220>
<223> oligonucleotide 6-mer sequences

<400> 254
gacaat                                                                  6

<210> 255
<211> 6
<212> DNA
<213> Artificial Sequence

<220>

<223> oligonucleotide 6-mer sequences

<400> 255
gtaaat                                                                  6

<210> 256
<211> 6
<212> DNA
<213> Artificial Sequence

<220>
<223> oligonucleotide 6-mer sequences

<400> 256
ctaaat                                                                  6

<210> 257
<211> 6
<212> DNA
<213> Artificial Sequence

<220>
<223> oligonucleotide 6-mer sequences

<400> 257
taaaat                                                                  6

<210> 258
<211> 6

<212> DNA
<213> Artificial Sequence

<220>
<223> oligonucleotide 6-mer sequences

<400> 258
aaaaat                                                                  6

<210> 259
<211> 6
<212> DNA
<213> Artificial Sequence

<220>
<223> oligonucleotide 6-mer sequences

<400> 259
tttttg                                                                  6

<210> 260
<211> 6
<212> DNA
<213> Artificial Sequence

<220>
<223> oligonucleotide 6-mer sequences

<400> 260
attttg                                                                  6

<210> 261
<211> 6
<212> DNA
<213> Artificial Sequence

<220>
<223> oligonucleotide 6-mer sequences

<400> 261
ggtttg                                                                  6

<210> 262

<211> 6
<212> DNA
<213> Artificial Sequence

<220>
<223> oligonucleotide 6-mer sequences

<400> 262
cctttg                                                                  6

<210> 263
<211> 6
<212> DNA
<213> Artificial Sequence

<220>
<223> oligonucleotide 6-mer sequences

<400> 263
ctgttg                                                                  6

<210> 264
<211> 6
<212> DNA
<213> Artificial Sequence

<220>
<223> oligonucleotide 6-mer sequences

<400> 264
atgttg                                                                  6

<210> 265
<211> 6
<212> DNA
<213> Artificial Sequence

<220>
<223> oligonucleotide 6-mer sequences

<400> 265
tggttg                                                                  6

<210> 266
<211> 6
<212> DNA
<213> Artificial Sequence

<220>
<223> oligonucleotide 6-mer sequences

<400> 266
cggttg                                                                  6

<210> 267
<211> 6
<212> DNA
<213> Artificial Sequence

<220>
<223> oligonucleotide 6-mer sequences

<400> 267
tcgttg                                                                  6

<210> 268
<211> 6
<212> DNA
<213> Artificial Sequence

<220>
<223> oligonucleotide 6-mer sequences

<400> 268
atcttg                                                                  6

<210> 269
<211> 6
<212> DNA
<213> Artificial Sequence

<220>
<223> oligonucleotide 6-mer sequences

<400> 269
atattg                                                                  6

<210> 270
<211> 6
<212> DNA
<213> Artificial Sequence

<220>
<223> oligonucleotide 6-mer sequences

<400> 270
ggattg                                                                 6

<210> 271
<211> 6
<212> DNA
<213> Artificial Sequence

<220>
<223> oligonucleotide 6-mer sequences

<400> 271
cgattg                                                                 6

<210> 272
<211> 6
<212> DNA
<213> Artificial Sequence

<220>
<223> oligonucleotide 6-mer sequences

<400> 272
agattg                                                                 6

<210> 273
<211> 6
<212> DNA
<213> Artificial Sequence

<220>
<223> oligonucleotide 6-mer sequences

<400> 273
gaattg                                                                 6

<210> 274
<211> 6
<212> DNA
<213> Artificial Sequence

<220>
<223> oligonucleotide 6-mer sequences

<400> 274
caattg                                                                 6

<210> 275
<211> 6
<212> DNA
<213> Artificial Sequence

<220>
<223> oligonucleotide 6-mer sequences

<400> 275

tgtgtg                                                                  6

<210> 276
<211> 6
<212> DNA
<213> Artificial Sequence

<220>
<223> oligonucleotide 6-mer sequences

<400> 276
cgtgtg                                                                  6

<210> 277
<211> 6
<212> DNA
<213> Artificial Sequence

<220>
<223> oligonucleotide 6-mer sequences

<400> 277
actgtg                                                                  6

<210> 278
<211> 6
<212> DNA
<213> Artificial Sequence

<220>
<223> oligonucleotide 6-mer sequences

<400> 278
tatgtg                                                                  6

<210> 279
<211> 6
<212> DNA
<213> Artificial Sequence

<220>
<223> oligonucleotide 6-mer sequences

<400> 279
aatgtg                                                                  6

<210> 280
<211> 6
<212> DNA
<213> Artificial Sequence

<220>
<223> oligonucleotide 6-mer sequences

<400> 280
agggtg                                                                  6

<210> 281
<211> 6
<212> DNA
<213> Artificial Sequence

<220>
<223> oligonucleotide 6-mer sequences

<400> 281
tcggtg                                                                  6

<210> 282
<211> 6
<212> DNA

<213> Artificial Sequence

<220>
<223> oligonucleotide 6-mer sequences

<400> 282
taggtg                                                                  6

<210> 283
<211> 6
<212> DNA
<213> Artificial Sequence

<220>
<223> oligonucleotide 6-mer sequences

<400> 283
gtcgtg                                                                  6

<210> 284
<211> 6
<212> DNA
<213> Artificial Sequence

<220>
<223> oligonucleotide 6-mer sequences

<400> 284
tacgtg                                                                  6

<210> 285
<211> 6
<212> DNA
<213> Artificial Sequence

<220>
<223> oligonucleotide 6-mer sequences

<400> 285
atagtg                                                                  6

<210> 286
<211> 6
<212> DNA
<213> Artificial Sequence

<220>
<223> oligonucleotide 6-mer sequences

<400> 286
tgagtg                                                                  6

<210> 287
<211> 6
<212> DNA
<213> Artificial Sequence

<220>

<223> oligonucleotide 6-mer sequences

<400> 287
agagtg                                                                 6

<210> 288
<211> 6
<212> DNA
<213> Artificial Sequence

<220>
<223> oligonucleotide 6-mer sequences

<400> 288
ccagtg                                                                 6

<210> 289
<211> 6
<212> DNA
<213> Artificial Sequence

<220>
<223> oligonucleotide 6-mer sequences

<400> 289
taagtg                                                                 6

<210> 290
<211> 6
<212> DNA
<213> Artificial Sequence

<220>
<223> oligonucleotide 6-mer sequences

<400> 290
caagtg                                                                 6

<210> 291
<211> 6
<212> DNA
<213> Artificial Sequence

<220>
<223> oligonucleotide 6-mer sequences

<400> 291
actctg                                                                 6

<210> 292
<211> 6
<212> DNA
<213> Artificial Sequence

<220>
<223> oligonucleotide 6-mer sequences

<400> 292
catctg                                                                 6

<210> 293
<211> 6
<212> DNA
<213> Artificial Sequence

<220>
<223> oligonucleotide 6-mer sequences

<400> 293
ttgctg                                                                 6

<210> 294
<211> 6
<212> DNA
<213> Artificial Sequence

<220>
<223> oligonucleotide 6-mer sequences

<400> 294
atgctg                                                                 6

<210> 295
<211> 6
<212> DNA
<213> Artificial Sequence

<220>
<223> oligonucleotide 6-mer sequences

<400> 295
ttcctg                                                                 6

<210> 296
<211> 6
<212> DNA
<213> Artificial Sequence

<220>
<223> oligonucleotide 6-mer sequences

<400> 296
tgcctg                                                                 6

<210> 297
<211> 6
<212> DNA
<213> Artificial Sequence

<220>
<223> oligonucleotide 6-mer sequences

<400> 297
tacctg                                                                 6

<210> 298
<211> 6
<212> DNA
<213> Artificial Sequence

<220>
<223> oligonucleotide 6-mer sequences

<400> 298
atactg                                                                 6

<210> 299
<211> 6
<212> DNA
<213> Artificial Sequence

<220>
<223> oligonucleotide 6-mer sequences

<400> 299
tgactg                                                                  6

<210> 300
<211> 6
<212> DNA
<213> Artificial Sequence

<220>
<223> oligonucleotide 6-mer sequences

<400> 300
agactg                                                                  6

<210> 301
<211> 6
<212> DNA
<213> Artificial Sequence

<220>
<223> oligonucleotide 6-mer sequences

<400> 301
gaactg                                                                  6

<210> 302
<211> 6
<212> DNA
<213> Artificial Sequence

<220>
<223> oligonucleotide 6-mer sequences

<400> 302
attatg                                                                  6

<210> 303
<211> 6
<212> DNA
<213> Artificial Sequence

<220>
<223> oligonucleotide 6-mer sequences

<400> 303
tgtatg                                                                  6

<210> 304
<211> 6
<212> DNA
<213> Artificial Sequence

<220>
<223> oligonucleotide 6-mer sequences

<400> 304
cgtatg                                                                  6

<210> 305
<211> 6
<212> DNA

<213> Artificial Sequence

<220>
<223> oligonucleotide 6-mer sequences

<400> 305
agtatg                                                                 6

<210> 306
<211> 6
<212> DNA
<213> Artificial Sequence

<220>
<223> oligonucleotide 6-mer sequences

<400> 306
tctatg                                                                 6

<210> 307
<211> 6
<212> DNA
<213> Artificial Sequence

<220>
<223> oligonucleotide 6-mer sequences

<400> 307
cctatg                                                                 6

<210> 308
<211> 6
<212> DNA
<213> Artificial Sequence

<220>
<223> oligonucleotide 6-mer sequences

<400> 308
actatg                                                                 6

<210> 309
<211> 6
<212> DNA
<213> Artificial Sequence

<220>
<223> oligonucleotide 6-mer sequences

<400> 309
tatatg                                                                 6

<210> 310
<211> 6
<212> DNA
<213> Artificial Sequence

<220>
<223> oligonucleotide 6-mer sequences

<400> 310
ttgatg                                                                 6

<210> 311
<211> 6

<212> DNA
<213> Artificial Sequence

<220>
<223> oligonucleotide 6-mer sequences

<400> 311
gtgatg                                                                  6

<210> 312
<211> 6
<212> DNA
<213> Artificial Sequence

<220>
<223> oligonucleotide 6-mer sequences

<400> 312
gggatg                                                                  6

<210> 313
<211> 6
<212> DNA
<213> Artificial Sequence

<220>
<223> oligonucleotide 6-mer sequences

<400> 313
cggatg                                                                  6

<210> 314
<211> 6
<212> DNA
<213> Artificial Sequence

<220>
<223> oligonucleotide 6-mer sequences

<400> 314
aggatg                                                                  6

<210> 315
<211> 6
<212> DNA
<213> Artificial Sequence

<220>
<223> oligonucleotide 6-mer sequences

<400> 315
tcgatg                                                                  6

<210> 316
<211> 6
<212> DNA
<213> Artificial Sequence

<220>
<223> oligonucleotide 6-mer sequences

<400> 316
ccgatg                                                                  6

<210> 317

<211> 6
<212> DNA
<213> Artificial Sequence

<220>
<223> oligonucleotide 6-mer sequences

<400> 317
acgatg                                                                  6

<210> 318
<211> 6
<212> DNA
<213> Artificial Sequence

<220>
<223> oligonucleotide 6-mer sequences

<400> 318
gagatg                                                                  6

<210> 319
<211> 6
<212> DNA
<213> Artificial Sequence

<220>
<223> oligonucleotide 6-mer sequences

<400> 319
aagatg                                                                  6

<210> 320
<211> 6
<212> DNA
<213> Artificial Sequence

<220>
<223> oligonucleotide 6-mer sequences

<400> 320
cgcatg                                                                  6

<210> 321
<211> 6
<212> DNA
<213> Artificial Sequence

<220>
<223> oligonucleotide 6-mer sequences

<400> 321
tacatg                                                                  6

<210> 322
<211> 6
<212> DNA
<213> Artificial Sequence

<220>
<223> oligonucleotide 6-mer sequences

<400> 322
gtaatg                                                                  6

<210> 323
<211> 6
<212> DNA
<213> Artificial Sequence

<220>
<223> oligonucleotide 6-mer sequences

<400> 323
ctaatg                                                                  6

<210> 324
<211> 6
<212> DNA
<213> Artificial Sequence

<220>
<223> oligonucleotide 6-mer sequences

<400> 324
ataatg                                                                  6

<210> 325
<211> 6
<212> DNA
<213> Artificial Sequence

<220>
<223> oligonucleotide 6-mer sequences

<400> 325
tgaatg                                                                  6

<210> 326
<211> 6
<212> DNA
<213> Artificial Sequence

<220>
<223> oligonucleotide 6-mer sequences

<400> 326
ggaatg                                                                  6

<210> 327
<211> 6
<212> DNA
<213> Artificial Sequence

<220>
<223> oligonucleotide 6-mer sequences

<400> 327
agaatg                                                                  6

<210> 328
<211> 6
<212> DNA
<213> Artificial Sequence

<220>
<223> oligonucleotide 6-mer sequences

<400> 328
gcaatg                                                                  6

<210> 329
<211> 6
<212> DNA
<213> Artificial Sequence

<220>
<223> oligonucleotide 6-mer sequences

<400> 329
aaaatg                                                                  6

<210> 330
<211> 6
<212> DNA
<213> Artificial Sequence

<220>
<223> oligonucleotide 6-mer sequences

<400> 330
ttttgg                                                                  6

<210> 331
<211> 6
<212> DNA
<213> Artificial Sequence

<220>
<223> oligonucleotide 6-mer sequences

<400> 331
gtttgg                                                                  6

<210> 332
<211> 6
<212> DNA
<213> Artificial Sequence

<220>
<223> oligonucleotide 6-mer sequences

<400> 332
tgttgg                                                                  6

<210> 333
<211> 6
<212> DNA
<213> Artificial Sequence

<220>
<223> oligonucleotide 6-mer sequences

<400> 333
gattgg                                                                  6

<210> 334
<211> 6
<212> DNA
<213> Artificial Sequence

<220>
<223> oligonucleotide 6-mer sequences

<400> 334

tcgtgg                                                                  6

<210> 335
<211> 6
<212> DNA
<213> Artificial Sequence

<220>
<223> oligonucleotide 6-mer sequences

<400> 335
aactgg                                                                  6

<210> 336
<211> 6
<212> DNA
<213> Artificial Sequence

<220>
<223> oligonucleotide 6-mer sequences

<400> 336
ctatgg                                                                  6

<210> 337
<211> 6
<212> DNA
<213> Artificial Sequence

<220>
<223> oligonucleotide 6-mer sequences

<400> 337
ggatgg                                                                  6

<210> 338
<211> 6
<212> DNA
<213> Artificial Sequence

<220>
<223> oligonucleotide 6-mer sequences

<400> 338
cgatgg                                                                  6

<210> 339
<211> 6
<212> DNA
<213> Artificial Sequence

<220>
<223> oligonucleotide 6-mer sequences

<400> 339
taatgg                                                                  6

<210> 340
<211> 6
<212> DNA
<213> Artificial Sequence

<220>
<223> oligonucleotide 6-mer sequences

<400> 340
attggg                                                                  6

<210> 341
<211> 6
<212> DNA
<213> Artificial Sequence

<220>
<223> oligonucleotide 6-mer sequences

<400> 341
gatggg                                                                  6

<210> 342
<211> 6
<212> DNA
<213> Artificial Sequence

<220>
<223> oligonucleotide 6-mer sequences

<400> 342
tagggg                                                                  6

<210> 343
<211> 6
<212> DNA
<213> Artificial Sequence

<220>
<223> oligonucleotide 6-mer sequences

<400> 343
ttgcgg                                                                  6

<210> 344
<211> 6
<212> DNA
<213> Artificial Sequence

<220>
<223> oligonucleotide 6-mer sequences

<400> 344
atgcgg                                                                  6

<210> 345
<211> 6
<212> DNA
<213> Artificial Sequence

<220>
<223> oligonucleotide 6-mer sequences

<400> 345
aagcgg                                                                  6

<210> 346
<211> 6
<212> DNA
<213> Artificial Sequence

<220>
<223> oligonucleotide 6-mer sequences

<400> 346
aaccgg                                                                 6

<210> 347
<211> 6
<212> DNA
<213> Artificial Sequence

<220>
<223> oligonucleotide 6-mer sequences

<400> 347
atacgg                                                                 6

<210> 348
<211> 6
<212> DNA
<213> Artificial Sequence

<220>
<223> oligonucleotide 6-mer sequences

<400> 348
tttagg                                                                 6

<210> 349
<211> 6
<212> DNA
<213> Artificial Sequence

<220>
<223> oligonucleotide 6-mer sequences

<400> 349
gttagg                                                                 6

<210> 350
<211> 6
<212> DNA
<213> Artificial Sequence

<220>
<223> oligonucleotide 6-mer sequences

<400> 350
tgtagg                                                                 6

<210> 351
<211> 6
<212> DNA
<213> Artificial Sequence

<220>
<223> oligonucleotide 6-mer sequences

<400> 351
tatagg                                                                 6

<210> 352
<211> 6
<212> DNA
<213> Artificial Sequence

<220>

<223> oligonucleotide 6-mer sequences

<400> 352
catagg                                                                  6

<210> 353
<211> 6
<212> DNA
<213> Artificial Sequence

<220>
<223> oligonucleotide 6-mer sequences

<400> 353
atgagg                                                                  6

<210> 354
<211> 6
<212> DNA
<213> Artificial Sequence

<220>
<223> oligonucleotide 6-mer sequences

<400> 354
aagagg                                                                  6

<210> 355
<211> 6
<212> DNA
<213> Artificial Sequence

<220>
<223> oligonucleotide 6-mer sequences

<400> 355
ttaagg                                                                  6

<210> 356
<211> 6
<212> DNA
<213> Artificial Sequence

<220>
<223> oligonucleotide 6-mer sequences

<400> 356
gtaagg                                                                  6

<210> 357
<211> 6
<212> DNA
<213> Artificial Sequence

<220>
<223> oligonucleotide 6-mer sequences

<400> 357
ataagg                                                                  6

<210> 358
<211> 6
<212> DNA
<213> Artificial Sequence

<220>
<223> oligonucleotide 6-mer sequences

<400> 358
tgaagg                                                                  6

<210> 359
<211> 6
<212> DNA
<213> Artificial Sequence

<220>
<223> oligonucleotide 6-mer sequences

<400> 359
acaagg                                                                  6

<210> 360
<211> 6
<212> DNA
<213> Artificial Sequence

<220>
<223> oligonucleotide 6-mer sequences

<400> 360
gaaagg                                                                  6

<210> 361
<211> 6
<212> DNA
<213> Artificial Sequence

<220>
<223> oligonucleotide 6-mer sequences

<400> 361
atttcg                                                                  6

<210> 362
<211> 6
<212> DNA
<213> Artificial Sequence

<220>
<223> oligonucleotide 6-mer sequences

<400> 362
tgttcg                                                                  6

<210> 363
<211> 6
<212> DNA
<213> Artificial Sequence

<220>
<223> oligonucleotide 6-mer sequences

<400> 363
aattcg                                                                  6

<210> 364
<211> 6
<212> DNA
<213> Artificial Sequence

<220>
<223> oligonucleotide 6-mer sequences

<400> 364
ctgtcg                                                                  6

<210> 365
<211> 6
<212> DNA
<213> Artificial Sequence

<220>
<223> oligonucleotide 6-mer sequences

<400> 365
tagtcg                                                                  6

<210> 366
<211> 6
<212> DNA
<213> Artificial Sequence

<220>
<223> oligonucleotide 6-mer sequences

<400> 366
gagtcg                                                                  6

<210> 367
<211> 6
<212> DNA
<213> Artificial Sequence

<220>
<223> oligonucleotide 6-mer sequences

<400> 367
tgctcg                                                                  6

<210> 368
<211> 6
<212> DNA
<213> Artificial Sequence

<220>
<223> oligonucleotide 6-mer sequences

<400> 368
atatcg                                                                  6

<210> 369
<211> 6
<212> DNA
<213> Artificial Sequence

<220>
<223> oligonucleotide 6-mer sequences

<400> 369
tcatcg                                                                  6

<210> 370
<211> 6
<212> DNA

<213> Artificial Sequence

<220>
<223> oligonucleotide 6-mer sequences

<400> 370
taatcg                                                                 6

<210> 371
<211> 6
<212> DNA
<213> Artificial Sequence

<220>
<223> oligonucleotide 6-mer sequences

<400> 371
gttgcg                                                                 6

<210> 372
<211> 6
<212> DNA
<213> Artificial Sequence

<220>
<223> oligonucleotide 6-mer sequences

<400> 372
tatgcg                                                                 6

<210> 373
<211> 6
<212> DNA
<213> Artificial Sequence

<220>
<223> oligonucleotide 6-mer sequences

<400> 373
gatgcg                                                                 6

<210> 374
<211> 6
<212> DNA
<213> Artificial Sequence

<220>
<223> oligonucleotide 6-mer sequences

<400> 374
ctggcg                                                                 6

<210> 375
<211> 6
<212> DNA
<213> Artificial Sequence

<220>
<223> oligonucleotide 6-mer sequences

<400> 375
aaggcg                                                                 6

<210> 376
<211> 6

<212> DNA
<213> Artificial Sequence

<220>
<223> oligonucleotide 6-mer sequences

<400> 376
tacgcg                                                                  6

<210> 377
<211> 6
<212> DNA
<213> Artificial Sequence

<220>
<223> oligonucleotide 6-mer sequences

<400> 377
aacgcg                                                                  6

<210> 378
<211> 6
<212> DNA
<213> Artificial Sequence

<220>
<223> oligonucleotide 6-mer sequences

<400> 378
atagcg                                                                  6

<210> 379
<211> 6
<212> DNA
<213> Artificial Sequence

<220>
<223> oligonucleotide 6-mer sequences

<400> 379
taagcg                                                                  6

<210> 380
<211> 6
<212> DNA
<213> Artificial Sequence

<220>
<223> oligonucleotide 6-mer sequences

<400> 380
catccg                                                                  6

<210> 381
<211> 6
<212> DNA
<213> Artificial Sequence

<220>
<223> oligonucleotide 6-mer sequences

<400> 381
aatccg                                                                  6

<210> 382

<211> 6
<212> DNA
<213> Artificial Sequence

<220>
<223> oligonucleotide 6-mer sequences

<400> 382
atgccg                                                                 6

<210> 383
<211> 6
<212> DNA
<213> Artificial Sequence

<220>
<223> oligonucleotide 6-mer sequences

<400> 383
aggccg                                                                 6

<210> 384
<211> 6
<212> DNA
<213> Artificial Sequence

<220>
<223> oligonucleotide 6-mer sequences

<400> 384
ataccg                                                                 6

<210> 385
<211> 6
<212> DNA
<213> Artificial Sequence

<220>
<223> oligonucleotide 6-mer sequences

<400> 385
agaccg                                                                 6

<210> 386
<211> 6
<212> DNA
<213> Artificial Sequence

<220>
<223> oligonucleotide 6-mer sequences

<400> 386
taaccg                                                                 6

<210> 387
<211> 6
<212> DNA
<213> Artificial Sequence

<220>
<223> oligonucleotide 6-mer sequences

<400> 387
aaaccg                                                                 6

<210> 388
<211> 6
<212> DNA
<213> Artificial Sequence

<220>
<223> oligonucleotide 6-mer sequences

<400> 388
tttacg                                                                  6

<210> 389
<211> 6
<212> DNA
<213> Artificial Sequence

<220>
<223> oligonucleotide 6-mer sequences

<400> 389
attacg                                                                  6

<210> 390
<211> 6
<212> DNA
<213> Artificial Sequence

<220>
<223> oligonucleotide 6-mer sequences

<400> 390
tgtacg                                                                  6

<210> 391
<211> 6
<212> DNA
<213> Artificial Sequence

<220>
<223> oligonucleotide 6-mer sequences

<400> 391
agtacg                                                                  6

<210> 392
<211> 6
<212> DNA
<213> Artificial Sequence

<220>
<223> oligonucleotide 6-mer sequences

<400> 392
gatacg                                                                  6

<210> 393
<211> 6
<212> DNA
<213> Artificial Sequence

<220>
<223> oligonucleotide 6-mer sequences

<400> 393
catacg                                                                  6

<210> 394
<211> 6
<212> DNA
<213> Artificial Sequence

<220>
<223> oligonucleotide 6-mer sequences

<400> 394
ctgacg                                                                  6

<210> 395
<211> 6
<212> DNA
<213> Artificial Sequence

<220>
<223> oligonucleotide 6-mer sequences

<400> 395
tcgacg                                                                  6

<210> 396
<211> 6
<212> DNA
<213> Artificial Sequence

<220>
<223> oligonucleotide 6-mer sequences

<400> 396
tagacg                                                                  6

<210> 397
<211> 6
<212> DNA
<213> Artificial Sequence

<220>
<223> oligonucleotide 6-mer sequences

<400> 397
gtcacg                                                                  6

<210> 398
<211> 6
<212> DNA
<213> Artificial Sequence

<220>
<223> oligonucleotide 6-mer sequences

<400> 398
tacacg                                                                  6

<210> 399
<211> 6
<212> DNA
<213> Artificial Sequence

<220>
<223> oligonucleotide 6-mer sequences

<400> 399

ctaacg                                                                  6

<210> 400
<211> 6
<212> DNA
<213> Artificial Sequence

<220>
<223> oligonucleotide 6-mer sequences

<400> 400
acaacg                                                                  6

<210> 401
<211> 6
<212> DNA
<213> Artificial Sequence

<220>
<223> oligonucleotide 6-mer sequences

<400> 401
gaaacg                                                                  6

<210> 402
<211> 6
<212> DNA
<213> Artificial Sequence

<220>
<223> oligonucleotide 6-mer sequences

<400> 402
caaacg                                                                  6

<210> 403
<211> 6
<212> DNA
<213> Artificial Sequence

<220>
<223> oligonucleotide 6-mer sequences

<400> 403
ttttag                                                                  6

<210> 404
<211> 6
<212> DNA
<213> Artificial Sequence

<220>
<223> oligonucleotide 6-mer sequences

<400> 404
ctttag                                                                  6

<210> 405
<211> 6
<212> DNA
<213> Artificial Sequence

<220>
<223> oligonucleotide 6-mer sequences

<400> 405
cgttag                                                                    6

<210> 406
<211> 6
<212> DNA
<213> Artificial Sequence

<220>
<223> oligonucleotide 6-mer sequences

<400> 406
ttgtag                                                                    6

<210> 407
<211> 6
<212> DNA
<213> Artificial Sequence

<220>
<223> oligonucleotide 6-mer sequences

<400> 407
ctgtag                                                                    6

<210> 408
<211> 6
<212> DNA
<213> Artificial Sequence

<220>
<223> oligonucleotide 6-mer sequences

<400> 408
atgtag                                                                    6

<210> 409
<211> 6
<212> DNA
<213> Artificial Sequence

<220>
<223> oligonucleotide 6-mer sequences

<400> 409
tcgtag                                                                    6

<210> 410
<211> 6
<212> DNA
<213> Artificial Sequence

<220>
<223> oligonucleotide 6-mer sequences

<400> 410
ccgtag                                                                    6

<210> 411
<211> 6
<212> DNA
<213> Artificial Sequence

<220>
<223> oligonucleotide 6-mer sequences

<400> 411
acgtag                                                                 6

<210> 412
<211> 6
<212> DNA
<213> Artificial Sequence

<220>
<223> oligonucleotide 6-mer sequences

<400> 412
ttctag                                                                 6

<210> 413
<211> 6
<212> DNA
<213> Artificial Sequence

<220>
<223> oligonucleotide 6-mer sequences

<400> 413
gtctag                                                                 6

<210> 414
<211> 6
<212> DNA
<213> Artificial Sequence

<220>
<223> oligonucleotide 6-mer sequences

<400> 414
cgctag                                                                 6

<210> 415
<211> 6
<212> DNA
<213> Artificial Sequence

<220>
<223> oligonucleotide 6-mer sequences

<400> 415
agctag                                                                 6

<210> 416
<211> 6
<212> DNA
<213> Artificial Sequence

<220>
<223> oligonucleotide 6-mer sequences

<400> 416
gcctag                                                                 6

<210> 417
<211> 6
<212> DNA
<213> Artificial Sequence

<220>

<223> oligonucleotide 6-mer sequences

<400> 417
ttatag                                                                 6

<210> 418
<211> 6
<212> DNA
<213> Artificial Sequence

<220>
<223> oligonucleotide 6-mer sequences

<400> 418
gtatag                                                                 6

<210> 419
<211> 6
<212> DNA
<213> Artificial Sequence

<220>
<223> oligonucleotide 6-mer sequences

<400> 419
ctatag                                                                 6

<210> 420
<211> 6
<212> DNA
<213> Artificial Sequence

<220>
<223> oligonucleotide 6-mer sequences

<400> 420
atatag                                                                 6

<210> 421
<211> 6
<212> DNA
<213> Artificial Sequence

<220>
<223> oligonucleotide 6-mer sequences

<400> 421
cgatag                                                                 6

<210> 422
<211> 6
<212> DNA
<213> Artificial Sequence

<220>
<223> oligonucleotide 6-mer sequences

<400> 422
ccatag                                                                 6

<210> 423
<211> 6
<212> DNA
<213> Artificial Sequence

<220>
<223> oligonucleotide 6-mer sequences

<400> 423
acatag                                                                  6

<210> 424
<211> 6
<212> DNA
<213> Artificial Sequence

<220>
<223> oligonucleotide 6-mer sequences

<400> 424
attgag                                                                  6

<210> 425
<211> 6
<212> DNA
<213> Artificial Sequence

<220>
<223> oligonucleotide 6-mer sequences

<400> 425
tgtgag                                                                  6

<210> 426
<211> 6
<212> DNA
<213> Artificial Sequence

<220>
<223> oligonucleotide 6-mer sequences

<400> 426
tatgag                                                                  6

<210> 427
<211> 6
<212> DNA
<213> Artificial Sequence

<220>
<223> oligonucleotide 6-mer sequences

<400> 427
catgag                                                                  6

<210> 428
<211> 6
<212> DNA
<213> Artificial Sequence

<220>
<223> oligonucleotide 6-mer sequences

<400> 428
ttcgag                                                                  6

<210> 429
<211> 6
<212> DNA
<213> Artificial Sequence

<220>
<223> oligonucleotide 6-mer sequences

<400> 429
ctcgag                                                                 6

<210> 430
<211> 6
<212> DNA
<213> Artificial Sequence

<220>
<223> oligonucleotide 6-mer sequences

<400> 430
aacgag                                                                 6

<210> 431
<211> 6
<212> DNA
<213> Artificial Sequence

<220>
<223> oligonucleotide 6-mer sequences

<400> 431
gtagag                                                                 6

<210> 432
<211> 6
<212> DNA
<213> Artificial Sequence

<220>
<223> oligonucleotide 6-mer sequences

<400> 432
atagag                                                                 6

<210> 433
<211> 6
<212> DNA
<213> Artificial Sequence

<220>
<223> oligonucleotide 6-mer sequences

<400> 433
tgagag                                                                 6

<210> 434
<211> 6
<212> DNA
<213> Artificial Sequence

<220>
<223> oligonucleotide 6-mer sequences

<400> 434
acagag                                                                 6

<210> 435
<211> 6
<212> DNA

<213> Artificial Sequence

<220>
<223> oligonucleotide 6-mer sequences

<400> 435
cttcag                                                                 6

<210> 436
<211> 6
<212> DNA
<213> Artificial Sequence

<220>
<223> oligonucleotide 6-mer sequences

<400> 436
aatcag                                                                 6

<210> 437
<211> 6
<212> DNA
<213> Artificial Sequence

<220>
<223> oligonucleotide 6-mer sequences

<400> 437
gcgcag                                                                 6

<210> 438
<211> 6
<212> DNA
<213> Artificial Sequence

<220>
<223> oligonucleotide 6-mer sequences

<400> 438
acgcag                                                                 6

<210> 439
<211> 6
<212> DNA
<213> Artificial Sequence

<220>
<223> oligonucleotide 6-mer sequences

<400> 439
taccag                                                                 6

<210> 440
<211> 6
<212> DNA
<213> Artificial Sequence

<220>
<223> oligonucleotide 6-mer sequences

<400> 440
gtacag                                                                 6

<210> 441
<211> 6

<212> DNA
<213> Artificial Sequence

<220>
<223> oligonucleotide 6-mer sequences

<400> 441
ctacag                                                                  6

<210> 442
<211> 6
<212> DNA
<213> Artificial Sequence

<220>
<223> oligonucleotide 6-mer sequences

<400> 442
atacag                                                                  6

<210> 443
<211> 6
<212> DNA
<213> Artificial Sequence

<220>
<223> oligonucleotide 6-mer sequences

<400> 443
cgacag                                                                  6

<210> 444
<211> 6
<212> DNA
<213> Artificial Sequence

<220>
<223> oligonucleotide 6-mer sequences

<400> 444
gcacag                                                                  6

<210> 445
<211> 6
<212> DNA
<213> Artificial Sequence

<220>
<223> oligonucleotide 6-mer sequences

<400> 445
gaacag                                                                  6

<210> 446
<211> 6
<212> DNA
<213> Artificial Sequence

<220>
<223> oligonucleotide 6-mer sequences

<400> 446
gttaag                                                                  6

<210> 447

<211> 6
<212> DNA
<213> Artificial Sequence

<220>
<223> oligonucleotide 6-mer sequences

<400> 447
tgtaag                                                                 6

<210> 448
<211> 6
<212> DNA
<213> Artificial Sequence

<220>
<223> oligonucleotide 6-mer sequences

<400> 448
ggtaag                                                                 6

<210> 449
<211> 6
<212> DNA
<213> Artificial Sequence

<220>
<223> oligonucleotide 6-mer sequences

<400> 449
cgtaag                                                                 6

<210> 450
<211> 6
<212> DNA
<213> Artificial Sequence

<220>
<223> oligonucleotide 6-mer sequences

<400> 450
agtaag                                                                 6

<210> 451
<211> 6
<212> DNA
<213> Artificial Sequence

<220>
<223> oligonucleotide 6-mer sequences

<400> 451
cctaag                                                                 6

<210> 452
<211> 6
<212> DNA
<213> Artificial Sequence

<220>
<223> oligonucleotide 6-mer sequences

<400> 452
tataag                                                                 6

<210> 453
<211> 6
<212> DNA
<213> Artificial Sequence

<220>
<223> oligonucleotide 6-mer sequences

<400> 453
gataag                                                                 6

<210> 454
<211> 6
<212> DNA
<213> Artificial Sequence

<220>
<223> oligonucleotide 6-mer sequences

<400> 454
cataag                                                                 6

<210> 455
<211> 6
<212> DNA
<213> Artificial Sequence

<220>
<223> oligonucleotide 6-mer sequences

<400> 455
aataag                                                                 6

<210> 456
<211> 6
<212> DNA
<213> Artificial Sequence

<220>
<223> oligonucleotide 6-mer sequences

<400> 456
gtgaag                                                                 6

<210> 457
<211> 6
<212> DNA
<213> Artificial Sequence

<220>
<223> oligonucleotide 6-mer sequences

<400> 457
atgaag                                                                 6

<210> 458
<211> 6
<212> DNA
<213> Artificial Sequence

<220>
<223> oligonucleotide 6-mer sequences

<400> 458
tggaag                                                                 6

<210> 459
<211> 6
<212> DNA
<213> Artificial Sequence

<220>
<223> oligonucleotide 6-mer sequences

<400> 459
tagaag                                                                  6

<210> 460
<211> 6
<212> DNA
<213> Artificial Sequence

<220>
<223> oligonucleotide 6-mer sequences

<400> 460
gagaag                                                                  6

<210> 461
<211> 6
<212> DNA
<213> Artificial Sequence

<220>
<223> oligonucleotide 6-mer sequences

<400> 461
tacaag                                                                  6

<210> 462
<211> 6
<212> DNA
<213> Artificial Sequence

<220>
<223> oligonucleotide 6-mer sequences

<400> 462
aacaag                                                                  6

<210> 463
<211> 6
<212> DNA
<213> Artificial Sequence

<220>
<223> oligonucleotide 6-mer sequences

<400> 463
gtaaag                                                                  6

<210> 464
<211> 6
<212> DNA
<213> Artificial Sequence

<220>
<223> oligonucleotide 6-mer sequences

<400> 464

taaaag                                                                  6

<210> 465
<211> 6
<212> DNA
<213> Artificial Sequence

<220>
<223> oligonucleotide 6-mer sequences

<400> 465
gttttc                                                                  6

<210> 466
<211> 6
<212> DNA
<213> Artificial Sequence

<220>
<223> oligonucleotide 6-mer sequences

<400> 466
gatttc                                                                  6

<210> 467
<211> 6
<212> DNA
<213> Artificial Sequence

<220>
<223> oligonucleotide 6-mer sequences

<400> 467
gagttc                                                                  6

<210> 468
<211> 6
<212> DNA
<213> Artificial Sequence

<220>
<223> oligonucleotide 6-mer sequences

<400> 468
atattc                                                                  6

<210> 469
<211> 6
<212> DNA
<213> Artificial Sequence

<220>
<223> oligonucleotide 6-mer sequences

<400> 469
cgattc                                                                  6

<210> 470
<211> 6
<212> DNA
<213> Artificial Sequence

<220>
<223> oligonucleotide 6-mer sequences

<400> 470
agattc                                                                  6

<210> 471
<211> 6
<212> DNA
<213> Artificial Sequence

<220>
<223> oligonucleotide 6-mer sequences

<400> 471
taattc                                                                  6

<210> 472
<211> 6
<212> DNA
<213> Artificial Sequence

<220>
<223> oligonucleotide 6-mer sequences

<400> 472
tttgtc                                                                  6

<210> 473
<211> 6
<212> DNA
<213> Artificial Sequence

<220>
<223> oligonucleotide 6-mer sequences

<400> 473
attgtc                                                                  6

<210> 474
<211> 6
<212> DNA
<213> Artificial Sequence

<220>
<223> oligonucleotide 6-mer sequences

<400> 474
agtgtc                                                                  6

<210> 475
<211> 6
<212> DNA
<213> Artificial Sequence

<220>
<223> oligonucleotide 6-mer sequences

<400> 475
gatgtc                                                                  6

<210> 476
<211> 6
<212> DNA
<213> Artificial Sequence

<220>
<223> oligonucleotide 6-mer sequences

<400> 476
taggtc                                                                 6

<210> 477
<211> 6
<212> DNA
<213> Artificial Sequence

<220>
<223> oligonucleotide 6-mer sequences

<400> 477
tacgtc                                                                 6

<210> 478
<211> 6
<212> DNA
<213> Artificial Sequence

<220>
<223> oligonucleotide 6-mer sequences

<400> 478
aacgtc                                                                 6

<210> 479
<211> 6
<212> DNA
<213> Artificial Sequence

<220>
<223> oligonucleotide 6-mer sequences

<400> 479
gtagtc                                                                 6

<210> 480
<211> 6
<212> DNA
<213> Artificial Sequence

<220>
<223> oligonucleotide 6-mer sequences

<400> 480
ctagtc                                                                 6

<210> 481
<211> 6
<212> DNA
<213> Artificial Sequence

<220>
<223> oligonucleotide 6-mer sequences

<400> 481
cgagtc                                                                 6

<210> 482
<211> 6
<212> DNA
<213> Artificial Sequence

<220>

<223> oligonucleotide 6-mer sequences

<400> 482
caagtc                                                                 6

<210> 483
<211> 6
<212> DNA
<213> Artificial Sequence

<220>
<223> oligonucleotide 6-mer sequences

<400> 483
aaagtc                                                                 6

<210> 484
<211> 6
<212> DNA
<213> Artificial Sequence

<220>
<223> oligonucleotide 6-mer sequences

<400> 484
attctc                                                                 6

<210> 485
<211> 6
<212> DNA
<213> Artificial Sequence

<220>
<223> oligonucleotide 6-mer sequences

<400> 485
cgtctc                                                                 6

<210> 486
<211> 6
<212> DNA
<213> Artificial Sequence

<220>
<223> oligonucleotide 6-mer sequences

<400> 486
gctctc                                                                 6

<210> 487
<211> 6
<212> DNA
<213> Artificial Sequence

<220>
<223> oligonucleotide 6-mer sequences

<400> 487
tatctc                                                                 6

<210> 488
<211> 6
<212> DNA
<213> Artificial Sequence

<220>
<223> oligonucleotide 6-mer sequences

<400> 488
tgcctc                                                                  6

<210> 489
<211> 6
<212> DNA
<213> Artificial Sequence

<220>
<223> oligonucleotide 6-mer sequences

<400> 489
agcctc                                                                  6

<210> 490
<211> 6
<212> DNA
<213> Artificial Sequence

<220>
<223> oligonucleotide 6-mer sequences

<400> 490
gtactc                                                                  6

<210> 491
<211> 6
<212> DNA
<213> Artificial Sequence

<220>
<223> oligonucleotide 6-mer sequences

<400> 491
tgactc                                                                  6

<210> 492
<211> 6
<212> DNA
<213> Artificial Sequence

<220>
<223> oligonucleotide 6-mer sequences

<400> 492
taactc                                                                  6

<210> 493
<211> 6
<212> DNA
<213> Artificial Sequence

<220>
<223> oligonucleotide 6-mer sequences

<400> 493
caactc                                                                  6

<210> 494
<211> 6
<212> DNA
<213> Artificial Sequence

<220>
<223> oligonucleotide 6-mer sequences

<400> 494
tttatc                                                                  6

<210> 495
<211> 6
<212> DNA
<213> Artificial Sequence

<220>
<223> oligonucleotide 6-mer sequences

<400> 495
attatc                                                                  6

<210> 496
<211> 6
<212> DNA
<213> Artificial Sequence

<220>
<223> oligonucleotide 6-mer sequences

<400> 496
tgtatc                                                                  6

<210> 497
<211> 6
<212> DNA
<213> Artificial Sequence

<220>
<223> oligonucleotide 6-mer sequences

<400> 497
agtatc                                                                  6

<210> 498
<211> 6
<212> DNA
<213> Artificial Sequence

<220>
<223> oligonucleotide 6-mer sequences

<400> 498
tctatc                                                                  6

<210> 499
<211> 6
<212> DNA
<213> Artificial Sequence

<220>
<223> oligonucleotide 6-mer sequences

<400> 499
gatatc                                                                  6

<210> 500
<211> 6
<212> DNA

<213> Artificial Sequence

<220>
<223> oligonucleotide 6-mer sequences

<400> 500
catatc                                                                  6

<210> 501
<211> 6
<212> DNA
<213> Artificial Sequence

<220>
<223> oligonucleotide 6-mer sequences

<400> 501
gtgatc                                                                  6

<210> 502
<211> 6
<212> DNA
<213> Artificial Sequence

<220>
<223> oligonucleotide 6-mer sequences

<400> 502
gtcatc                                                                  6

<210> 503
<211> 6
<212> DNA
<213> Artificial Sequence

<220>
<223> oligonucleotide 6-mer sequences

<400> 503
ctcatc                                                                  6

<210> 504
<211> 6
<212> DNA
<213> Artificial Sequence

<220>
<223> oligonucleotide 6-mer sequences

<400> 504
tccatc                                                                  6

<210> 505
<211> 6
<212> DNA
<213> Artificial Sequence

<220>
<223> oligonucleotide 6-mer sequences

<400> 505
tacatc                                                                  6

<210> 506
<211> 6

<212> DNA
<213> Artificial Sequence

<220>
<223> oligonucleotide 6-mer sequences

<400> 506
cgaatc                                                                  6

<210> 507
<211> 6
<212> DNA
<213> Artificial Sequence

<220>
<223> oligonucleotide 6-mer sequences

<400> 507
taaatc                                                                  6

<210> 508
<211> 6
<212> DNA
<213> Artificial Sequence

<220>
<223> oligonucleotide 6-mer sequences

<400> 508
gtttgc                                                                  6

<210> 509
<211> 6
<212> DNA
<213> Artificial Sequence

<220>
<223> oligonucleotide 6-mer sequences

<400> 509
tgttgc                                                                  6

<210> 510
<211> 6
<212> DNA
<213> Artificial Sequence

<220>
<223> oligonucleotide 6-mer sequences

<400> 510
agttgc                                                                  6

<210> 511
<211> 6
<212> DNA
<213> Artificial Sequence

<220>
<223> oligonucleotide 6-mer sequences

<400> 511
gtgtgc                                                                  6

<210> 512

<211> 6
<212> DNA
<213> Artificial Sequence

<220>
<223> oligonucleotide 6-mer sequences

<400> 512
ctgtgc                                                                  6

<210> 513
<211> 6
<212> DNA
<213> Artificial Sequence

<220>
<223> oligonucleotide 6-mer sequences

<400> 513
atgtgc                                                                  6

<210> 514
<211> 6
<212> DNA
<213> Artificial Sequence

<220>
<223> oligonucleotide 6-mer sequences

<400> 514
tagtgc                                                                  6

<210> 515
<211> 6
<212> DNA
<213> Artificial Sequence

<220>
<223> oligonucleotide 6-mer sequences

<400> 515
tactgc                                                                  6

<210> 516
<211> 6
<212> DNA
<213> Artificial Sequence

<220>
<223> oligonucleotide 6-mer sequences

<400> 516
ttatgc                                                                  6

<210> 517
<211> 6
<212> DNA
<213> Artificial Sequence

<220>
<223> oligonucleotide 6-mer sequences

<400> 517
gtatgc                                                                  6

<210> 518
<211> 6
<212> DNA
<213> Artificial Sequence

<220>
<223> oligonucleotide 6-mer sequences

<400> 518
ctatgc                                                                 6

<210> 519
<211> 6
<212> DNA
<213> Artificial Sequence

<220>
<223> oligonucleotide 6-mer sequences

<400> 519
tgatgc                                                                 6

<210> 520
<211> 6
<212> DNA
<213> Artificial Sequence

<220>
<223> oligonucleotide 6-mer sequences

<400> 520
ggatgc                                                                 6

<210> 521
<211> 6
<212> DNA
<213> Artificial Sequence

<220>
<223> oligonucleotide 6-mer sequences

<400> 521
taatgc                                                                 6

<210> 522
<211> 6
<212> DNA
<213> Artificial Sequence

<220>
<223> oligonucleotide 6-mer sequences

<400> 522
caatgc                                                                 6

<210> 523
<211> 6
<212> DNA
<213> Artificial Sequence

<220>
<223> oligonucleotide 6-mer sequences

<400> 523
gttggc                                                                 6

<210> 524
<211> 6
<212> DNA
<213> Artificial Sequence

<220>
<223> oligonucleotide 6-mer sequences

<400> 524
aatggc                                                                  6

<210> 525
<211> 6
<212> DNA
<213> Artificial Sequence

<220>
<223> oligonucleotide 6-mer sequences

<400> 525
taaggc                                                                  6

<210> 526
<211> 6
<212> DNA
<213> Artificial Sequence

<220>
<223> oligonucleotide 6-mer sequences

<400> 526
gaaggc                                                                  6

<210> 527
<211> 6
<212> DNA
<213> Artificial Sequence

<220>
<223> oligonucleotide 6-mer sequences

<400> 527
tgtcgc                                                                  6

<210> 528
<211> 6
<212> DNA
<213> Artificial Sequence

<220>
<223> oligonucleotide 6-mer sequences

<400> 528
agtcgc                                                                  6

<210> 529
<211> 6
<212> DNA
<213> Artificial Sequence

<220>
<223> oligonucleotide 6-mer sequences

<400> 529

tatcgc                                                                 6

<210> 530
<211> 6
<212> DNA
<213> Artificial Sequence

<220>
<223> oligonucleotide 6-mer sequences

<400> 530
aagcgc                                                                 6

<210> 531
<211> 6
<212> DNA
<213> Artificial Sequence

<220>
<223> oligonucleotide 6-mer sequences

<400> 531
ttacgc                                                                 6

<210> 532
<211> 6
<212> DNA
<213> Artificial Sequence

<220>
<223> oligonucleotide 6-mer sequences

<400> 532
ctacgc                                                                 6

<210> 533
<211> 6
<212> DNA
<213> Artificial Sequence

<220>
<223> oligonucleotide 6-mer sequences

<400> 533
tgacgc                                                                 6

<210> 534
<211> 6
<212> DNA
<213> Artificial Sequence

<220>
<223> oligonucleotide 6-mer sequences

<400> 534
gctagc                                                                 6

<210> 535
<211> 6
<212> DNA
<213> Artificial Sequence

<220>
<223> oligonucleotide 6-mer sequences

<400> 535
actagc                                                                    6

<210> 536
<211> 6
<212> DNA
<213> Artificial Sequence

<220>
<223> oligonucleotide 6-mer sequences

<400> 536
tatagc                                                                    6

<210> 537
<211> 6
<212> DNA
<213> Artificial Sequence

<220>
<223> oligonucleotide 6-mer sequences

<400> 537
gatagc                                                                    6

<210> 538
<211> 6
<212> DNA
<213> Artificial Sequence

<220>
<223> oligonucleotide 6-mer sequences

<400> 538
catagc                                                                    6

<210> 539
<211> 6
<212> DNA
<213> Artificial Sequence

<220>
<223> oligonucleotide 6-mer sequences

<400> 539
aatagc                                                                    6

<210> 540
<211> 6
<212> DNA
<213> Artificial Sequence

<220>
<223> oligonucleotide 6-mer sequences

<400> 540
tcgagc                                                                    6

<210> 541
<211> 6
<212> DNA
<213> Artificial Sequence

<220>
<223> oligonucleotide 6-mer sequences

<400> 541
atcagc                                                                 6

<210> 542
<211> 6
<212> DNA
<213> Artificial Sequence

<220>
<223> oligonucleotide 6-mer sequences

<400> 542
tacagc                                                                 6

<210> 543
<211> 6
<212> DNA
<213> Artificial Sequence

<220>
<223> oligonucleotide 6-mer sequences

<400> 543
cacagc                                                                 6

<210> 544
<211> 6
<212> DNA
<213> Artificial Sequence

<220>
<223> oligonucleotide 6-mer sequences

<400> 544
gtaagc                                                                 6

<210> 545
<211> 6
<212> DNA
<213> Artificial Sequence

<220>
<223> oligonucleotide 6-mer sequences

<400> 545
ctaagc                                                                 6

<210> 546
<211> 6
<212> DNA
<213> Artificial Sequence

<220>
<223> oligonucleotide 6-mer sequences

<400> 546
ataagc                                                                 6

<210> 547
<211> 6
<212> DNA
<213> Artificial Sequence

<220>

<223> oligonucleotide 6-mer sequences

<400> 547
tgaagc                                                                  6

<210> 548
<211> 6
<212> DNA
<213> Artificial Sequence

<220>
<223> oligonucleotide 6-mer sequences

<400> 548
gcttcc                                                                  6

<210> 549
<211> 6
<212> DNA
<213> Artificial Sequence

<220>
<223> oligonucleotide 6-mer sequences

<400> 549
aggtcc                                                                  6

<210> 550
<211> 6
<212> DNA
<213> Artificial Sequence

<220>
<223> oligonucleotide 6-mer sequences

<400> 550
acgtcc                                                                  6

<210> 551
<211> 6
<212> DNA
<213> Artificial Sequence

<220>
<223> oligonucleotide 6-mer sequences

<400> 551
tagtcc                                                                  6

<210> 552
<211> 6
<212> DNA
<213> Artificial Sequence

<220>
<223> oligonucleotide 6-mer sequences

<400> 552
aagtcc                                                                  6

<210> 553
<211> 6
<212> DNA
<213> Artificial Sequence

<220>
<223> oligonucleotide 6-mer sequences

<400> 553
gtctcc                                                                 6

<210> 554
<211> 6
<212> DNA
<213> Artificial Sequence

<220>
<223> oligonucleotide 6-mer sequences

<400> 554
agctcc                                                                 6

<210> 555
<211> 6
<212> DNA
<213> Artificial Sequence

<220>
<223> oligonucleotide 6-mer sequences

<400> 555
aaatcc                                                                 6

<210> 556
<211> 6
<212> DNA
<213> Artificial Sequence

<220>
<223> oligonucleotide 6-mer sequences

<400> 556
tgtgcc                                                                 6

<210> 557
<211> 6
<212> DNA
<213> Artificial Sequence

<220>
<223> oligonucleotide 6-mer sequences

<400> 557
tatgcc                                                                 6

<210> 558
<211> 6
<212> DNA
<213> Artificial Sequence

<220>
<223> oligonucleotide 6-mer sequences

<400> 558
gatgcc                                                                 6

<210> 559
<211> 6
<212> DNA
<213> Artificial Sequence

<220>
<223> oligonucleotide 6-mer sequences

<400> 559
taggcc                                                                 6

<210> 560
<211> 6
<212> DNA
<213> Artificial Sequence

<220>
<223> oligonucleotide 6-mer sequences

<400> 560
ctagcc                                                                 6

<210> 561
<211> 6
<212> DNA
<213> Artificial Sequence

<220>
<223> oligonucleotide 6-mer sequences

<400> 561
atagcc                                                                 6

<210> 562
<211> 6
<212> DNA
<213> Artificial Sequence

<220>
<223> oligonucleotide 6-mer sequences

<400> 562
acagcc                                                                 6

<210> 563
<211> 6
<212> DNA
<213> Artificial Sequence

<220>
<223> oligonucleotide 6-mer sequences

<400> 563
agtccc                                                                 6

<210> 564
<211> 6
<212> DNA
<213> Artificial Sequence

<220>
<223> oligonucleotide 6-mer sequences

<400> 564
tagccc                                                                 6

<210> 565
<211> 6
<212> DNA

<213> Artificial Sequence

<220>
<223> oligonucleotide 6-mer sequences

<400> 565
gtaccc                                                                  6

<210> 566
<211> 6
<212> DNA
<213> Artificial Sequence

<220>
<223> oligonucleotide 6-mer sequences

<400> 566
ctaccc                                                                  6

<210> 567
<211> 6
<212> DNA
<213> Artificial Sequence

<220>
<223> oligonucleotide 6-mer sequences

<400> 567
ggtacc                                                                  6

<210> 568
<211> 6
<212> DNA
<213> Artificial Sequence

<220>
<223> oligonucleotide 6-mer sequences

<400> 568
cgtacc                                                                  6

<210> 569
<211> 6
<212> DNA
<213> Artificial Sequence

<220>
<223> oligonucleotide 6-mer sequences

<400> 569
agtacc                                                                  6

<210> 570
<211> 6
<212> DNA
<213> Artificial Sequence

<220>
<223> oligonucleotide 6-mer sequences

<400> 570
tctacc                                                                  6

<210> 571
<211> 6

<212> DNA
<213> Artificial Sequence

<220>
<223> oligonucleotide 6-mer sequences

<400> 571
cctacc                                                                  6

<210> 572
<211> 6
<212> DNA
<213> Artificial Sequence

<220>
<223> oligonucleotide 6-mer sequences

<400> 572
gatacc                                                                  6

<210> 573
<211> 6
<212> DNA
<213> Artificial Sequence

<220>
<223> oligonucleotide 6-mer sequences

<400> 573
aatacc                                                                  6

<210> 574
<211> 6
<212> DNA
<213> Artificial Sequence

<220>
<223> oligonucleotide 6-mer sequences

<400> 574
gtgacc                                                                  6

<210> 575
<211> 6
<212> DNA
<213> Artificial Sequence

<220>
<223> oligonucleotide 6-mer sequences

<400> 575
tggacc                                                                  6

<210> 576
<211> 6
<212> DNA
<213> Artificial Sequence

<220>
<223> oligonucleotide 6-mer sequences

<400> 576
atcacc                                                                  6

<210> 577

<211> 6
<212> DNA
<213> Artificial Sequence

<220>
<223> oligonucleotide 6-mer sequences

<400> 577
gtaacc                                                                  6

<210> 578
<211> 6
<212> DNA
<213> Artificial Sequence

<220>
<223> oligonucleotide 6-mer sequences

<400> 578
tattac                                                                  6

<210> 579
<211> 6
<212> DNA
<213> Artificial Sequence

<220>
<223> oligonucleotide 6-mer sequences

<400> 579
cattac                                                                  6

<210> 580
<211> 6
<212> DNA
<213> Artificial Sequence

<220>
<223> oligonucleotide 6-mer sequences

<400> 580
ttgtac                                                                  6

<210> 581
<211> 6
<212> DNA
<213> Artificial Sequence

<220>
<223> oligonucleotide 6-mer sequences

<400> 581
atgtac                                                                  6

<210> 582
<211> 6
<212> DNA
<213> Artificial Sequence

<220>
<223> oligonucleotide 6-mer sequences

<400> 582
aggtac                                                                  6

<210> 583
<211> 6
<212> DNA
<213> Artificial Sequence

<220>
<223> oligonucleotide 6-mer sequences

<400> 583
tagtac                                                                 6

<210> 584
<211> 6
<212> DNA
<213> Artificial Sequence

<220>
<223> oligonucleotide 6-mer sequences

<400> 584
gagtac                                                                 6

<210> 585
<211> 6
<212> DNA
<213> Artificial Sequence

<220>
<223> oligonucleotide 6-mer sequences

<400> 585
cagtac                                                                 6

<210> 586
<211> 6
<212> DNA
<213> Artificial Sequence

<220>
<223> oligonucleotide 6-mer sequences

<400> 586
aagtac                                                                 6

<210> 587
<211> 6
<212> DNA
<213> Artificial Sequence

<220>
<223> oligonucleotide 6-mer sequences

<400> 587
ctctac                                                                 6

<210> 588
<211> 6
<212> DNA
<213> Artificial Sequence

<220>
<223> oligonucleotide 6-mer sequences

<400> 588
atctac                                                                 6

<210> 589
<211> 6
<212> DNA
<213> Artificial Sequence

<220>
<223> oligonucleotide 6-mer sequences

<400> 589
gcctac                                                                 6

<210> 590
<211> 6
<212> DNA
<213> Artificial Sequence

<220>
<223> oligonucleotide 6-mer sequences

<400> 590
ccctac                                                                 6

<210> 591
<211> 6
<212> DNA
<213> Artificial Sequence

<220>
<223> oligonucleotide 6-mer sequences

<400> 591
ttatac                                                                 6

<210> 592
<211> 6
<212> DNA
<213> Artificial Sequence

<220>
<223> oligonucleotide 6-mer sequences

<400> 592
gtatac                                                                 6

<210> 593
<211> 6
<212> DNA
<213> Artificial Sequence

<220>
<223> oligonucleotide 6-mer sequences

<400> 593
ggatac                                                                 6

<210> 594
<211> 6
<212> DNA
<213> Artificial Sequence

<220>
<223> oligonucleotide 6-mer sequences

<400> 594

cgatac                                                                 6

<210> 595
<211> 6
<212> DNA
<213> Artificial Sequence

<220>
<223> oligonucleotide 6-mer sequences

<400> 595
agatac                                                                 6

<210> 596
<211> 6
<212> DNA
<213> Artificial Sequence

<220>
<223> oligonucleotide 6-mer sequences

<400> 596
tcatac                                                                 6

<210> 597
<211> 6
<212> DNA
<213> Artificial Sequence

<220>
<223> oligonucleotide 6-mer sequences

<400> 597
gcatac                                                                 6

<210> 598
<211> 6
<212> DNA
<213> Artificial Sequence

<220>
<223> oligonucleotide 6-mer sequences

<400> 598
taatac                                                                 6

<210> 599
<211> 6
<212> DNA
<213> Artificial Sequence

<220>
<223> oligonucleotide 6-mer sequences

<400> 599
gaatac                                                                 6

<210> 600
<211> 6
<212> DNA
<213> Artificial Sequence

<220>
<223> oligonucleotide 6-mer sequences

<400> 600
aaatac                                                                 6

<210> 601
<211> 6
<212> DNA
<213> Artificial Sequence

<220>
<223> oligonucleotide 6-mer sequences

<400> 601
tgtgac                                                                 6

<210> 602
<211> 6
<212> DNA
<213> Artificial Sequence

<220>
<223> oligonucleotide 6-mer sequences

<400> 602
ggtgac                                                                 6

<210> 603
<211> 6
<212> DNA
<213> Artificial Sequence

<220>
<223> oligonucleotide 6-mer sequences

<400> 603
agtgac                                                                 6

<210> 604
<211> 6
<212> DNA
<213> Artificial Sequence

<220>
<223> oligonucleotide 6-mer sequences

<400> 604
cctgac                                                                 6

<210> 605
<211> 6
<212> DNA
<213> Artificial Sequence

<220>
<223> oligonucleotide 6-mer sequences

<400> 605
catgac                                                                 6

<210> 606
<211> 6
<212> DNA
<213> Artificial Sequence

<220>
<223> oligonucleotide 6-mer sequences

<400> 606
atggac                                                                  6

<210> 607
<211> 6
<212> DNA
<213> Artificial Sequence

<220>
<223> oligonucleotide 6-mer sequences

<400> 607
tgggac                                                                  6

<210> 608
<211> 6
<212> DNA
<213> Artificial Sequence

<220>
<223> oligonucleotide 6-mer sequences

<400> 608
gtcgac                                                                  6

<210> 609
<211> 6
<212> DNA
<213> Artificial Sequence

<220>
<223> oligonucleotide 6-mer sequences

<400> 609
atcgac                                                                  6

<210> 610
<211> 6
<212> DNA
<213> Artificial Sequence

<220>
<223> oligonucleotide 6-mer sequences

<400> 610
tgcgac                                                                  6

<210> 611
<211> 6
<212> DNA
<213> Artificial Sequence

<220>
<223> oligonucleotide 6-mer sequences

<400> 611
aacgac                                                                  6

<210> 612
<211> 6
<212> DNA
<213> Artificial Sequence

<220>

<223> oligonucleotide 6-mer sequences

<400> 612
ctagac                                                                  6

<210> 613
<211> 6
<212> DNA
<213> Artificial Sequence

<220>
<223> oligonucleotide 6-mer sequences

<400> 613
atagac                                                                  6

<210> 614
<211> 6
<212> DNA
<213> Artificial Sequence

<220>
<223> oligonucleotide 6-mer sequences

<400> 614
agagac                                                                  6

<210> 615
<211> 6
<212> DNA
<213> Artificial Sequence

<220>
<223> oligonucleotide 6-mer sequences

<400> 615
taagac                                                                  6

<210> 616
<211> 6
<212> DNA
<213> Artificial Sequence

<220>
<223> oligonucleotide 6-mer sequences

<400> 616
actcac                                                                  6

<210> 617
<211> 6
<212> DNA
<213> Artificial Sequence

<220>
<223> oligonucleotide 6-mer sequences

<400> 617
tatcac                                                                  6

<210> 618
<211> 6
<212> DNA
<213> Artificial Sequence

<220>
<223> oligonucleotide 6-mer sequences

<400> 618
gatcac                                                                  6

<210> 619
<211> 6
<212> DNA
<213> Artificial Sequence

<220>
<223> oligonucleotide 6-mer sequences

<400> 619
tcgcac                                                                  6

<210> 620
<211> 6
<212> DNA
<213> Artificial Sequence

<220>
<223> oligonucleotide 6-mer sequences

<400> 620
tgccac                                                                  6

<210> 621
<211> 6
<212> DNA
<213> Artificial Sequence

<220>
<223> oligonucleotide 6-mer sequences

<400> 621
aaccac                                                                  6

<210> 622
<211> 6
<212> DNA
<213> Artificial Sequence

<220>
<223> oligonucleotide 6-mer sequences

<400> 622
gtacac                                                                  6

<210> 623
<211> 6
<212> DNA
<213> Artificial Sequence

<220>
<223> oligonucleotide 6-mer sequences

<400> 623
agacac                                                                  6

<210> 624
<211> 6
<212> DNA
<213> Artificial Sequence

<220>
<223> oligonucleotide 6-mer sequences

<400> 624
aaacac                                                                 6

<210> 625
<211> 6
<212> DNA
<213> Artificial Sequence

<220>
<223> oligonucleotide 6-mer sequences

<400> 625
gttaac                                                                 6

<210> 626
<211> 6
<212> DNA
<213> Artificial Sequence

<220>
<223> oligonucleotide 6-mer sequences

<400> 626
tctaac                                                                 6

<210> 627
<211> 6
<212> DNA
<213> Artificial Sequence

<220>
<223> oligonucleotide 6-mer sequences

<400> 627
gctaac                                                                 6

<210> 628
<211> 6
<212> DNA
<213> Artificial Sequence

<220>
<223> oligonucleotide 6-mer sequences

<400> 628
tataac                                                                 6

<210> 629
<211> 6
<212> DNA
<213> Artificial Sequence

<220>
<223> oligonucleotide 6-mer sequences

<400> 629
ccgaac                                                                 6

<210> 630
<211> 6
<212> DNA

<213> Artificial Sequence

<220>
<223> oligonucleotide 6-mer sequences

<400> 630
cagaac                                                                  6

<210> 631
<211> 6
<212> DNA
<213> Artificial Sequence

<220>
<223> oligonucleotide 6-mer sequences

<400> 631
cgcaac                                                                  6

<210> 632
<211> 6
<212> DNA
<213> Artificial Sequence

<220>
<223> oligonucleotide 6-mer sequences

<400> 632
cccaac                                                                  6

<210> 633
<211> 6
<212> DNA
<213> Artificial Sequence

<220>
<223> oligonucleotide 6-mer sequences

<400> 633
tacaac                                                                  6

<210> 634
<211> 6
<212> DNA
<213> Artificial Sequence

<220>
<223> oligonucleotide 6-mer sequences

<400> 634
cacaac                                                                  6

<210> 635
<211> 6
<212> DNA
<213> Artificial Sequence

<220>
<223> oligonucleotide 6-mer sequences

<400> 635
ataaac                                                                  6

<210> 636
<211> 6

<212> DNA
<213> Artificial Sequence

<220>
<223> oligonucleotide 6-mer sequences

<400> 636
tgaaac                                                                  6

<210> 637
<211> 6
<212> DNA
<213> Artificial Sequence

<220>
<223> oligonucleotide 6-mer sequences

<400> 637
agaaac                                                                  6

<210> 638
<211> 6
<212> DNA
<213> Artificial Sequence

<220>
<223> oligonucleotide 6-mer sequences

<400> 638
gcaaac                                                                  6

<210> 639
<211> 6
<212> DNA
<213> Artificial Sequence

<220>
<223> oligonucleotide 6-mer sequences

<400> 639
ccaaac                                                                  6

<210> 640
<211> 6
<212> DNA
<213> Artificial Sequence

<220>
<223> oligonucleotide 6-mer sequences

<400> 640
atttta                                                                  6

<210> 641
<211> 6
<212> DNA
<213> Artificial Sequence

<220>
<223> oligonucleotide 6-mer sequences

<400> 641
tgttta                                                                  6

<210> 642

<211> 6
<212> DNA
<213> Artificial Sequence

<220>
<223> oligonucleotide 6-mer sequences

<400> 642
aattta                                                                 6

<210> 643
<211> 6
<212> DNA
<213> Artificial Sequence

<220>
<223> oligonucleotide 6-mer sequences

<400> 643
atgtta                                                                 6

<210> 644
<211> 6
<212> DNA
<213> Artificial Sequence

<220>
<223> oligonucleotide 6-mer sequences

<400> 644
cggtta                                                                 6

<210> 645
<211> 6
<212> DNA
<213> Artificial Sequence

<220>
<223> oligonucleotide 6-mer sequences

<400> 645
acgtta                                                                 6

<210> 646
<211> 6
<212> DNA
<213> Artificial Sequence

<220>
<223> oligonucleotide 6-mer sequences

<400> 646
gagtta                                                                 6

<210> 647
<211> 6
<212> DNA
<213> Artificial Sequence

<220>
<223> oligonucleotide 6-mer sequences

<400> 647
gtctta                                                                 6

<210> 648
<211> 6
<212> DNA
<213> Artificial Sequence

<220>
<223> oligonucleotide 6-mer sequences

<400> 648
atctta                                                                 6

<210> 649
<211> 6
<212> DNA
<213> Artificial Sequence

<220>
<223> oligonucleotide 6-mer sequences

<400> 649
cgctta                                                                 6

<210> 650
<211> 6
<212> DNA
<213> Artificial Sequence

<220>
<223> oligonucleotide 6-mer sequences

<400> 650
aactta                                                                 6

<210> 651
<211> 6
<212> DNA
<213> Artificial Sequence

<220>
<223> oligonucleotide 6-mer sequences

<400> 651
atatta                                                                 6

<210> 652
<211> 6
<212> DNA
<213> Artificial Sequence

<220>
<223> oligonucleotide 6-mer sequences

<400> 652
agatta                                                                 6

<210> 653
<211> 6
<212> DNA
<213> Artificial Sequence

<220>
<223> oligonucleotide 6-mer sequences

<400> 653
tcatta                                                                 6

<210> 654
<211> 6
<212> DNA
<213> Artificial Sequence

<220>
<223> oligonucleotide 6-mer sequences

<400> 654
acatta                                                                  6

<210> 655
<211> 6
<212> DNA
<213> Artificial Sequence

<220>
<223> oligonucleotide 6-mer sequences

<400> 655
gttgta                                                                  6

<210> 656
<211> 6
<212> DNA
<213> Artificial Sequence

<220>
<223> oligonucleotide 6-mer sequences

<400> 656
cttgta                                                                  6

<210> 657
<211> 6
<212> DNA
<213> Artificial Sequence

<220>
<223> oligonucleotide 6-mer sequences

<400> 657
attgta                                                                  6

<210> 658
<211> 6
<212> DNA
<213> Artificial Sequence

<220>
<223> oligonucleotide 6-mer sequences

<400> 658
cgtgta                                                                  6

<210> 659
<211> 6
<212> DNA
<213> Artificial Sequence

<220>
<223> oligonucleotide 6-mer sequences

<400> 659

tatgta                                                                 6

<210> 660
<211> 6
<212> DNA
<213> Artificial Sequence

<220>
<223> oligonucleotide 6-mer sequences

<400> 660
gatgta                                                                 6

<210> 661
<211> 6
<212> DNA
<213> Artificial Sequence

<220>
<223> oligonucleotide 6-mer sequences

<400> 661
aatgta                                                                 6

<210> 662
<211> 6
<212> DNA
<213> Artificial Sequence

<220>
<223> oligonucleotide 6-mer sequences

<400> 662
ttggta                                                                 6

<210> 663
<211> 6
<212> DNA
<213> Artificial Sequence

<220>
<223> oligonucleotide 6-mer sequences

<400> 663
ctggta                                                                 6

<210> 664
<211> 6
<212> DNA
<213> Artificial Sequence

<220>
<223> oligonucleotide 6-mer sequences

<400> 664
gaggta                                                                 6

<210> 665
<211> 6
<212> DNA
<213> Artificial Sequence

<220>
<223> oligonucleotide 6-mer sequences

<400> 665
aaggta                                                                    6

<210> 666
<211> 6
<212> DNA
<213> Artificial Sequence

<220>
<223> oligonucleotide 6-mer sequences

<400> 666
gtcgta                                                                    6

<210> 667
<211> 6
<212> DNA
<213> Artificial Sequence

<220>
<223> oligonucleotide 6-mer sequences

<400> 667
atcgta                                                                    6

<210> 668
<211> 6
<212> DNA
<213> Artificial Sequence

<220>
<223> oligonucleotide 6-mer sequences

<400> 668
agcgta                                                                    6

<210> 669
<211> 6
<212> DNA
<213> Artificial Sequence

<220>
<223> oligonucleotide 6-mer sequences

<400> 669
cccgta                                                                    6

<210> 670
<211> 6
<212> DNA
<213> Artificial Sequence

<220>
<223> oligonucleotide 6-mer sequences

<400> 670
accgta                                                                    6

<210> 671
<211> 6
<212> DNA
<213> Artificial Sequence

<220>
<223> oligonucleotide 6-mer sequences

<400> 671
gacgta                                                                 6

<210> 672
<211> 6
<212> DNA
<213> Artificial Sequence

<220>
<223> oligonucleotide 6-mer sequences

<400> 672
cacgta                                                                 6

<210> 673
<211> 6
<212> DNA
<213> Artificial Sequence

<220>
<223> oligonucleotide 6-mer sequences

<400> 673
aacgta                                                                 6

<210> 674
<211> 6
<212> DNA
<213> Artificial Sequence

<220>
<223> oligonucleotide 6-mer sequences

<400> 674
ctagta                                                                 6

<210> 675
<211> 6
<212> DNA
<213> Artificial Sequence

<220>
<223> oligonucleotide 6-mer sequences

<400> 675
tgagta                                                                 6

<210> 676
<211> 6
<212> DNA
<213> Artificial Sequence

<220>
<223> oligonucleotide 6-mer sequences

<400> 676
ggagta                                                                 6

<210> 677
<211> 6
<212> DNA
<213> Artificial Sequence

<220>

<223> oligonucleotide 6-mer sequences

<400> 677
cgagta                                                                  6

<210> 678
<211> 6
<212> DNA
<213> Artificial Sequence

<220>
<223> oligonucleotide 6-mer sequences

<400> 678
acagta                                                                  6

<210> 679
<211> 6
<212> DNA
<213> Artificial Sequence

<220>
<223> oligonucleotide 6-mer sequences

<400> 679
taagta                                                                  6

<210> 680
<211> 6
<212> DNA
<213> Artificial Sequence

<220>
<223> oligonucleotide 6-mer sequences

<400> 680
tttcta                                                                  6

<210> 681
<211> 6
<212> DNA
<213> Artificial Sequence

<220>
<223> oligonucleotide 6-mer sequences

<400> 681
cttcta                                                                  6

<210> 682
<211> 6
<212> DNA
<213> Artificial Sequence

<220>
<223> oligonucleotide 6-mer sequences

<400> 682
agtcta                                                                  6

<210> 683
<211> 6
<212> DNA
<213> Artificial Sequence

<220>
<223> oligonucleotide 6-mer sequences

<400> 683
tctcta                                                                 6

<210> 684
<211> 6
<212> DNA
<213> Artificial Sequence

<220>
<223> oligonucleotide 6-mer sequences

<400> 684
gatcta                                                                 6

<210> 685
<211> 6
<212> DNA
<213> Artificial Sequence

<220>
<223> oligonucleotide 6-mer sequences

<400> 685
gtgcta                                                                 6

<210> 686
<211> 6
<212> DNA
<213> Artificial Sequence

<220>
<223> oligonucleotide 6-mer sequences

<400> 686
aggcta                                                                 6

<210> 687
<211> 6
<212> DNA
<213> Artificial Sequence

<220>
<223> oligonucleotide 6-mer sequences

<400> 687
gcgcta                                                                 6

<210> 688
<211> 6
<212> DNA
<213> Artificial Sequence

<220>
<223> oligonucleotide 6-mer sequences

<400> 688
tagcta                                                                 6

<210> 689
<211> 6
<212> DNA
<213> Artificial Sequence

<220>
<223> oligonucleotide 6-mer sequences

<400> 689
aagcta                                                                  6

<210> 690
<211> 6
<212> DNA
<213> Artificial Sequence

<220>
<223> oligonucleotide 6-mer sequences

<400> 690
atccta                                                                  6

<210> 691
<211> 6
<212> DNA
<213> Artificial Sequence

<220>
<223> oligonucleotide 6-mer sequences

<400> 691
tgccta                                                                  6

<210> 692
<211> 6
<212> DNA
<213> Artificial Sequence

<220>
<223> oligonucleotide 6-mer sequences

<400> 692
ggccta                                                                  6

<210> 693
<211> 6
<212> DNA
<213> Artificial Sequence

<220>
<223> oligonucleotide 6-mer sequences

<400> 693
cgccta                                                                  6

<210> 694
<211> 6
<212> DNA
<213> Artificial Sequence

<220>
<223> oligonucleotide 6-mer sequences

<400> 694
acccta                                                                  6

<210> 695
<211> 6
<212> DNA

<213> Artificial Sequence

<220>
<223> oligonucleotide 6-mer sequences

<400> 695
gaccta                                                                 6

<210> 696
<211> 6
<212> DNA
<213> Artificial Sequence

<220>
<223> oligonucleotide 6-mer sequences

<400> 696
aaccta                                                                 6

<210> 697
<211> 6
<212> DNA
<213> Artificial Sequence

<220>
<223> oligonucleotide 6-mer sequences

<400> 697
ttacta                                                                 6

<210> 698
<211> 6
<212> DNA
<213> Artificial Sequence

<220>
<223> oligonucleotide 6-mer sequences

<400> 698
gtacta                                                                 6

<210> 699
<211> 6
<212> DNA
<213> Artificial Sequence

<220>
<223> oligonucleotide 6-mer sequences

<400> 699
atacta                                                                 6

<210> 700
<211> 6
<212> DNA
<213> Artificial Sequence

<220>
<223> oligonucleotide 6-mer sequences

<400> 700
gcacta                                                                 6

<210> 701
<211> 6

<212> DNA
<213> Artificial Sequence

<220>
<223> oligonucleotide 6-mer sequences

<400> 701
acacta                                                                  6

<210> 702
<211> 6
<212> DNA
<213> Artificial Sequence

<220>
<223> oligonucleotide 6-mer sequences

<400> 702
tttata                                                                  6

<210> 703
<211> 6
<212> DNA
<213> Artificial Sequence

<220>
<223> oligonucleotide 6-mer sequences

<400> 703
gttata                                                                  6

<210> 704
<211> 6
<212> DNA
<213> Artificial Sequence

<220>
<223> oligonucleotide 6-mer sequences

<400> 704
cttata                                                                  6

<210> 705
<211> 6
<212> DNA
<213> Artificial Sequence

<220>
<223> oligonucleotide 6-mer sequences

<400> 705
attata                                                                  6

<210> 706
<211> 6
<212> DNA
<213> Artificial Sequence

<220>
<223> oligonucleotide 6-mer sequences

<400> 706
tgtata                                                                  6

<210> 707

<211> 6
<212> DNA
<213> Artificial Sequence

<220>
<223> oligonucleotide 6-mer sequences

<400> 707
ggtata                                                                  6

<210> 708
<211> 6
<212> DNA
<213> Artificial Sequence

<220>
<223> oligonucleotide 6-mer sequences

<400> 708
cgtata                                                                  6

<210> 709
<211> 6
<212> DNA
<213> Artificial Sequence

<220>
<223> oligonucleotide 6-mer sequences

<400> 709
agtata                                                                  6

<210> 710
<211> 6
<212> DNA
<213> Artificial Sequence

<220>
<223> oligonucleotide 6-mer sequences

<400> 710
gctata                                                                  6

<210> 711
<211> 6
<212> DNA
<213> Artificial Sequence

<220>
<223> oligonucleotide 6-mer sequences

<400> 711
actata                                                                  6

<210> 712
<211> 6
<212> DNA
<213> Artificial Sequence

<220>
<223> oligonucleotide 6-mer sequences

<400> 712
tatata                                                                  6

<210> 713
<211> 6
<212> DNA
<213> Artificial Sequence

<220>
<223> oligonucleotide 6-mer sequences

<400> 713
gatata                                                                 6

<210> 714
<211> 6
<212> DNA
<213> Artificial Sequence

<220>
<223> oligonucleotide 6-mer sequences

<400> 714
catata                                                                 6

<210> 715
<211> 6
<212> DNA
<213> Artificial Sequence

<220>
<223> oligonucleotide 6-mer sequences

<400> 715
gggata                                                                 6

<210> 716
<211> 6
<212> DNA
<213> Artificial Sequence

<220>
<223> oligonucleotide 6-mer sequences

<400> 716
aggata                                                                 6

<210> 717
<211> 6
<212> DNA
<213> Artificial Sequence

<220>
<223> oligonucleotide 6-mer sequences

<400> 717
tcgata                                                                 6

<210> 718
<211> 6
<212> DNA
<213> Artificial Sequence

<220>
<223> oligonucleotide 6-mer sequences

<400> 718
gcgata                                                                 6

<210> 719
<211> 6
<212> DNA
<213> Artificial Sequence

<220>
<223> oligonucleotide 6-mer sequences

<400> 719
ccgata                                                                  6

<210> 720
<211> 6
<212> DNA
<213> Artificial Sequence

<220>
<223> oligonucleotide 6-mer sequences

<400> 720
acgata                                                                  6

<210> 721
<211> 6
<212> DNA
<213> Artificial Sequence

<220>
<223> oligonucleotide 6-mer sequences

<400> 721
gagata                                                                  6

<210> 722
<211> 6
<212> DNA
<213> Artificial Sequence

<220>
<223> oligonucleotide 6-mer sequences

<400> 722
cagata                                                                  6

<210> 723
<211> 6
<212> DNA
<213> Artificial Sequence

<220>
<223> oligonucleotide 6-mer sequences

<400> 723
aagata                                                                  6

<210> 724
<211> 6
<212> DNA
<213> Artificial Sequence

<220>
<223> oligonucleotide 6-mer sequences

<400> 724

ctcata                                                                  6

<210> 725
<211> 6
<212> DNA
<213> Artificial Sequence

<220>
<223> oligonucleotide 6-mer sequences

<400> 725
atcata                                                                  6

<210> 726
<211> 6
<212> DNA
<213> Artificial Sequence

<220>
<223> oligonucleotide 6-mer sequences

<400> 726
tgcata                                                                  6

<210> 727
<211> 6
<212> DNA
<213> Artificial Sequence

<220>
<223> oligonucleotide 6-mer sequences

<400> 727
cgcata                                                                  6

<210> 728
<211> 6
<212> DNA
<213> Artificial Sequence

<220>
<223> oligonucleotide 6-mer sequences

<400> 728
tccata                                                                  6

<210> 729
<211> 6
<212> DNA
<213> Artificial Sequence

<220>
<223> oligonucleotide 6-mer sequences

<400> 729
tacata                                                                  6

<210> 730
<211> 6
<212> DNA
<213> Artificial Sequence

<220>
<223> oligonucleotide 6-mer sequences

<400> 730
gacata                                                                 6

<210> 731
<211> 6
<212> DNA
<213> Artificial Sequence

<220>
<223> oligonucleotide 6-mer sequences

<400> 731
aacata                                                                 6

<210> 732
<211> 6
<212> DNA
<213> Artificial Sequence

<220>
<223> oligonucleotide 6-mer sequences

<400> 732
gtaata                                                                 6

<210> 733
<211> 6
<212> DNA
<213> Artificial Sequence

<220>
<223> oligonucleotide 6-mer sequences

<400> 733
cgaata                                                                 6

<210> 734
<211> 6
<212> DNA
<213> Artificial Sequence

<220>
<223> oligonucleotide 6-mer sequences

<400> 734
ccaata                                                                 6

<210> 735
<211> 6
<212> DNA
<213> Artificial Sequence

<220>
<223> oligonucleotide 6-mer sequences

<400> 735
acaata                                                                 6

<210> 736
<211> 6
<212> DNA
<213> Artificial Sequence

<220>
<223> oligonucleotide 6-mer sequences

<400> 736
taaata                                                                 6

<210> 737
<211> 6
<212> DNA
<213> Artificial Sequence

<220>
<223> oligonucleotide 6-mer sequences

<400> 737
caaata                                                                 6

<210> 738
<211> 6
<212> DNA
<213> Artificial Sequence

<220>
<223> oligonucleotide 6-mer sequences

<400> 738
cgttga                                                                 6

<210> 739
<211> 6
<212> DNA
<213> Artificial Sequence

<220>
<223> oligonucleotide 6-mer sequences

<400> 739
acttga                                                                 6

<210> 740
<211> 6
<212> DNA
<213> Artificial Sequence

<220>
<223> oligonucleotide 6-mer sequences

<400> 740
gattga                                                                 6

<210> 741
<211> 6
<212> DNA
<213> Artificial Sequence

<220>
<223> oligonucleotide 6-mer sequences

<400> 741
aattga                                                                 6

<210> 742
<211> 6
<212> DNA
<213> Artificial Sequence

<220>

<223> oligonucleotide 6-mer sequences

<400> 742
gtgtga                                                                  6

<210> 743
<211> 6
<212> DNA
<213> Artificial Sequence

<220>
<223> oligonucleotide 6-mer sequences

<400> 743
atgtga                                                                  6

<210> 744
<211> 6
<212> DNA
<213> Artificial Sequence

<220>
<223> oligonucleotide 6-mer sequences

<400> 744
tggtga                                                                  6

<210> 745
<211> 6
<212> DNA
<213> Artificial Sequence

<220>
<223> oligonucleotide 6-mer sequences

<400> 745
cggtga                                                                  6

<210> 746
<211> 6
<212> DNA
<213> Artificial Sequence

<220>
<223> oligonucleotide 6-mer sequences

<400> 746
ccgtga                                                                  6

<210> 747
<211> 6
<212> DNA
<213> Artificial Sequence

<220>
<223> oligonucleotide 6-mer sequences

<400> 747
acgtga                                                                  6

<210> 748
<211> 6
<212> DNA
<213> Artificial Sequence

<220>
<223> oligonucleotide 6-mer sequences

<400> 748
gagtga                                                                  6

<210> 749
<211> 6
<212> DNA
<213> Artificial Sequence

<220>
<223> oligonucleotide 6-mer sequences

<400> 749
tgctga                                                                  6

<210> 750
<211> 6
<212> DNA
<213> Artificial Sequence

<220>
<223> oligonucleotide 6-mer sequences

<400> 750
acctga                                                                  6

<210> 751
<211> 6
<212> DNA
<213> Artificial Sequence

<220>
<223> oligonucleotide 6-mer sequences

<400> 751
cactga                                                                  6

<210> 752
<211> 6
<212> DNA
<213> Artificial Sequence

<220>
<223> oligonucleotide 6-mer sequences

<400> 752
gtatga                                                                  6

<210> 753
<211> 6
<212> DNA
<213> Artificial Sequence

<220>
<223> oligonucleotide 6-mer sequences

<400> 753
atatga                                                                  6

<210> 754
<211> 6
<212> DNA
<213> Artificial Sequence

<220>
<223> oligonucleotide 6-mer sequences

<400> 754
ggatga                                                                  6

<210> 755
<211> 6
<212> DNA
<213> Artificial Sequence

<220>
<223> oligonucleotide 6-mer sequences

<400> 755
cgatga                                                                  6

<210> 756
<211> 6
<212> DNA
<213> Artificial Sequence

<220>
<223> oligonucleotide 6-mer sequences

<400> 756
tcatga                                                                  6

<210> 757
<211> 6
<212> DNA
<213> Artificial Sequence

<220>
<223> oligonucleotide 6-mer sequences

<400> 757
gcatga                                                                  6

<210> 758
<211> 6
<212> DNA
<213> Artificial Sequence

<220>
<223> oligonucleotide 6-mer sequences

<400> 758
acatga                                                                  6

<210> 759
<211> 6
<212> DNA
<213> Artificial Sequence

<220>
<223> oligonucleotide 6-mer sequences

<400> 759
gaatga                                                                  6

<210> 760
<211> 6
<212> DNA

<213> Artificial Sequence

<220>
<223> oligonucleotide 6-mer sequences

<400> 760
tgtgga                                                                 6

<210> 761
<211> 6
<212> DNA
<213> Artificial Sequence

<220>
<223> oligonucleotide 6-mer sequences

<400> 761
cctgga                                                                 6

<210> 762
<211> 6
<212> DNA
<213> Artificial Sequence

<220>
<223> oligonucleotide 6-mer sequences

<400> 762
tatgga                                                                 6

<210> 763
<211> 6
<212> DNA
<213> Artificial Sequence

<220>
<223> oligonucleotide 6-mer sequences

<400> 763
ctggga                                                                 6

<210> 764
<211> 6
<212> DNA
<213> Artificial Sequence

<220>
<223> oligonucleotide 6-mer sequences

<400> 764
ctcgga                                                                 6

<210> 765
<211> 6
<212> DNA
<213> Artificial Sequence

<220>
<223> oligonucleotide 6-mer sequences

<400> 765
tgagga                                                                 6

<210> 766
<211> 6

<212> DNA
<213> Artificial Sequence

<220>
<223> oligonucleotide 6-mer sequences

<400> 766
taagga                                                                  6

<210> 767
<211> 6
<212> DNA
<213> Artificial Sequence

<220>
<223> oligonucleotide 6-mer sequences

<400> 767
attcga                                                                  6

<210> 768
<211> 6
<212> DNA
<213> Artificial Sequence

<220>
<223> oligonucleotide 6-mer sequences

<400> 768
cgtcga                                                                  6

<210> 769
<211> 6
<212> DNA
<213> Artificial Sequence

<220>
<223> oligonucleotide 6-mer sequences

<400> 769
agtcga                                                                  6

<210> 770
<211> 6
<212> DNA
<213> Artificial Sequence

<220>
<223> oligonucleotide 6-mer sequences

<400> 770
gctcga                                                                  6

<210> 771
<211> 6
<212> DNA
<213> Artificial Sequence

<220>
<223> oligonucleotide 6-mer sequences

<400> 771
actcga                                                                  6

<210> 772

<211> 6
<212> DNA
<213> Artificial Sequence

<220>
<223> oligonucleotide 6-mer sequences

<400> 772
tatcga                                                                 6

<210> 773
<211> 6
<212> DNA
<213> Artificial Sequence

<220>
<223> oligonucleotide 6-mer sequences

<400> 773
gatcga                                                                 6

<210> 774
<211> 6
<212> DNA
<213> Artificial Sequence

<220>
<223> oligonucleotide 6-mer sequences

<400> 774
ttgcga                                                                 6

<210> 775
<211> 6
<212> DNA
<213> Artificial Sequence

<220>
<223> oligonucleotide 6-mer sequences

<400> 775
atgcga                                                                 6

<210> 776
<211> 6
<212> DNA
<213> Artificial Sequence

<220>
<223> oligonucleotide 6-mer sequences

<400> 776
acgcga                                                                 6

<210> 777
<211> 6
<212> DNA
<213> Artificial Sequence

<220>
<223> oligonucleotide 6-mer sequences

<400> 777
gtccga                                                                 6

<210> 778
<211> 6
<212> DNA
<213> Artificial Sequence

<220>
<223> oligonucleotide 6-mer sequences

<400> 778
taccga                                                                 6

<210> 779
<211> 6
<212> DNA
<213> Artificial Sequence

<220>
<223> oligonucleotide 6-mer sequences

<400> 779
cgacga                                                                 6

<210> 780
<211> 6
<212> DNA
<213> Artificial Sequence

<220>
<223> oligonucleotide 6-mer sequences

<400> 780
agacga                                                                 6

<210> 781
<211> 6
<212> DNA
<213> Artificial Sequence

<220>
<223> oligonucleotide 6-mer sequences

<400> 781
gcacga                                                                 6

<210> 782
<211> 6
<212> DNA
<213> Artificial Sequence

<220>
<223> oligonucleotide 6-mer sequences

<400> 782
acacga                                                                 6

<210> 783
<211> 6
<212> DNA
<213> Artificial Sequence

<220>
<223> oligonucleotide 6-mer sequences

<400> 783
taacga                                                                 6

<210> 784
<211> 6
<212> DNA
<213> Artificial Sequence

<220>
<223> oligonucleotide 6-mer sequences

<400> 784
gaacga                                                                  6

<210> 785
<211> 6
<212> DNA
<213> Artificial Sequence

<220>
<223> oligonucleotide 6-mer sequences

<400> 785
caacga                                                                  6

<210> 786
<211> 6
<212> DNA
<213> Artificial Sequence

<220>
<223> oligonucleotide 6-mer sequences

<400> 786
tttaga                                                                  6

<210> 787
<211> 6
<212> DNA
<213> Artificial Sequence

<220>
<223> oligonucleotide 6-mer sequences

<400> 787
tgtaga                                                                  6

<210> 788
<211> 6
<212> DNA
<213> Artificial Sequence

<220>
<223> oligonucleotide 6-mer sequences

<400> 788
cgtaga                                                                  6

<210> 789
<211> 6
<212> DNA
<213> Artificial Sequence

<220>
<223> oligonucleotide 6-mer sequences

<400> 789

agtaga                                                                  6

<210> 790
<211> 6
<212> DNA
<213> Artificial Sequence

<220>
<223> oligonucleotide 6-mer sequences

<400> 790
gctaga                                                                  6

<210> 791
<211> 6
<212> DNA
<213> Artificial Sequence

<220>
<223> oligonucleotide 6-mer sequences

<400> 791
cctaga                                                                  6

<210> 792
<211> 6
<212> DNA
<213> Artificial Sequence

<220>
<223> oligonucleotide 6-mer sequences

<400> 792
tataga                                                                  6

<210> 793
<211> 6
<212> DNA
<213> Artificial Sequence

<220>
<223> oligonucleotide 6-mer sequences

<400> 793
gataga                                                                  6

<210> 794
<211> 6
<212> DNA
<213> Artificial Sequence

<220>
<223> oligonucleotide 6-mer sequences

<400> 794
cataga                                                                  6

<210> 795
<211> 6
<212> DNA
<213> Artificial Sequence

<220>
<223> oligonucleotide 6-mer sequences

<400> 795
gtgaga                                                                 6

<210> 796
<211> 6
<212> DNA
<213> Artificial Sequence

<220>
<223> oligonucleotide 6-mer sequences

<400> 796
atgaga                                                                 6

<210> 797
<211> 6
<212> DNA
<213> Artificial Sequence

<220>
<223> oligonucleotide 6-mer sequences

<400> 797
acgaga                                                                 6

<210> 798
<211> 6
<212> DNA
<213> Artificial Sequence

<220>
<223> oligonucleotide 6-mer sequences

<400> 798
tagaga                                                                 6

<210> 799
<211> 6
<212> DNA
<213> Artificial Sequence

<220>
<223> oligonucleotide 6-mer sequences

<400> 799
cagaga                                                                 6

<210> 800
<211> 6
<212> DNA
<213> Artificial Sequence

<220>
<223> oligonucleotide 6-mer sequences

<400> 800
cgcaga                                                                 6

<210> 801
<211> 6
<212> DNA
<213> Artificial Sequence

<220>
<223> oligonucleotide 6-mer sequences

<400> 801
tacaga                                                                 6

<210> 802
<211> 6
<212> DNA
<213> Artificial Sequence

<220>
<223> oligonucleotide 6-mer sequences

<400> 802
aacaga                                                                 6

<210> 803
<211> 6
<212> DNA
<213> Artificial Sequence

<220>
<223> oligonucleotide 6-mer sequences

<400> 803
gtaaga                                                                 6

<210> 804
<211> 6
<212> DNA
<213> Artificial Sequence

<220>
<223> oligonucleotide 6-mer sequences

<400> 804
ataaga                                                                 6

<210> 805
<211> 6
<212> DNA
<213> Artificial Sequence

<220>
<223> oligonucleotide 6-mer sequences

<400> 805
tgaaga                                                                 6

<210> 806
<211> 6
<212> DNA
<213> Artificial Sequence

<220>
<223> oligonucleotide 6-mer sequences

<400> 806
cgaaga                                                                 6

<210> 807
<211> 6
<212> DNA
<213> Artificial Sequence

<220>

<223> oligonucleotide 6-mer sequences

<400> 807
acaaga                                                                  6

<210> 808
<211> 6
<212> DNA
<213> Artificial Sequence

<220>
<223> oligonucleotide 6-mer sequences

<400> 808
taaaga                                                                  6

<210> 809
<211> 6
<212> DNA
<213> Artificial Sequence

<220>
<223> oligonucleotide 6-mer sequences

<400> 809
gattca                                                                  6

<210> 810
<211> 6
<212> DNA
<213> Artificial Sequence

<220>
<223> oligonucleotide 6-mer sequences

<400> 810
ccctca                                                                  6

<210> 811
<211> 6
<212> DNA
<213> Artificial Sequence

<220>
<223> oligonucleotide 6-mer sequences

<400> 811
tactca                                                                  6

<210> 812
<211> 6
<212> DNA
<213> Artificial Sequence

<220>
<223> oligonucleotide 6-mer sequences

<400> 812
aactca                                                                  6

<210> 813
<211> 6
<212> DNA
<213> Artificial Sequence

<220>
<223> oligonucleotide 6-mer sequences

<400> 813
gtatca                                                                  6

<210> 814
<211> 6
<212> DNA
<213> Artificial Sequence

<220>
<223> oligonucleotide 6-mer sequences

<400> 814
tgatca                                                                  6

<210> 815
<211> 6
<212> DNA
<213> Artificial Sequence

<220>
<223> oligonucleotide 6-mer sequences

<400> 815
caatca                                                                  6

<210> 816
<211> 6
<212> DNA
<213> Artificial Sequence

<220>
<223> oligonucleotide 6-mer sequences

<400> 816
gttgca                                                                  6

<210> 817
<211> 6
<212> DNA
<213> Artificial Sequence

<220>
<223> oligonucleotide 6-mer sequences

<400> 817
tgtgca                                                                  6

<210> 818
<211> 6
<212> DNA
<213> Artificial Sequence

<220>
<223> oligonucleotide 6-mer sequences

<400> 818
tatgca                                                                  6

<210> 819
<211> 6
<212> DNA
<213> Artificial Sequence

<220>
<223> oligonucleotide 6-mer sequences

<400> 819
ccggca                                                                 6

<210> 820
<211> 6
<212> DNA
<213> Artificial Sequence

<220>
<223> oligonucleotide 6-mer sequences

<400> 820
acggca                                                                 6

<210> 821
<211> 6
<212> DNA
<213> Artificial Sequence

<220>
<223> oligonucleotide 6-mer sequences

<400> 821
gtcgca                                                                 6

<210> 822
<211> 6
<212> DNA
<213> Artificial Sequence

<220>
<223> oligonucleotide 6-mer sequences

<400> 822
tgcgca                                                                 6

<210> 823
<211> 6
<212> DNA
<213> Artificial Sequence

<220>
<223> oligonucleotide 6-mer sequences

<400> 823
agcgca                                                                 6

<210> 824
<211> 6
<212> DNA
<213> Artificial Sequence

<220>
<223> oligonucleotide 6-mer sequences

<400> 824
tacgca                                                                 6

<210> 825
<211> 6
<212> DNA

<213> Artificial Sequence

<220>
<223> oligonucleotide 6-mer sequences

<400> 825
gtagca                                                                  6

<210> 826
<211> 6
<212> DNA
<213> Artificial Sequence

<220>
<223> oligonucleotide 6-mer sequences

<400> 826
atagca                                                                  6

<210> 827
<211> 6
<212> DNA
<213> Artificial Sequence

<220>
<223> oligonucleotide 6-mer sequences

<400> 827
ggagca                                                                  6

<210> 828
<211> 6
<212> DNA
<213> Artificial Sequence

<220>
<223> oligonucleotide 6-mer sequences

<400> 828
taagca                                                                  6

<210> 829
<211> 6
<212> DNA
<213> Artificial Sequence

<220>
<223> oligonucleotide 6-mer sequences

<400> 829
aaagca                                                                  6

<210> 830
<211> 6
<212> DNA
<213> Artificial Sequence

<220>
<223> oligonucleotide 6-mer sequences

<400> 830
gtccca                                                                  6

<210> 831
<211> 6

<212> DNA
<213> Artificial Sequence

<220>
<223> oligonucleotide 6-mer sequences

<400> 831
agccca                                                                 6

<210> 832
<211> 6
<212> DNA
<213> Artificial Sequence

<220>
<223> oligonucleotide 6-mer sequences

<400> 832
gtacca                                                                 6

<210> 833
<211> 6
<212> DNA
<213> Artificial Sequence

<220>
<223> oligonucleotide 6-mer sequences

<400> 833
atacca                                                                 6

<210> 834
<211> 6
<212> DNA
<213> Artificial Sequence

<220>
<223> oligonucleotide 6-mer sequences

<400> 834
tgacca                                                                 6

<210> 835
<211> 6
<212> DNA
<213> Artificial Sequence

<220>
<223> oligonucleotide 6-mer sequences

<400> 835
cttaca                                                                 6

<210> 836
<211> 6
<212> DNA
<213> Artificial Sequence

<220>
<223> oligonucleotide 6-mer sequences

<400> 836
ggtaca                                                                 6

<210> 837

<211> 6
<212> DNA
<213> Artificial Sequence

<220>
<223> oligonucleotide 6-mer sequences

<400> 837
cgtaca                                                                  6

<210> 838
<211> 6
<212> DNA
<213> Artificial Sequence

<220>
<223> oligonucleotide 6-mer sequences

<400> 838
agtaca                                                                  6

<210> 839
<211> 6
<212> DNA
<213> Artificial Sequence

<220>
<223> oligonucleotide 6-mer sequences

<400> 839
gctaca                                                                  6

<210> 840
<211> 6
<212> DNA
<213> Artificial Sequence

<220>
<223> oligonucleotide 6-mer sequences

<400> 840
actaca                                                                  6

<210> 841
<211> 6
<212> DNA
<213> Artificial Sequence

<220>
<223> oligonucleotide 6-mer sequences

<400> 841
tataca                                                                  6

<210> 842
<211> 6
<212> DNA
<213> Artificial Sequence

<220>
<223> oligonucleotide 6-mer sequences

<400> 842
gataca                                                                  6

<210> 843
<211> 6
<212> DNA
<213> Artificial Sequence

<220>
<223> oligonucleotide 6-mer sequences

<400> 843
aataca                                                                 6

<210> 844
<211> 6
<212> DNA
<213> Artificial Sequence

<220>
<223> oligonucleotide 6-mer sequences

<400> 844
gtgaca                                                                 6

<210> 845
<211> 6
<212> DNA
<213> Artificial Sequence

<220>
<223> oligonucleotide 6-mer sequences

<400> 845
atgaca                                                                 6

<210> 846
<211> 6
<212> DNA
<213> Artificial Sequence

<220>
<223> oligonucleotide 6-mer sequences

<400> 846
tcgaca                                                                 6

<210> 847
<211> 6
<212> DNA
<213> Artificial Sequence

<220>
<223> oligonucleotide 6-mer sequences

<400> 847
gcgaca                                                                 6

<210> 848
<211> 6
<212> DNA
<213> Artificial Sequence

<220>
<223> oligonucleotide 6-mer sequences

<400> 848
acgaca                                                                 6

<210> 849
<211> 6
<212> DNA
<213> Artificial Sequence

<220>
<223> oligonucleotide 6-mer sequences

<400> 849
aagaca                                                                  6

<210> 850
<211> 6
<212> DNA
<213> Artificial Sequence

<220>
<223> oligonucleotide 6-mer sequences

<400> 850
tgcaca                                                                  6

<210> 851
<211> 6
<212> DNA
<213> Artificial Sequence

<220>
<223> oligonucleotide 6-mer sequences

<400> 851
gacaca                                                                  6

<210> 852
<211> 6
<212> DNA
<213> Artificial Sequence

<220>
<223> oligonucleotide 6-mer sequences

<400> 852
ttaaca                                                                  6

<210> 853
<211> 6
<212> DNA
<213> Artificial Sequence

<220>
<223> oligonucleotide 6-mer sequences

<400> 853
cgaaca                                                                  6

<210> 854
<211> 6
<212> DNA
<213> Artificial Sequence

<220>
<223> oligonucleotide 6-mer sequences

<400> 854

agaaca                                                                  6

<210> 855
<211> 6
<212> DNA
<213> Artificial Sequence

<220>
<223> oligonucleotide 6-mer sequences

<400> 855
caaaca                                                                  6

<210> 856
<211> 6
<212> DNA
<213> Artificial Sequence

<220>
<223> oligonucleotide 6-mer sequences

<400> 856
gtttaa                                                                  6

<210> 857
<211> 6
<212> DNA
<213> Artificial Sequence

<220>
<223> oligonucleotide 6-mer sequences

<400> 857
tgttaa                                                                  6

<210> 858
<211> 6
<212> DNA
<213> Artificial Sequence

<220>
<223> oligonucleotide 6-mer sequences

<400> 858
ggttaa                                                                  6

<210> 859
<211> 6
<212> DNA
<213> Artificial Sequence

<220>
<223> oligonucleotide 6-mer sequences

<400> 859
agttaa                                                                  6

<210> 860
<211> 6
<212> DNA
<213> Artificial Sequence

<220>
<223> oligonucleotide 6-mer sequences

<400> 860
tattaa                                                                  6

<210> 861
<211> 6
<212> DNA
<213> Artificial Sequence

<220>
<223> oligonucleotide 6-mer sequences

<400> 861
cattaa                                                                  6

<210> 862
<211> 6
<212> DNA
<213> Artificial Sequence

<220>
<223> oligonucleotide 6-mer sequences

<400> 862
ttgtaa                                                                  6

<210> 863
<211> 6
<212> DNA
<213> Artificial Sequence

<220>
<223> oligonucleotide 6-mer sequences

<400> 863
gtgtaa                                                                  6

<210> 864
<211> 6
<212> DNA
<213> Artificial Sequence

<220>
<223> oligonucleotide 6-mer sequences

<400> 864
atgtaa                                                                  6

<210> 865
<211> 6
<212> DNA
<213> Artificial Sequence

<220>
<223> oligonucleotide 6-mer sequences

<400> 865
cggtaa                                                                  6

<210> 866
<211> 6
<212> DNA
<213> Artificial Sequence

<220>
<223> oligonucleotide 6-mer sequences

<400> 866
aggtaa                                                                    6

<210> 867
<211> 6
<212> DNA
<213> Artificial Sequence

<220>
<223> oligonucleotide 6-mer sequences

<400> 867
tcgtaa                                                                    6

<210> 868
<211> 6
<212> DNA
<213> Artificial Sequence

<220>
<223> oligonucleotide 6-mer sequences

<400> 868
ccgtaa                                                                    6

<210> 869
<211> 6
<212> DNA
<213> Artificial Sequence

<220>
<223> oligonucleotide 6-mer sequences

<400> 869
acgtaa                                                                    6

<210> 870
<211> 6
<212> DNA
<213> Artificial Sequence

<220>
<223> oligonucleotide 6-mer sequences

<400> 870
tagtaa                                                                    6

<210> 871
<211> 6
<212> DNA
<213> Artificial Sequence

<220>
<223> oligonucleotide 6-mer sequences

<400> 871
gagtaa                                                                    6

<210> 872
<211> 6
<212> DNA
<213> Artificial Sequence

<220>

<223> oligonucleotide 6-mer sequences

<400> 872
tgctaa                                                                  6

<210> 873
<211> 6
<212> DNA
<213> Artificial Sequence

<220>
<223> oligonucleotide 6-mer sequences

<400> 873
ggctaa                                                                  6

<210> 874
<211> 6
<212> DNA
<213> Artificial Sequence

<220>
<223> oligonucleotide 6-mer sequences

<400> 874
cgctaa                                                                  6

<210> 875
<211> 6
<212> DNA
<213> Artificial Sequence

<220>
<223> oligonucleotide 6-mer sequences

<400> 875
gcctaa                                                                  6

<210> 876
<211> 6
<212> DNA
<213> Artificial Sequence

<220>
<223> oligonucleotide 6-mer sequences

<400> 876
ccctaa                                                                  6

<210> 877
<211> 6
<212> DNA
<213> Artificial Sequence

<220>
<223> oligonucleotide 6-mer sequences

<400> 877
acctaa                                                                  6

<210> 878
<211> 6
<212> DNA
<213> Artificial Sequence

<220>
<223> oligonucleotide 6-mer sequences

<400> 878
tactaa                                                                 6

<210> 879
<211> 6
<212> DNA
<213> Artificial Sequence

<220>
<223> oligonucleotide 6-mer sequences

<400> 879
ttataa                                                                 6

<210> 880
<211> 6
<212> DNA
<213> Artificial Sequence

<220>
<223> oligonucleotide 6-mer sequences

<400> 880
gtataa                                                                 6

<210> 881
<211> 6
<212> DNA
<213> Artificial Sequence

<220>
<223> oligonucleotide 6-mer sequences

<400> 881
ctataa                                                                 6

<210> 882
<211> 6
<212> DNA
<213> Artificial Sequence

<220>
<223> oligonucleotide 6-mer sequences

<400> 882
atataa                                                                 6

<210> 883
<211> 6
<212> DNA
<213> Artificial Sequence

<220>
<223> oligonucleotide 6-mer sequences

<400> 883
cgataa                                                                 6

<210> 884
<211> 6
<212> DNA
<213> Artificial Sequence

<220>
<223> oligonucleotide 6-mer sequences

<400> 884
agataa                                                                  6

<210> 885
<211> 6
<212> DNA
<213> Artificial Sequence

<220>
<223> oligonucleotide 6-mer sequences

<400> 885
gcataa                                                                  6

<210> 886
<211> 6
<212> DNA
<213> Artificial Sequence

<220>
<223> oligonucleotide 6-mer sequences

<400> 886
acataa                                                                  6

<210> 887
<211> 6
<212> DNA
<213> Artificial Sequence

<220>
<223> oligonucleotide 6-mer sequences

<400> 887
taataa                                                                  6

<210> 888
<211> 6
<212> DNA
<213> Artificial Sequence

<220>
<223> oligonucleotide 6-mer sequences

<400> 888
caataa                                                                  6

<210> 889
<211> 6
<212> DNA
<213> Artificial Sequence

<220>
<223> oligonucleotide 6-mer sequences

<400> 889
cgtgaa                                                                  6

<210> 890
<211> 6
<212> DNA

<213> Artificial Sequence

<220>
<223> oligonucleotide 6-mer sequences

<400> 890
gatgaa 6

<210> 891
<211> 6
<212> DNA
<213> Artificial Sequence

<220>
<223> oligonucleotide 6-mer sequences

<400> 891
catgaa 6

<210> 892
<211> 6
<212> DNA
<213> Artificial Sequence

<220>
<223> oligonucleotide 6-mer sequences

<400> 892
ttggaa 6

<210> 893
<211> 6
<212> DNA
<213> Artificial Sequence

<220>
<223> oligonucleotide 6-mer sequences

<400> 893
tgcgaa 6

<210> 894
<211> 6
<212> DNA
<213> Artificial Sequence

<220>
<223> oligonucleotide 6-mer sequences

<400> 894
agcgaa 6

<210> 895
<211> 6
<212> DNA
<213> Artificial Sequence

<220>
<223> oligonucleotide 6-mer sequences

<400> 895
accgaa 6

<210> 896
<211> 6

<212> DNA
<213> Artificial Sequence

<220>
<223> oligonucleotide 6-mer sequences

<400> 896
aacgaa                                                                  6

<210> 897
<211> 6
<212> DNA
<213> Artificial Sequence

<220>
<223> oligonucleotide 6-mer sequences

<400> 897
ttagaa                                                                  6

<210> 898
<211> 6
<212> DNA
<213> Artificial Sequence

<220>
<223> oligonucleotide 6-mer sequences

<400> 898
gtagaa                                                                  6

<210> 899
<211> 6
<212> DNA
<213> Artificial Sequence

<220>
<223> oligonucleotide 6-mer sequences

<400> 899
ggagaa                                                                  6

<210> 900
<211> 6
<212> DNA
<213> Artificial Sequence

<220>
<223> oligonucleotide 6-mer sequences

<400> 900
ggtcaa                                                                  6

<210> 901
<211> 6
<212> DNA
<213> Artificial Sequence

<220>
<223> oligonucleotide 6-mer sequences

<400> 901
cctcaa                                                                  6

<210> 902

<211> 6
<212> DNA
<213> Artificial Sequence

<220>
<223> oligonucleotide 6-mer sequences

<400> 902
catcaa                                                                 6

<210> 903
<211> 6
<212> DNA
<213> Artificial Sequence

<220>
<223> oligonucleotide 6-mer sequences

<400> 903
ctgcaa                                                                 6

<210> 904
<211> 6
<212> DNA
<213> Artificial Sequence

<220>
<223> oligonucleotide 6-mer sequences

<400> 904
atgcaa                                                                 6

<210> 905
<211> 6
<212> DNA
<213> Artificial Sequence

<220>
<223> oligonucleotide 6-mer sequences

<400> 905
cggcaa                                                                 6

<210> 906
<211> 6
<212> DNA
<213> Artificial Sequence

<220>
<223> oligonucleotide 6-mer sequences

<400> 906
tcgcaa                                                                 6

<210> 907
<211> 6
<212> DNA
<213> Artificial Sequence

<220>
<223> oligonucleotide 6-mer sequences

<400> 907
ccgcaa                                                                 6

<210> 908
<211> 6
<212> DNA
<213> Artificial Sequence

<220>
<223> oligonucleotide 6-mer sequences

<400> 908
tagcaa                                                                  6

<210> 909
<211> 6
<212> DNA
<213> Artificial Sequence

<220>
<223> oligonucleotide 6-mer sequences

<400> 909
gagcaa                                                                  6

<210> 910
<211> 6
<212> DNA
<213> Artificial Sequence

<220>
<223> oligonucleotide 6-mer sequences

<400> 910
ttacaa                                                                  6

<210> 911
<211> 6
<212> DNA
<213> Artificial Sequence

<220>
<223> oligonucleotide 6-mer sequences

<400> 911
ctacaa                                                                  6

<210> 912
<211> 6
<212> DNA
<213> Artificial Sequence

<220>
<223> oligonucleotide 6-mer sequences

<400> 912
atacaa                                                                  6

<210> 913
<211> 6
<212> DNA
<213> Artificial Sequence

<220>
<223> oligonucleotide 6-mer sequences

<400> 913
tgacaa                                                                  6

<210> 914
<211> 6
<212> DNA
<213> Artificial Sequence

<220>
<223> oligonucleotide 6-mer sequences

<400> 914
ggacaa                                                                 6

<210> 915
<211> 6
<212> DNA
<213> Artificial Sequence

<220>
<223> oligonucleotide 6-mer sequences

<400> 915
cgacaa                                                                 6

<210> 916
<211> 6
<212> DNA
<213> Artificial Sequence

<220>
<223> oligonucleotide 6-mer sequences

<400> 916
gcacaa                                                                 6

<210> 917
<211> 6
<212> DNA
<213> Artificial Sequence

<220>
<223> oligonucleotide 6-mer sequences

<400> 917
acacaa                                                                 6

<210> 918
<211> 6
<212> DNA
<213> Artificial Sequence

<220>
<223> oligonucleotide 6-mer sequences

<400> 918
taacaa                                                                 6

<210> 919
<211> 6
<212> DNA
<213> Artificial Sequence

<220>
<223> oligonucleotide 6-mer sequences

<400> 919

aaacaa                                                                  6

<210> 920
<211> 6
<212> DNA
<213> Artificial Sequence

<220>
<223> oligonucleotide 6-mer sequences

<400> 920
tgtaaa                                                                  6

<210> 921
<211> 6
<212> DNA
<213> Artificial Sequence

<220>
<223> oligonucleotide 6-mer sequences

<400> 921
cgtaaa                                                                  6

<210> 922
<211> 6
<212> DNA
<213> Artificial Sequence

<220>
<223> oligonucleotide 6-mer sequences

<400> 922
gctaaa                                                                  6

<210> 923
<211> 6
<212> DNA
<213> Artificial Sequence

<220>
<223> oligonucleotide 6-mer sequences

<400> 923
cctaaa                                                                  6

<210> 924
<211> 6
<212> DNA
<213> Artificial Sequence

<220>
<223> oligonucleotide 6-mer sequences

<400> 924
tataaa                                                                  6

<210> 925
<211> 6
<212> DNA
<213> Artificial Sequence

<220>
<223> oligonucleotide 6-mer sequences

<400> 925
cataaa                                                                  6

<210> 926
<211> 6
<212> DNA
<213> Artificial Sequence

<220>
<223> oligonucleotide 6-mer sequences

<400> 926
gcgaaa                                                                  6

<210> 927
<211> 6
<212> DNA
<213> Artificial Sequence

<220>
<223> oligonucleotide 6-mer sequences

<400> 927
tgcaaa                                                                  6

<210> 928
<211> 6
<212> DNA
<213> Artificial Sequence

<220>
<223> oligonucleotide 6-mer sequences

<400> 928
cgcaaa                                                                  6

<210> 929
<211> 6
<212> DNA
<213> Artificial Sequence

<220>
<223> oligonucleotide 6-mer sequences

<400> 929
tccaaa                                                                  6

<210> 930
<211> 6
<212> DNA
<213> Artificial Sequence

<220>
<223> oligonucleotide 6-mer sequences

<400> 930
ctaaaa                                                                  6

<210> 931
<211> 6
<212> DNA
<213> Artificial Sequence

<220>
<223> oligonucleotide 6-mer sequences

<400> 931
ggaaaa                                                                 6

<210> 932
<211> 6
<212> DNA
<213> Artificial Sequence

<220>
<223> oligonucleotide 6-mer sequences

<400> 932
gcaaaa                                                                 6

<210> 933
<211> 6
<212> DNA
<213> Artificial Sequence

<220>
<223> oligonucleotide 6-mer sequences

<400> 933
taaaaa                                                                 6

<210> 934
<211> 26
<212> DNA
<213> Artificial Sequence

<220>
<223> T7 bacteriophage promoter

<400> 934
aattaatacg actcactata gggaga                                          26

<210> 935
<211> 23
<212> DNA
<213> Artificial Sequence

<220>
<221> misc_feature
<222> 22
<223> n = A,T,C or G

<220>
<223> SP6 bacteriophage promoter

<400> 935
atttaggtga cactatagaa gng                                             23

<210> 936
<211> 23
<212> DNA
<213> Artificial Sequence

<220>
<223> T3 bacteriophage promoter

<400> 936
aattaaccct cactaaaggg aga                                             23

<210> 937

<211> 15
<212> DNA
<213> Artificial Sequence

<220>
<223> Oligonucleotide

<400> 937
gcacgcgcac aagct                                                        15

<210> 938
<211> 16
<212> DNA
<213> Artificial Sequence

<220>
<223> Oligonucleotide

<400> 938
gggtcaccag caggca                                                       16

<210> 939
<211> 20
<212> DNA
<213> Artificial Sequence

<220>
<223> Oligonucleotide

<400> 939
acttcaagct cctaagccac                                                   20

<210>  940
<211>  18
<212>  DNA
<213>  Artificial Sequence

<220>
<223>  Synthetic primer

<400>  940
acggtggcca tggaagtc                                                       18

<210>  941
<211>  21
<212>  DNA
<213>  Artificial Sequence

<220>
<223>  Synthetic primer

<400>  941
tcggcaggtg agttgttaca c                                                   21

<210>  942
<211>  17
<212>  DNA
<213>  Artificial Sequence

<220>
<223>  Synthetic primer

<400>  942

actctttagc ggattcc                                                    17


<210>  943
<211>  15
<212>  DNA
<213>  Artificial Sequence

<220>
<223>  Synthetic primer

<400>  943
gcacgcgcac aagct                                                      15


<210>  944
<211>  16
<212>  DNA
<213>  Artificial Sequence

<220>
<223>  Synthetic primer

<400>  944
gggtcaccag caggca                                                     16


<210>  945
<211>  20
<212>  DNA
<213>  Artificial Sequence

<220>
<223>  Synthetic primer

<400>  945
acttcaagct cctaagccac                                                 20


<210>  946
<211>  25
<212>  DNA
<213>  Artificial Sequence

<220>
<223>  Synthetic primer

<400>  946
cgcaattaat acgactcact atagg                                           25


<210>  947
<211>  31
<212>  DNA
<213>  Artificial Sequence

<220>
<223>  Synthetic primer


<220>
<221>  misc_feature
<222>  (23)..(31)
<223>  n is a, c, g, or t

<400>  947
tgcattgaga gggtgtaatt tgnnnnnnnn n                                    31


<210>  948
<211>  22
<212>  DNA
<213>  Artificial Sequence

<220>
<223>  Synthetic primer

<400>  948
tgcattgaga gggtgtaatt tg                                              22


<210>  949
<211>  31
<212>  DNA
<213>  Artificial Sequence

<220>
<223>  Synthetic primer


<220>
<221>  misc_feature
<222>  (23)..(31)
<223>  n is a, c, g, or t

<400>  949
gtatcttggg cctgttgact tcnnnnnnnn n                                    31


<210>  950
<211>  22
<212>  DNA
<213>  Artificial Sequence

<220>
<223>  Synthetic primer

<400>  950
gtatcttggg cctgttgact tc                                              22


<210>  951
<211>  31
<212>  DNA
<213>  Artificial Sequence

<220>
<223>  Synthetic primer


<220>
<221>  misc_feature
<222>  (23)..(31)
<223>  n is a, c, g, or t

<400>  951
attgttcctt cagctgttcc atnnnnnnnn n                                    31


<210>  952

<211>  22
<212>  DNA
<213>  Artificial Sequence

<220>
<223>  Synthetic primer

<400>  952
attgttcctt cagctgttcc at                                                22


<210>  953
<211>  31
<212>  DNA
<213>  Artificial Sequence

<220>
<223>  Synthetic primer


<220>
<221>  misc_feature
<222>  (23)..(31)
<223>  n is a, c, g, or t

<400>  953
cctccacact cctaaggtca tcnnnnnnnn n                                      31


<210>  954
<211>  22
<212>  DNA
<213>  Artificial Sequence

<220>
<223>  Synthetic primer

<400>  954
cctccacact cctaaggtca tc                                                22


<210>  955
<211>  22
<212>  DNA
<213>  Artificial Sequence

<220>
<223>  Synthetic primer

<400>  955
gcgcaattaa tacgactcac ta                                                22


<210>  956
<211>  20
<212>  DNA
<213>  Artificial Sequence

<220>
<223>  Synthetic primer

<400>  956
ccgaactacc cacttgcatt                                                   20

<210>  957
<211>  29
<212>  DNA
<213>  Artificial Sequence

<220>
<223>  Synthetic primer


<220>
<221>  misc_feature
<222>  (21)..(29)
<223>  n is a, c, g, or t

<400>  957
ccactccatt tgttcgtgtg nnnnnnnnn                                         29


<210>  958
<211>  20
<212>  DNA
<213>  Artificial Sequence

<220>
<223>  Synthetic primer

<400>  958
ccgaactacc cacttgcatt                                                   20


<210>  959
<211>  20
<212>  DNA
<213>  Artificial Sequence

<220>
<223>  Synthetic primer

<400>  959
ccactccatt tgttcgtgtg                                                   20